# EUROPEAN PATENT SPECIFICATION

(11) **EP 1 753 719 B1**
(45) Date of publication and mention of the grant of the patent: **03.03.2010**
(21) Application number: 05749343.9
(22) Date of filing: 27.05.2005
(51) Int. Cl.: C07D 207/22, C07D 401/06, C07D 403/04, C07D 405/04, C07D 417/04, C07D 409/10, C07D 407/10, A61K 31/44, A61P 25/00, C07D 409/14, C07D 417/14

(54) **SUBSTITUTED PYRROLIDINE-2-ONES**
SUBSTITUIERTE PYRROLIDIN-2-ONE
PYRROLIDINE-2-ONES SUBSTITUEES

(30) Priority: 28.05.2004 GB 0412019
(43) Date of publication of application: 21.02.2007
(73) Proprietor: Novartis AG, 4056 Basel (CH)
(72) Inventor: MÜLLER, Werner, CH-3073 Gümligen (CH); NOZULAK, Joachim, 79423 Heitersheim (DE); ROY, Bernard, Lucien, CH-1700 Fribourg (CH); FEUERBACH, Dominik, 79379 Müllheim (DE)
(74) Representative: Vögeli-Lange, Regina
(86) International application number: PCT/EP2005/005722
(87) International publication number: WO 2005/118535

(56) References cited:
- EP-A- 0 413 191
- US-A1- 2003 119 880
- ACHESON, R. MORRIN ET AL: "Transformations involving the pyrrolidine ring of nicotine" JOURNAL OF THE CHEMICAL SOCIETY, PERKIN TRANSACTIONS 1: ORGANIC AND BIO-ORGANIC CHEMISTRY (1972-1999) , (2), 579-85 CODEN: JCPRB4; ISSN: 0300-922X, 1980, XP001076609

## Description

### Summary of the Invention

The invention relates to novel 3,5-disubstituted pyrrolidin-2-one compounds, to processes for their manufacture, their use as pharmaceuticals, their use in diagnosis, their use as PET ligands and to pharmaceutical or diagnostic compositions comprising such compounds, as well as other aspects related to the compounds, their manufacture and use.

### Background of the Invention

Alpha-7 nicotinic acetylcholine receptor agonists are useful in the treatment of psychotic disorders such as schizophrenia, mania, depression and anxiety, as well as for the treatment of neurodegenerative disorders such as senile dementia, Alzheimer's disease and other intellectual impairment disorders, such as attention deficit hyperactivity disorders (ADHD); Parkinson's disease, Huntington's chorea, amyotrophic lateral sclerosis and multiple sclerosis and others as desribed below.

A problem to be solved by the present invention is to provide novel alpha-7-nicotinic acetylcholine receptor agonists (α7 nicotinic acetylcholine receptor agonists or α7-nAChR agonists) with advantageous pharmaceutical properties.

### General Description of the Invention

A novel class of α7-nAChR binding compounds has been found that is based on 3,5-disubstituted pyrrolidin-2-one compounds and/or one or more salts thereof.

Among the advantageous properties of these compounds, inter alia a good activity as α7-nAChR agonists, in combination with sufficiently low activity as agonists or antagonists for other receptors, such as human muscle nicotinergic receptor, α3β4 nicotinergic receptor, and/or especially α4β2 nicotinergic receptor.

### Detailed Description of the Invention

The invention relates especially to (3,5-disubstituted pyrrolidin-2-one) compounds of the formula I, 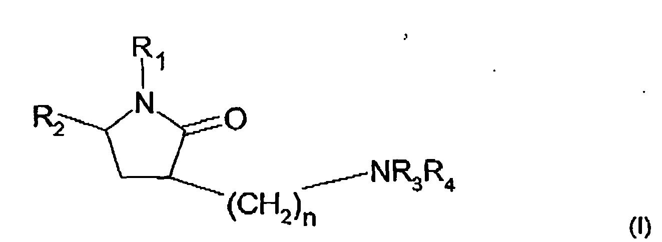 wherein
R₁ is hydrogen or unsubsituted or substituted lower alkyl,
R₂ is unsubstituted or substituted aryl, unsubstituted or substituted cycloalkyl, unsubstituted or substituted heterocyclyl, unsubstituted or substituted alkyl, substituted alkenyl or unsubstituted or substituted alkynyl,
R₃ and R₄ are, independently of each other, unsubstituted or substituted alkyl,
or NR₃R₄ is an unsubstituted or substituted heterocyclic ring, and
n is 1,
and/or a (preferably pharmaceutically acceptable) salts thereof as claimed.

Unless otherwise indicated, the general terms and names used in the description of the present invention preferably have the following meanings (where more specific definitions, in each case separately, or in combination, may be used to replace more general terms in order to define more preferred embodiments of the invention):

The term "lower" or "C₁-C₇-" defines a moiety with up to and including maximally 7, especially up to and including maximally 4, carbon atoms, said moiety being branched or straight-chained. Lower or C₁-C₇-alkyl, for example, is methyl, ethyl, n-propyl, sec-propyl, n-butyl, isobutyl, sec-butyl, tert-butyl, or further n-pentyl, n-hexyl or n-heptyl.

Where substituents are present, e.g. in "substituted" moieties selected from alkyl, aryl, heterocyclyl, cycloalkyl, cycloalkenyl, cycloalkynyl, carbocyclic rings and heterocyclic rings, the substituents, as far as chemically possible, are advantageously selected from alkyl, preferably C₁-C₇-alkyl, such as methyl, ethyl, n-propyl, isopropyl, n-butyl, isobutyl, tert-butyl, pentyl or hexyl (especially n-hexyl); cycloalkyl, especially C₃-C₈-cycloalkyl, such as cyclopentyl or cyclohexyl; phenyl or (1- or 2-) napthyl, each of which is unsubstituted or substituted with one or more, especially up to three, substituents selected from C₁-C₇-alkyl, halo-C₁-C₇-alkyl, such as trifluoromethyl, C₁-C₇-alkoxy, such as methoxy, halo-C₁-C₇-alkoxy, such as trifluoromethoxy, nitro, cyano, and halo, such as fluoro, chloro or bromo; unsubstituted, C₁-C₇-alkoxy-substituted or halosubstituted phenyl- C₁-C₇ alkyl such as benzyl, di(methoxy)-benzyl or chlorobenzyl; hydroxy; hydroxy-C₁-C₇-alkyl, such as hydroxymethyl; alkoxy, preferably C₁-C₇-alkoxy, especially methoxy, ethoxy or n-hexoxy; phenoxy; alkanoyloxy, especially C₁-C₇-alkanoyloxy, such as acetyloxy; C₁-C₇-alkanoylthio, such as methylthio; halo; amino; N-mono- or N,N-di-(C₁-C₇-alkyl)amino, such as dimethylamino; C₁-C₇-alkanoylamino, such as acetylamino; C₁-C₇-alkanoyl, such as acetyl; carboxy; C₁-C₇-alkoxycarbonyl, such as ethoxycarbonyl; carbamoyl, N-mono- or N,N-di-(C₁-C₇-alkyl)carbamoyl; C₁-C₇-alkylsulfonyl, such as mesyl; sulfamoyl; heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo, such as chloro, and C₁-C₇-alkyl, such as methyl, for example pyrrolidinyl, such as pyrrolidin-1-yl, thiophenyl, such as thiophen-2-yl or thiophen-3-yl, halo-thiophenyl, such as 3-chloro-thiophen-2-yl, thiazolyl, such as 2-thiazolyl, C₁-C₇-alkyl-substituted thiazolyl, such as 2-methyl-thiazol-4-yl, pyridinyl, such as pyridin-2- or pyridin-3-yl, indolyl, such as indol-4-yl, C₁-C₇-alkylindolyl, such as N-methyl-5-indolyl, quinolinyl, such as quinolin-5-yl or quinolin-8-yl, benzofuranyl, such as benzofuran-2-yl or benzofuran-5-yl, benzothiophenyl, such as 5-benzo[b]thiophenyl, benzothiazolyl, such as 2-benzothiazolyl, 2*H*-1,3-benzodioxolyl, such as 3,4-(-O-CH₂-O-)phenyl, 2,1,3-benzoxadiazolyl, such as 3,4-(=N-O-N=)phenyl, 2,1,3-benzothiadiazolyl, such as 3,4-(=N-S-N=)phenyl; and in the case of substituents of aryl a bivalent ligand that is bound to two adjacent carbon atoms in the aryl ring (thus forming a ring with the atoms to which it is bound) where the bivalent ligand is preferably selected from the group consisting of -O-CH₂-O-,-O-CH₂-CH₂-O-, -CH₂-O-CH₂-, -CH₂-CH₂-CH₂-, -CH₂-CH₂-CH₂-CH₂-, -O-CF₂-O-, =N-O-N= (with two instead of three conjugated double bonds in the benzo part here an in each case where mentioned below, thus together forming 2,1,3-benzoxadiazolyl) and =N-S-N= (with two instead of three conjugated double bonds in the benzo part here an in each case where mentioned below, thus together forming 2,1,3-benzothiadiazolyl). Where in the preceding and subsequent disclosure "substituted" moieties are mentioned, in a first preferred embodiment of the inventtion the substituents are selected from one or more, especially up to three, substituents independently selected from these substituents. Where in any heterocyclyl moieties or heterocyclic rings "unsaturated" is mentioned, this is intended to mean that the maximum number of non-cumulated double bonds is present.

Unsubstituted or substituted alkyl R₁ or R₂ is preferably C₁-C₇-alkyl that is unsubstituted or preferably substituted (especially at a terminal carbon atom) by one or more, preferably one, substituents as mentioned under substituted, preferably independently selected from the group consisting of
unsubstituted or substituted aryl, especially unsubstituted or substituted phenyl or unsubsituted or substituted naphthyl, where the substituents are preferably selected from C₁-C₇-alkyl, such as methyl, ethyl, n-propyl, isopropyl, n-butyl, isobutyl, tert-butyl, pentyl or hexyl (especially n-hexyl); phenyl or (1- or 2-) napthyl, each of which is unsubstituted or substituted with one or more, especially up to three, substituents selected from alkyl, preferably C₁-C₇-alkyl, halo-C₁-C₇-alkyl, such as trifluoromethyl, alkoxy, preferably C₁-C₇-alkoxy, such as methoxy or ethoxy, halo-C₁-C₇-alkoxy, such as trifluoromethoxy, nitro, cyano, and halo, such as fluoro, chloro or bromo; halo-lower alkyl, such as trifluoromethyl, nitro, cyano, hydroxy; hydroxy-C₁-C₇-alkyl, such as hydroxylmethyl; alkanoyloxy, especially C₁-C₇-alkanoyloxy, such as acetyloxy; halo, especially fluoro, chloro or bromo; amino; N-mono- or N,N-di-(C₁-C₇-alkyl)amino, such as dimethylamino; C₁-C₇-alkanoylamino, such as acetylamino; C₁-C₇-alkanoyl, such as acetyl; carboxy; C₁-C₇-alkoxycarbonyl, such as ethoxycarbonyl; carbamoyl, N-mono- or N,N-di-(C₁-C₇-alkyl)carbamoyl; C₁-C₇-alkylsulfonyl, such as mesyl; sulfamoyl; a bivalent ligand that is bound to two adjacent carbon atoms in the aryl ring (thus forming a ring with the atoms to which it is bound) where the bivalent ligand is preferably selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; and
unsubstituted or substituted heterocyclyl, especially heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo, such as chloro, and C₁-C₇-alkyl, such as methyl, for example thiophenyl, especially thiophen-2-yl or thiophen-3-yl, thiazolyl, such as 2-thiazolyl, pyridinyl, such as pyridin-2- or pyridin-3-yl, benzofuranyl, such as benzofuran-2-yl, indolyl, such as indol-4-yl, C₁-C₇-alkyl-indolyl, such as N-methyl-5-indolyl, benzothiophenyl, such as 5-benzo[b]thiophenyl, or benzothiazolyl, such as 2-benzothiazolyl.

In unsubstituted or substituted aryl, aryl is preferably a mono-, bi- or tricyclic aromatic hydrocarbon group with 6 to 14 ring carbon atoms, especially phenyl, naphthyl or fluorenyl, each of which is unsubstituted or substituted by one or more, especially 1 to 3, substituents selected preferably from those (mono- or bivalently bonded) mentioned above under "substituted". As R₂, unsubstituted or substituted aryl is preferably naphthyl or especially phenyl each of which is unsubstituted or substituted by one or more, especially up to three, moieties independently selected from alkyl, preferably C₁-C₇-alkyl, such as methyl, ethyl, n-propyl, isopropyl, n-butyl, isobutyl, tert-butyl, pentyl or hexyl (especially n-hexyl); cycloalkyl, especially C₃-C₈-cycloalkyl, such as cyclopentyl or cyclohexyl; unsubstituted, halo and/or C₁-C₇-alkoxy-substituted phenyl- or naphthyl-C₁-C₇-alkyl, such as benzyl or 2,4-dimethoxybenzyl; halo-lower alkyl, such as trifluoromethyl; nitro; cyano; phenyl or (1- or 2-) naphthyl, each phenyl or naphthyl of which is preferably present in the p-position to the bond with which the substituted aryl is bound to the rest of the molecule and is unsubstituted or substituted with one or more, especially up to three, substituents selected from C₁-C₇-alkyl, halo-C₁-C₇-alkyl, such as trifluoromethyl, C₁-C₇-alkoxy, such as methoxy, halo-C₁-C₇-alkoxy, such as trifluoromethoxy, phenoxy, C₁-C₇-alkylthio, such as methylthio, nitro, cyano, halo, such as fluoro, chloro or bromo, and a bivalent ligand that is bound to two adjacent carbon atoms in the aryl ring (thus forming a ring with the atoms to which it is bound) where the bivalent ligand is preferably selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; hydroxy; hydroxy-C₁-C₇-alkyl, such as hydroxylmethyl; alkoxy, preferably C₁-C₇-alkoxy, especially methoxy, ethoxy or n-hexyloxy; halo-lower alkyloxy, such as trifluoromethoxy; phenoxy; alkanoyloxy, especially C₁-C₇-alkanoyloxy, such as acetyloxy; halo, such as fluoro, cloro or bromo; amino; N-mono- or N,N-di-(C₁-C₇-alkyl)amino, such as dimethylamino; C₁-C₇-alkanoylamino, such as acetylamino; C₁-C₇-alkanoyl, such as acetyl; carboxy; C₁-C₇-alkoxycarbonyl, such as ethoxycarbonyl; carbamoyl, N-mono- or N,N-di-(C₁-C₇-alkyl)carbamoyl; sulfamoyl; C₁-C₇-alkylsulfonyl, such as mesyl; a bivalent ligand that is bound to two adjacent carbon atoms in the aryl ring (thus forming a ring with the atoms to which it is bound) where the bivalent ligand is preferably selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; and unsubstituted or substituted heterocyclyl with 3 to 10, especially 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo, such as chloro, and C₁-C₇-alkyl, such as methyl; for example pyrrolidinyl, such as pyrrolidin-1-yl, thiophenyl, such as thiophen-2-yl or thiophen-3-yl, halo-thiophenyl, such as 3-chloro-thiophen-2-yl, thiazolyl, such as 2-thiazolyl, C₁-C₇-alkyl-substituted thiazolyl, such as 2-methyl-thiazol-4-yl, pyridinyl, such as pyridin-2- or pyridin-3-yl, benzofuranyl, such as benzofuran-2-yl or benzofuran-5-yl, indolyl, such as indol-4-yl, C₁-C₇-alkyl-indolyl, such as N-methyl-5-indolyl, quinolinyl, such as quinolin-5-yl or quinolin-8-yl, benzothiophenyl, such as 5-benzo[b]thiophenyl, benzothiazolyl, such as 2-benzothiazolyl, 2H-1,3-benzodioxolyl, such as 3,4-(-O-CH₂-O-)phenyl, 2,1,3-benzoxadiazolyl, such as 3,4-(=N-O-N=)phenyl, or 2,1,3-benzothiadiazolyl, such as 3,4-(=N-O-N=)phenyl.

Unsubstituted or substituted cycloalkyl is preferably C₃-C₈-cycloalkyl, such as cyclopentyl or cyclohexyl or especially cyclopropyl where, if substituents are present which is the preferred case, preferably one is present selected from unsubstituted or substituted aryl as defined above, especially from phenyl that is unsubstituted or substituted by one or more, especially up to three, halo substituents, especially fluoro, chloro or bromo.

In unsubstituted or substituted heterocyclyl, heterocyclyl is preferably a ring with 3 to 8, preferably 5 to 7 ring atoms which is unsaturated, partially saturated or saturated, has one to three heteroatoms selected from O, N (or NH) and S as such or annealed to benzo, and is unsubstituted or substituted by up to three moieties independently selected from those mentioned above under "substituents"; unsubstituted or substituted heterocyclyl is, preferably, an unsubstituted or substituted moiety selected from pyrrolidinyl, such as pyrrolidin-1-yl, imidazolyl (very preferred), such as imidazol-2-yl, thiophenyl (very preferred), such as thiophen-2-yl, thiazolyl (very preferred), such as 2-thiazolyl, pyridinyl, such as pyridin-2- or pyridin-3-yl, indolyl, such as indol-4-yl, quinolinyl, such as quinolin-5-yl or quinolin-8-yl, benzofuranyl, such as benzofuran-2-yl or benzofuran-5-yl, benzothiophenyl, such as 5-benzo[b]-thiophenyl or benzothiazolyl, such as 2-benzothiazolyl; where unsubstituted or substituted heterocyclyl is especially selected from
(a) unsubstituted or substituted thiophenyl, such as thiophen-3-yl or especially thiophen-2-yl, halo-thiophenyl, such as 5-bromo-thiophen-2-yl, C₁-C₇- C₁-C₇-alkylthiophenyl, such as 5-methyl-thiophen-2-yl, (unsubstituted or substituted aryl)-thiophenyl, especially 4- or 5-(unsubstituted or substituted phenyl or naphthyl)-thiophen-2-yl, where the phenyl or naphthyl substituents are as defined above under "substituents", preferably one or more, especially up to two substituents independently selected from halo, such as fluoro, chloro or bromo, C₁-C₇-alkyl, such as methyl, halo-C₁-C₇-alkyl, such as trifluoromethyl, C₁-C₇-alkoxy, halo-C₁-C₇- alkoxy, such as trifluoromethoxy, nitro, cyano and a bivalent ligand that is bound to two adjacent carbon atoms in the aryl ring (thus forming a ring with the atoms to which it is bound) where the bivalent ligand is preferably selected from the group consisting of -O-CH₂-O-, -O-CH₂-CH₂-O-, -CH₂₋O-CH₂-, -CH₂-CH₂-CH₂-, -CH₂-CH₂-CH₂-CH₂-, -O-CF₂-O-, =N-O-N= and =N-S-N=, and thiophenyl substituted by an unsubstituted or halo or C₁-C₇-alkylsubstituted heterocyclyl as defined above, especially thiophenyl, such as thiophen-2-yl or thiophen-3-yl, chloro-thiophenyl, such as 3-chloro-thiophen-2-yl, pyridinyl, such as pyridine-3-yl, thiazolyl, such as thiazol-4-yl, C₁-C₇-thiazolyl, such as 2-methyl-thiazol-4-yl, quinolinyl, such as quinolin-5-yl or quinolin-8-yl, or benzofuranyl, such as benzofuran-2-yl;
(b) unsubstituted or substituted thiazolyl, especially unsubstituted or substituted thiazol-5-yl where the substituents are as defined under "substituents", especially one or more, preferably up to three moieties, most preferably one substituent independently selected from (i) unsubstituted or substituted aryl (especially as in (unsubstituted or substituted aryl or unsubstituted or substituted aryl-C₁-C₇-alkyl)-thiazol(especially-5-), yl, more especially 2-(unsubstituted or substituted phenyl, phenyl-C₁-C₇-alkyl (especially benzyl) or naphthyl)-thiazol-5-yl, where the aryl or especially phenyl or naphthyl substituents are as defined above, preferably being one or more, especially up to two substituents independently selected from halo, such as fluoro, chloro or bromo, C₁-C₇-alkyl, such as methyl, halo-C₁-C₇-alkyl, such as trifluoromethyl, C₁-C₇-alkoxy, halo-C₁-C₇-alkoxy, such as trifluoromethoxy, nitro, cyano, and a bivalent ligand that is bound to two adjacent carbon atoms in the aryl ring (thus forming a ring with the atoms to which it is bound) where the bivalent ligand is preferably selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; (ii) from heterocyclyl as defined above, especially thiophenyl, such as thiophen-2-yl or thiophen-3-yl, chloro-thiophenyl, such as 3-chloro-thiophen-2-yl, pyridinyl, such as pyridine-3-yl, thiazolyl, such as thiazol-4-yl, C₁-C₇-thiazolyl, such as 2-methyl-thiazol-4-yl, quinolinyl, such as quinolin-5-yl or quinolin-8-yl, or benzofuranyl, such as benzofuran-2-yl; and from (iii) unsubsituted or substituted aryl-C₁-C₇-alkyl, such as unsubstituted or substituted benzyl wherein the substitutents are preferably selected from those mentioned above, especially halo, such as chloro, and C₁-C₇-alkoxy, such as methoxy; and from
(c) unsubstituted or (especially mono- or di-) substituted imidazolyl, especially 4- or 5-substituted imidazol-2-yl that is unsubstituted or substituted at the 1-nitrogen, where the 4- or 5-substituent is preferably selected from unsubstituted and substituted aryl, especially phenyl, where the aryl, especially phenyl, substituents are preferably selected from halo, such as chloro, C₁-C₇-alkoxy, such as methoxy, and a bivalent ligand that is bound to two adjacent carbon atoms in the aryl ring (thus forming a ring with the atoms to which it is bound) where the bivalent ligand is preferably selected from the group consisting of -O-CH₂-O- and -O-CH₂CH₂-O-, and the 1-nitrogen substituent if present is preferably selected from aryl-C₁-C₇-alkyl, such as benzyl, wherein the aryl or especially benzene ring is unsubstituted or substituted by one or more substituents, especially up to three substituents, preferably C₁-C₇-alkoxy, such as methoxy.

Unsubstituted or substituted alkyl R₂ is preferably unsubstituted or substituted alkyl as described above. Preferred is ethyl that is terminally substituted either by unsubstituted or substituted aryl, especially unsubstituted or substituted phenyl or naphthyl, wherein the substituents, preferably one or more, especially up to three, are independently selected from the substituents mentioned above under "substituents", especially from halo, such as fluoro, chloro or bromo, C₁-C₇-alkyl, such as methyl or tert-butyl, halo-C₁-C₇-alkyl, such as trifluoromethyl, C₁-C₇-alkoxy, such as methoxy, halo-C₁-C₇-alkoxy, such as trifluoromethoxy, hydroxyl, hydroxyl-C₁-C₇-alkyl, such as hydroxymethyl, nitro, cyano, amino, N-mono- or N,N-di-C₁-C₇-alkylamino, such as dimethylamino, N-C₁-C₇-alkanoylamino, such as acetylamino, C₁-C₇-alkanoyloxy, such as acetoxy, C₁-C₇-alkanoyl, such as acetyl, carboxy, C₁-C₇-alkoxycarbonyl, such as ethoxycarbonyl, carbamoyl, N-mono- or N,N,di-(C₁-C₇-alkyl)-carbamoyl, such as N,N-di(ethyl)-carbamoyl, sulfamoyl, phenyl; from a bivalent ligand that is bound to two adjacent carbon atoms in the aryl ring (thus forming a ring with the atoms to which it is bound) where the bivalent ligand is preferably selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; and from unsubstituted or substituted heterocyclyl as defined above, especially pyridinyl, such as pyridin-2-yl or pyridin-3-yl, thiazolyl, such as thiazol-2-yl, indolyl, such as indol-5-yl, C₁-C₇-alkyl-indolyl, such as N-methyl-5-indolyl, benzofuranyl, such as 5-benzofuranyl, benzothiophenyl, such as 5-benzo[b]thiophenyl, or benzothiazolyl, such as 2-benzothiazolyl.

Substituted alkenyl R₂ is preferably C₂-C₄-alkenyl, especially vinyl, that is terminally substituted by unsubstituted or substituted aryl, especially unsubstituted or substituted phenyl, where preferably the aryl or phenyl substituents are up to three halo substituents, such as chloro; and carries a hydrogen or a C₁-C₇-alkyl in the 1-position (the carbon bound to the central pyrrolidinone ring in formula I); whereby the double bond, with respect to the terminal substituents and the central pyrrolidinone ring in formula I, is in the cis,trans- or preferably in the trans- or most preferably in the cis-configuration.

Unsubstituted or substituted alkynyl R₂ is preferably C₂-C₄-alkynyl, especially ethynyl (H-C≡C-), that is unsubstituted or preferably substituted (especially terminally) either by unsubstituted or substituted aryl, especially unsubstituted or substituted phenyl or naphthyl, wherein the substituents, preferably one or more, especially up to three, are preferably independently selected from the substituents mentioned above under "substituents", especially from halo, such as fluoro, chloro or bromo, C₁-C₇-alkyl, such as methyl or tert-butyl, C₁-C₇-alkoxy, such as methoxy, halo-C₁-C₇-alkyl, such as trifluoromethyl, hydroxyl, hydroxyl-C₁-C₇-alkyl, such as hydroxymethyl, cyano, amino, N-mono- or N,N-di-C₁-C₇-alkylamino, such as dimethylamino, N-C₁-C₇-alkanoylamino, such as acetylamino, C₁-C₇- alkanoyloxy, such as acetoxy, C₁-C₇-alkanoyl, such as acetyl, carboxy, C₁-C₇-alkoxycarbonyl, such as ethoxycarbonyl, carbamoyl, N-mono- or N,N-di-(C₁-C₇-alkyl)-carbamoyl, sulfamoyl, phenyl, and a bivalent ligand that is bound to two adjacent carbon atoms in the aryl ring (thus forming a ring with the atoms to which it is bound) where the bivalent ligand is preferably selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; or by unsubstituted or substituted heterocyclyl as defined above, especially pyridinyl, such as pyridin-2-yl or pyridin-3-yl, thiazolyl, such as thiazol-2-yl, indolyl, such as indol-5-yl, C₁-C₇-alkyl-indolyl, such as N-methyl-5-indolyl, benzofuranyl, such as 5-benzofuranyl, benzothiophenyl, such as 5-benzo[b]-thiophenyl, or benzothiazolyl, such as 2-benzothiazolyl.

Where R₃ and R₄ are, independently of each other, unsubstituted or substituted lower alkyl, methyl, n-propyl or especially ethyl are preferred.

An unsubstituted or substituted heterocyclic ring NR₃R₄ is preferably a ring with (including the binding nitrogen) 3 to 10 ring atoms, more preferably 5 to 7 ring atoms, which ring is unsubstituted or substituted by one or more substituents as mentioned above under "substituents", preferably up to three substituents selected from C₁-C₇-alkyl, such as methyl, and phenyl; where up to two, preferably up to one ring atom is a heteroatom selected from N, O and S; and which ring is saturated or comprises one or more double bonds; and is preferably 1,2,3,4-isoquinolinyl, piperazin-1-yl, 4-methyl-piperazin-1-yl, N-pyrrolidinyl, N-(4-phenyl)-1,2,3,4-tetrahydropyridyl or preferably N-pyrrolidinyl, N-azepanyl or especially N-piperidinyl.

The symbol n stands for 1.

Due to the asymmetrical carbon atom(s) present in the compounds of formula I and their salts, the compounds may exist in optically active form as isolated enantiomers or in the form of mixtures of two or more optical isomers, e.g. in form of racemic mixtures or as diastereomers. All optical isomers and their mixtures including the racemic mixtures are part of the present invention. In addition, due to the plane formed by the central pyrrolidinone ring which allows for the E- or Z-formation (trans or cis) of the substituents R₂ and R₃R₄N-(CH₂)ₙ-relatively to each other, and possibly also on further double bonds or at least partially unsaturated rings where present which may also be in the E- or Z- form (cis or trans), compounds of the formula I may also be present as mixtures of the respective cis and trans isomers or preferably only in one of these forms at each relevant bond or ring. Preferably, the compounds are in Z (cis)- form with regard to the pyrrolidinone ring substituents.

Salts of compounds of formula I are especially acid addition salts (if a basic group, such as the nitrogen carrying R₃ and R₄, is present in a compound of formula I, or amino is present), salts with bases (if an acidic group is present in a compound of formula I, such as the phosponomethyl moiety) or, where several salt-forming groups are present, can also be mixed salts or internal salts. Salts are especially pharmaceutically acceptable salts of compounds of formula I. Acid addition salts are formed, for example, from compounds of formula I with the basic nitrogen group carrying R₃ and R₄ are for example salts with inorganic acids, for example hydrohalic acids, such as hydrochloric acid, sulfuric acid or phosphoric acid, or with organic carboxylic, sulfonic, sulfo or phospho acids or N-substituted sulfamic acids, for example acetic acid, methanesulfonic acid, N-cyclohexylsulfamic acid (forming cyclamates) or with other acidic organic compounds, such as ascorbic acid. Acid groups in a compound of the formula I, such as carboxy, are, for example, salts thereof with suitable bases, such as non-toxic metal salts derived from metals of groups la, Ib, IIa and IIb of the Periodic Table of the Elements, for example sodium or potassium salts, or alkaline earth metal salts, for example magnesium or calcium salts, also zinc salts or ammonium salts, as well as salts formed with ammonia or organic amines or with quaternary ammonium compounds. Compounds of formula I having both acidic and basic groups can also form internal salts. For manufacturing, isolation and/or purification purposes, it is also possible to use pharmaceutically inacceptable salts, for example a perchlorate or picolinate salt.

Where compounds or a compound (especially of formula I) is mentioned herein, this is (if not explicitely mentioned otherwise) always intended to mean the free compound and/or a salt thereof, where salt-forming groups are present, and is also intended to comprise solvates of such a compound or salt, e.g. hydrates.

The compounds of the invention and their pharmaceutically acceptable acid addition salts, hereinafter referred to as compounds of the invention, exhibit valuable pharmacological properties when tested in vitro and in animals, and are therefore useful as pharmaceuticals.

Thus, the novel 3,5-disubstituted pyrrolidin-2-one compounds are found to be cholinergic ligands of the nAChR. In addition preferred compounds of the invention show selective α7-nAChR activity. The compounds of the present invention may in particular be found to be agonists, partial agonists, antagonists or allosteric modulators of the receptor.

Due to their pharmacological profiles, compounds of the invention are anticipated to be useful for the treatment of diseases or conditions as diverse as CNS related diseases, PNS related diseases, diseases related to inflammation, pain and withdrawal symptoms caused by an abuse of chemical substances; diseases or disorders related to the CNS include general anxiety disorders, cognitive disorders, learning and memory deficits and dysfunctions, Alzheimer's disease, ADHD, Parkinson's disease, Huntington's disease, ALS, prionic neurodegenerative disorders such as Cretzfeld-Jacob disease and kuru disease, Gilles de la Tourette's syndrome, psychosis, depression and depressive disorders, mania, manic depression, schizophrenia, the cognitive deficits in schizophrenia, obsessive compulsive disorders, panic disorders, eating disorders, narcolepsy, nociception, AIDS-dementia, senile dementia, mild cognitive dysfunctions related to age, autism, dyslexia, tardive dyskinesia, epilepsy, and convulsive disorders, post-traumatic stress disorders, transient anoxia, pseudodementia, pre-menstrual syndrome, late luteal phase syndrome, chronic fatigue syndrome and jet lag. Furthermore, compounds of the invention may be useful for the treatment of endocrine disorders, such as thyrotoxicosis, pheochromocytoma, hypertension and arrhythmias as well as angina pectoris, hyperkinesia, premature ejaculation and erectile difficulty. Still further, compounds of the invention may be useful in the treatment of inflammatory disorders (Wang et al., Nature 2003, 421,384), disorders or conditions including inflammatory skin disorders, Crohn's diesease, inflammatory bowel disease, ulcerative colitis and diarrhoea. Compounds of the invention may further be useful for the treatment of withdrawal symptoms caused by termination of the use of addictive substances, like tobacco, nicotine, opioids, benzodiazepines and alcohol. Also, compounds of the invention may be useful for the treatment of pain, e.g. caused by migraine, postoperative pain, phantom limb pain or pain associated with cancer. The pain may comprise inflammatory or neuropathic pain, central pain, chronic headache, pain related to diabetic neuropathy, to post therapeutic neuralgia or to peripheral nerve injury. Finally, degenerative ocular disorders may be treated, including ocular diseases which may directly or indirectly involve the degeneration of retinal cells, including ischemic retinopathies in general, anterior ischemic optic neuropathy, all forms of optic neuritis, age-related macular degeneration (AMD), in its dry forms (dry AMD) and/or its wet forms (wet AMD), diabetic retinopathy, cystoid macular edeme (CME), retinal detachment, retinitis prgmentosa, Stargardt's disease, Best's vitelliform retinal degeneration, Leber's congenital amaurosis and other hereditary retinal degenerations, pathologic myopia, retinopathy of prematurity and Leber's hereditary otic neuropathy.

In another aspect, the compounds of the invention are used as diagnostic agents and/or PET ligands, e.g. for the identification and localization of nicotine receptors in various tissues.

In particular, the agents of the invention are α7 nicotinic acetylcholine receptor (α7 nAChR) agonists.

In functional assays, the agents of the invention display high affinity at the α7 nAChR as shown in the following tests:
a) A functional assay for affinity at the α7 nAChR is carried out with a rat pituitary cell line stably expressing the α7 nAChR. Briefly, GH3 cells recombinantly expressing the nAChR α7 are seeded on black 96-well plates 72 h prior to the experiment and incubated at 37°C in a humidified atmosphere (5 % CO₂/95 % air). On the day of the experiment, medium is removed by flicking the plates and is replaced with 100 µl growth medium containing the fluorescent calcium sensitive dye Fluo-4, in the presence of 2.5 mM pro-benicid (Sigma). The cells are incubated at 37°C in a humidified atmosphere (5 % CO₂/95 % air) for 1 h. Plates are flicked to remove excess of Fluo-4, washed twice with Hepes-buffered salt solution (in mM: NaCl 130, KCl 5.4, CaCl₂ 2, MgSO₄ 0.8, NaH₂PO₄ 0.9, glucose 25, Hepes 20; pH 7.4; HBS) and refilled with 100 µl of HBS containing antagonists when appropriate. The incubation in the presence of the antagonist lasts between 3 and 5 min. Plates are then placed into an imaging plate reader, and the fluorescence signal is recorded: In this assay, compounds of the invention exhibit pEC₅₀ values of about 5 to about 9. Partial and potent agonists in this test are preferred.
b) To assess the antagonist activity of the compounds of the invention on the human neuronal nAChR α4β2, a similar functional assay is carried out using a human epithelial cell line stably expressing the human α4β2 subtype (Michelmore et al., Naunyn-Schmiedeberg's Arch. Pharmacol. (2002) 366, 235) In this assay, the preferred compounds of the invention show selectivity for the α7 nAChR subtypes.
c) To assess the antagonist activity of the compounds of the invention on the "ganglionic subtype" (α3β4), the muscle type of nicotinic receptor (α1β1γδ) and the 5-HT₃ receptor, similar functional tests as just described under a) are carried out with a human epithelial cell line stably expressing the human ganglionic subtype, a cell line endogenously expressing the human muscle type of nicotinic receptors or a cell line endogenously expressing the murine 5-HT₃ receptor (Michelmore et al., Naunyn-Schmiedeberg's Arch. Pharmacol. (2002) 366, 235. Compounds which display little or no activity on the α3β4 nAChR, the muscle subtype of nicotinic receptor as well as the5-HT₃ receptor are especially preferred.

In the model of mice showing sensory gating deficit (DBA/2-mice) described by S. Leonard et al. in Schizophrenia Bulletin 22, 431-445 (1996), the compounds of the invention induce significant sensory gating at concentrations of about 10 to about 40 µM.

The compounds of the invention may be shown to increase attention in a test of attention for rodents (Robbins, J. Neuropsychiatry Clin. Neurosci. (2001) 13, 326-35), namely the 5-choice serial reaction time test (5-CSRTT). In this test, the rat must observe a wall containing 5 holes. When a light flash appears in one of them, the rat must respond with a nose-poke into the correct hole within 5 sec. in order to receive a food pellet reward, delivered to a feeder in the opposite wall.

Compounds of the invention may also show learning/memory enhancing effects in the social recognition test in mice and rats (Ennaceur and Delacour, Behav. Brain Res. (1988) 31, 47-59).

The compounds of the invention are therefore useful for the prevention and treatment (including mitigation and prevention) of various disorders, especially those mentioned above. The usefulness of α7 nAChR agonists in neurodegeneration is documented in the literature, e.g. in Wang et al., J. Biol. Chem. 275, 5626-5632 (2000).

For the treatment of the above or other disorders, the appropriate dosage of a compound (active ingredient) of the invention will, of course, vary depending upon, for example, the host, the mode of administration and the nature and severity of the condition being treated as well as the relative potency of the particular agent of the invention employed. For example, the amount of active agent required may be determined on the basis of known in vitro and in vivo techniques, determining how long a particular active agent concentration in the blood plasma remains at an acceptable level for a therapeutic effect. In general, satisfactory results in animals are indicated to be obtained at daily dosages of from about 0.01 to about 30.0 mg/kg p.o. In humans, an indicated daily dosage is in the range of from about 0.7 to about 1400 mg/day p.o., e.g. from about 50 to 200 mg (70 kg man), conveniently administered once or in divided doses up to 4 x per day or in sustained release form. Oral dosage forms accordingly suitably comprise from about 1.75 or 2.0 to about 700 or 1400 mg of a compound of the invention admixed with an appropriate pharmaceutically acceptable diluent or carrier therefor.

Pharmaceutical compositions contain, for example, from about 0.1 % to about 99.9 %, preferably from about 20 % to about 60 %, of the active ingredient(s).

Examples for compositions comprising a compound of the invention include, for example, a solid dispersion, an aqueous solution, e.g. containing a solubilising agent, a microemulsion and a suspension of, e.g. a salt of a compound of formula I or a free compound of the formula I in the range of from 0.1 to 1 %, e.g. 0.5 %. The composition may be buffered to a pH in the range of, e.g. from 3.5 to 9.5, e.g. to pH 4.5, by a suitable buffer.

The compounds of the invention are also commercially useful as research chemicals.

For use according to the invention, a compound of the formula I and/or a pharmaceutically acceptable salt thereof may be administered as single active agent or in combination with one or more other active agents of the formula I and/or a pharmaceutically acceptable salt thereof or especially other active agents commonly employed especially for the treatment of the disorders mentioned herein or further other disorders, in any customary manner, e.g. orally, for example in the form of tablets, capsules, or as nasal spray, or parenterally, for example in the form of injection solutions or suspensions.

In the case of a combination, the pharmaceutical compositions for separate administration of the combination partners and/or those for administration in a fixed combination, i.e. a single galenical composition comprising at least two combination partners, according to the inventtion can be prepared in a manner known per se and are those suitable for enteral, such as oral or rectal, and parenteral administration to mammals, including man, comprising a therapeutically effective amount of at least one pharmacologically active combination partner alone or in combination with one or more .pharmaceutically acceptable carriers, especially suitable for enteral or parenteral application.

Pharmaceutical preparations for the combination therapy for enteral or parenteral administration are, for example, those in unit dosage forms, such as sugar-coated tablets, tablets, capsules or suppositories, or furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can instead with a single dosage unit also be reached by administration of a two or more dosage units.

In particular, a therapeutically effective amount of each of the combination partners may be administered simultaneously or sequentially and in any order, and the components may be administered separately (e.g. sequentially after fixed or variable periods of time), or as a fixed combination. For example, the method of treatment (including mitigation) of a disorder according to the invention may comprise (i) administration of the combination partner (a) (a compound of the present invention) in free or pharmaceutically acceptable salt form and (ii) administration of a combination partner (b) (e.g. a different compound of the present inventtion or an active ingredient of a different formula) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners can be administered separately at different times during the course of therapy or concurrently in divided or single combination forms. Furthermore, the term "administering" also encompasses the use of a prodrug of a combination partner that convert *in vivo* to the combination partner as such. The instant invention is therefore to be understood as embracing all such regimes of simultaneous and/ or alternating treatment and the term "administering" is to be interpreted accordingly.

The effective dosage of the combination partners employed may vary, for example depending on the particular compound or pharmaceutical composition employed, the mode of administration, the disorder being treated, and/or the severity of the disorder being treated. Thus, the dosage regimen is selected in accordance with a variety of factors including the route of administration, metabolism by and the renal and hepatic function of the patient. A physician, clinician or veterinarian of ordinary skill can readily determine and prescribe the effective amount of the single active ingredients required to prevent, mitigate, counter or arrest the disorder. Optimal precision in achieving concentration of the active ingredients within the range that yields efficacy without toxicity requires a regimen based on the kinetics of the active ingredients' availability to target sites.

In accordance with the foregoing, the present invention also provides:
(1) A compound of the formula I, and/or a salt thereof, for use in the diagnostic or therapeutic treatment of a mammal, especially a human; especially for use as an alpha-7 receptor agonist, for example for use in the treatment (including mitigation) of any one or more disorders, especially of any one or more of the particular disorders set forth hereinbefore and hereinafter.
(2) A pharmaceutical composition comprising a compound of the formula I, and/or a pharmaceutically acceptable salt thereof, as active ingredient together with a pharmaceutically acceptable diluent or carrier.
(2') A pharmaceutical composition for the treatment or prevention of a disorder in the treatment of which alpha-7 receptor activation plays a role or is involved and/or in which alpha-7 receptor activity is involved, especially any one or more of the disorders mentioned hereinbefore or hereinafter, comprising a compound of the formula I, and/or a pharmaceutically acceptable salt thereof, and a pharmaceutically acceptable diluent or carrier.
(3) A method for the treatment of a disorder, especially any one or more of the particular disorders set forth hereinbefore, in a subject in need of such treatment, comprising administering a pharmaceutically effective amount of a compound of the formula I, or a pharmaceutically acceptable salt thereof.
(3') A method for treating or preventing a disorder in the treatment of which alpha-7 receptor activation plays a role or is involved and/or in which alpha-7 receptor activity is involved, comprising administering to a mammal in need thereof a therapeutically effective amount of a compound of the formula I, and/or a pharmaceutically acceptable salt thereof.
(4) The use of a compound of the formula I, and/or a pharmaceutically acceptable salt thereof, for the manufacture of a medicament for the treatment or prevention of a disease or condition in the treatment of which alpha-7 receptor activation plays a role or is involved and/or in which alpha-7 receptor activity is involved, especially one or more of the disorders mentioned above.
(5) A method as defined above comprising co-administration, e.g. concomitantly or in sequence, of a therapeutically effective amount of an alpha-7 agonist of the formula I, and/or a pharmaceutically acceptable salt thereof, and a second pharmaceutically active compound and/or a pharmaceutically acceptable salt thereof, said second pharmaceutically active compound and/or salt thereof being especially for use in the treatment of any one or more of the disorders set forth hereinbefore or hereinafter.
(6) A combination comprising a therapeutically effective amount of an alpha-7 agonist of the formula I, and/or a pharmaceutically acceptable salt thereof, and a second pharmaceutically active compound and/or a pharmaceutically acceptable salt thereof, said second pharmaceutically active compound being especially for use or of use in the treatment of any one or more of the particular disorders set forth hereinbefore.

A preferred embodiment of the invention relates to a compound of the formula I, wherein
R₁ is C₁-C₇-alkyl, especially methyl;
R₂ is phenyl that is unsubstituted or substituted by one or more, especially up to three, substituents independently selected from C₁-C₇-alkyl, such as methyl, ethyl, n-propyl, isopropyl, n-butyl, isobutyl, tert-butyl, pentyl or hexyl (especially n-hexyl); C₃-C₈-cycloalkyl, such as cyclopentyl or cyclohexyl; unsubstituted, halo and/or C₁-C₇-alkoxy-substituted phenyl- or naphthyl-C₁-C₇-alkyl, such as benzyl or 2,4-dimethoxybenzyl; phenyl or (1- or 2-) napthyl, each phenyl or naphthyl of which is preferably present in the p-position to the bond with which the substituted phenyl is bound to the rest of the molecule and is unsubstituted or substituted with one or more, especially up to three, substituents selected from C₁-C₇-alkyl, halo-C₁-C₇-alkyl, such as trifluoromethyl, C₁-C₇-alkoxy, such as methoxy, halo-C₁-C₇-alkoxy, such as trifluoromethoxy, phenoxy, C₁-C₇-alkylthio, such as methylthio, nitro, cyano, halo, such as fluoro, chloro or bromo, and a bivalent ligand that is bound to two adjacent carbon atoms in the aryl ring (thus forming a ring with the atoms to which it is bound) where the bivalent ligand is selected from the group consisting of -O-CH₂-O-,-O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; hydroxy; hydroxy-C₁-C₇-alkyl, such as hydroxylmethyl; C₁-C₇-alkoxy, especially methoxy, ethoxy or n-hexyloxy; phenoxy; alkanoyloxy, especially C₁-C₇-alkanoyloxy, such as acetyloxy; halo, especially fluoro, chloro or bromo; halo-C₁-C₇alkyl, such as trifluoromethyl; nitro; amino; N-mono- or N,N-di-(C₁-C₇-alkyl)amino, such as dimethylamino; C₁-C₇-alkanoylamino, such as acetylamino; C₁-C₇-alkanoyl, such as acetyl; carboxy; C₁-C₇-alkoxycarbonyl, such as ethoxycarbonyl; carbamoyl, N-mono- or N,N-di-(C₁-C₇-alkyl)carbamoyl; sulfamoyl; C₁-C₇-alkylsulfonyl, such as mesyl; a bivalent ligand that is bound to two adjacent carbon atoms in the phenyl or naphthyl ring (thus forming a ring with the atoms to which it is bound) where the bivalent ligand is selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; and unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo, such as chloro, and C₁-C₇-alkyl, such as methyl, for example pyrrolidinyl, such as pyrrolidin-1-yl, thiophenyl, especially thiophen-2-yl or thiophen-3-yl, thiazolyl, such as 2-thiazolyl, pyridinyl, such as pyridin-2- or pyridin-3-yl, benzofuranyl, such as benzofuran-2-yl, indolyl, such as indol-4-yl, benzothiophenyl, such as 5-benzo[b]thiophenyl, and benzothiazolyl, such as 2-benzothiazolyl;
   C₃-C₈-cycloalkyl, especially cyclopentyl, cyclohexyl or preferably cyclopropyl that is substituted, preferably at a ring carbon different from that which binds to the central pyrrolidinone ring in formula I, especially in 2-position, by phenyl that is unsubstituted or substituted by one or more, especially up to three, substituents independently selected from those just mentioned for substituted phenyl R₂, especially phenyl or halo-substituted phenyl, such as fluoro, chloro or bromophenyl;
   unsubstituted or substituted heterocyclyl with 5 to 7 ring atoms which is unsaturated, partially saturated or saturated, and has one to three heteroatoms selected from O, N (or NH) and S as such or annealed to benzo, such as an unsubstituted or substituted moiety selected from pyrrolidinyl, such as pyrrolidin-1-yl, imidazolyl, such as imidazol-2-yl (very preferred), thiophenyl, such as thiophen-2-yl (very preferred), thiazolyl, such as 2-thiazolyl (very preferred), pyridinyl, such as pyridin-2- or pyridin-3-yl, indolyl, such as indol-4-yl, quinolinyl, such as quinolin-5-yl or quinolin-8-yl, benzofuranyl, such as benzofuran-2-yl or benzofuran-5-yl, benzothiophenyl, such as 5-benzo[b]thiophenyl, and benzothiazolyl, such as 2-benzothiazolyl; whereby heterocyclyl is unsubstituted or substituted by up to three moieties independently selected from halo, such as fluoro, chloro or bromo; C₁-C₇-alkyl, such as methyl; unsubstituted or substituted phenyl or unsubstituted or substituted naphthyl, in each case with up to three substituents independently selected from the group consisting of C₁-C₇-alkyl, such as methyl, ethyl, n-propyl, isopropyl, n-butyl, isobutyl, tert-butyl, pentyl or hexyl; phenyl-C₁-C₇-alkyl which is unsubstituted or substituted at the phenyl ring by up to three halo substituents, such as fluoro, chloro or bromo; C₁-C₇-alkoxy, especially methoxy or ethoxy; halo, such as fluoro, chloro or bromo; halo-C₁-C₇-alkyl, such as trifluoromethyl; halo-C₁-C₇-alkoxy, such as trifluoromethoxy; cyano; nitro; a bivalent ligand that is bound to two adjacent carbon atoms in the aryl- ring (thus forming a ring with the atoms to which it is bound) where the bivalent ligand is selected from the group consisting of -O-CH₂-O-,-O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; unsubstituted or substituted phenyl-C₁-C₇alkyl wherein the substituents are up to three substituents independently selected from halo, such as chloro, and C₁-C₇-alkoxy, such as methoxy; and unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo, such as chloro, and C₁-C₇-alkyl, such as methyl, for example thiophenyl, such as thiophen-2-yl or thiophen-3-yl, halo-thiophenyl, such as 3-chloro-thiophen-2-yl, C₁-C₇-alkylthiophenyl, such as 5-methyl-thiophen-2-yl, thiazolyl, such as 2-thiazolyl, C₁-C₇-alkyl-substituted thiazolyl, such as 2-methyl-thiazol-4-yl, pyridinyl, such as pyridin-2- or pyridin-3-yl, benzofuranyl, such as benzofuran-2-yl, or quinolinyl, such as quinolin-5-yl or quinolin-8-yl;
   substituted alkyl which is C₁-C₇-alkyl, preferably C₂-C₄-alkyl, that is substituted by unsubstituted or substituted phenyl or naphthyl, wherein the substituents, preferably one or more, especially up to three, are independently selected from halo, such as fluoro, chloro or bromo, C₁-C₇-alkyl, such as methyl, ethyl, isopropyl or tert-butyl, halo-C₁-C₇-alkalkyl, such as trifluoromethyl, C₁-C₇-alkoxy, such as methoxy, halo-C₁-C₇-alkoxy, such as trifluoromethoxy, hydroxyl, hydroxyl-C₁-C₇-alkyl, such as hydroxymethyl, nitro, cyano, amino, N-mono- or N,N-di-C₁-C₇-alkylamino, such as dimethylamino, N-C₁-C₇-alkanoylamino, such as acetylamino, C₁-C₇-alkanoyloxy, such as acetoxy, C₁-C₇-alkanoyl, such as acetyl, carboxy, C₁-C₇-alkoxycarbonyl, such as ethoxycarbonyl, carbamoyl, N-mono- or N,N-di-(C₁-C₇-alkyl)-carbamoyl, such as N,N-di(ethyl)-carbamoyl, sulfamoyl, phenyl; from a bivalent ligand that is bound to two adjacent carbon atoms in the aryl ring (thus forming a ring with the atoms to which it is bound) where the bivalent ligand is selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; and from unsubstituted or substituted heterocyclyl selected from pyridinyl, such as pyridine-2-yl or pyridine-3-yl, thiazolyl, such as thiazol-2-yl, indolyl, such as indol-5-yl, C₁-C₇-alkyl-indolyl, such as N-methyl-5-indolyl, benzofuranyl, such as 5-benzofuranyl, benzothiophenyl, such as 5-benzo[b]thiophenyl, and benzothiazolyl, such as 2-benzothiazolyl;
   unsubstituted or substituted alkenyl which is C₁-C₇-alkenyl, preferably C₂-C₄-alkenyl, especially vinyl, that is terminally substituted by unsubstituted or substituted phenyl with up to three halo substituents, especially chloro; and carries a hydrogen or a C₁-C₇-alkyl in the 1-position (the carbon bound to the central pyrrolidinone ring in formula I); whereby the double bond, with respect to the terminal substituents and the central pyrrolidinone ring in formula I, is in the cis,trans- or preferably in the trans- or most preferably in the cis-configuration;
   or unsubstituted or substituted alkynyl which is C₂-C₄-alkynyl, especially ethynyl that is substituted (especially terminally) either by unsubstituted or substituted phenyl or naphthyl, wherein the substituents, preferably one or more, especially up to three, are independently selected from halo, such as fluoro, chloro or bromo, C₁-C₇-alkyl, such as methyl or tert-butyl, C₁-C₇-alkoxy, such as methoxy, halo-C₁-C₇-alkyl, such as trifluoromethyl, hydroxyl, hydroxyl-C₁-C₇-alkyl, such as hydroxymethyl, cyano, amino, N-mono- or N,N-di-C₁-C₇-alkylamino, such as dimethylamino, N-C₁-C₇-alkanoylamino, such as acetylamino, C₁-C₇-alkanoyloxy, such as acetoxy, C₁-C₇-alkanoyl, such as acetyl, carboxy, C₁-C₇-alkoxycarbonyl, such as ethoxycarbonyl, carbamoyl, N-mono- or N,N-di-(C₁-C₇-alkyl)-carbamoyl, such as N,N-di(ethyl)-carbamoyl, sulfamoyl, phenyl, and a bivalent ligand that is bound to two adjacent carbon atoms in the aryl ring (thus forming a ring with the atoms to which it is bound) where the bivalent ligand is selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-. =N-O-N= and =N-S-N=; or by unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo, such as chloro, and C₁-C₇-alkyl, such as methyl, especially pyridin-2-yl or pyridin-3-yl, thiazolyl, such as thiazol-2-yl, indolyl, such as indol-5-yl, C₁-C₇-alkyl-indolyl, such as N-methyl-5-indolyl, benzofuranyl, such as 5-benzofuranyl, benzothiophenyl, such as 5-benzo[b]thiophenyl, or benzothiazolyl, such as 2-benzothiazolyl;
R₃ and R₄ are C₁-C₇-alkyl, especially n-propyl or preferably ethyl, or together with the binding nitrogen form ring with (including the binding nitrogen) 3 to 10 ring atoms, more preferably an N-piperidinyl (very preferred), an N-pyrrolidinyl or an N-azepanyl ring; and
n is 1;
and/or a (preferably pharmaceutically acceptable) salt thereof.

A more preferred embodiment of the invention relates to a compound of the formula -I, wherein
R₁ is C₁-C₇-alkyl, especially methyl;
R₂ is phenyl that is unsubstituted or substituted by one or more, especially up to three, substituents independently selected from C₁-C₇-alkyl, such as methyl, ethyl, n-propyl, isopropyl, n-butyl, isobutyl, tert-butyl, pentyl or hexyl (especially n-hexyl); C₁-C₇-alkoxy, especially n-hexyloxy; C₃-C₈-cycloalkyl, such as cyclopentyl or cyclohexyl; phenyl or (1- or 2-) napthyl, each phenyl or naphthyl of which is preferably present in the p-position to the bond with which the substituted phenyl is bound to the rest of the molecule and is unsubstituted or substituted with one or more, especially up to three, substituents selected from halo, such as fluoro, chloro or bromo; a bivalent ligand that is bound to two adjacent carbon atoms in the aryl ring (thus forming a ring with the atoms to which it is bound) where the bivalent ligand is selected from the group consisting of -O-CH₂-O-,-O-CH,-CH₂-O-, -CH₂-O-CH₂-, -CH₂-CH₂-CH₂-, -CH₂-CH₂-CH₂-CH₂-, -O-CF₂-O-, =N-O-N= and =N-S-N=; pyrrolidinyl, especially 4-pyrrolidin-1-yl; and thiophenyl, especially thiophen-2-yl or thiophen-3-yl, preferably in the 4-position of the phenyl to which it is bound as substituent,
   C₃-C₈-cycloalkyl, especially cyclopentyl, cyclohexyl or preferably cyclopropyl that is substituted, preferably at a ring carbon different from that which binds to the central pyrrolidinone ring in formula I, especially in 2-position, by phenyl or halo-substituted phenyl, such as fluoro, chloro or bromophenyl;
   imidazolyl, such as imidazol-2-yl, thiophenyl, such as thiophen-2-yl (very preferred), or thiazolyl, such as 2-thiazolyl, each of which is unsubstituted or substituted by up to three moieties independently selected from unsubstituted or substituted phenyl or unsubstituted or substituted naphthyl, if substituted in each case with up to three substituents independently selected from the group consisting of C₁-C₇-alkyl, such as methyl; C₁-C₇-alkoxy, especially methoxy, halo, such as fluoro, chloro or bromo, halo-C₁-C₇-alkyl, such as trifluoromethyl, cyano, nitro, a bivalent ligand that is bound to two adjacent carbon atoms in the aryl ring (thus forming a ring with the atoms to which it is bound) where the bivalent ligand is selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=, halo, such as chloro or bromo, C₁-C₇-alkyl, such as methyl, thiophenyl, such as thiophen-2-yl (preferred) or thiophen-3-yl, halo-thiophenyl, such as 3-chloro-thiophen-2-yl, and quinolinyl, such as quinolin-8-yl or especially quinolin-8-yl;
   unsubstituted or substituted vinyl that is terminally substituted by unsubstituted or substituted phenyl with up to three halo substituents, especially chloro; and carries a hydrogen or a C₁-C₇-alkyl, especially methyl, in the 1-position (the carbon bound to the central pyrrolidinone ring in formula I); whereby the double bond, with respect to the terminal substituents and the central pyrrolidinone ring in formula I, is in the cis,trans- or preferably in the trans- or most preferably in the cis-configuration;
   or unsubstituted or substituted ethynyl that is substituted either by unsubstituted or substituted phenyl, wherein the substituents, preferably one or more, especially up to three, are independently selected from halo, such as fluoro, chloro or bromo, C₁-C₇-alkyl, such as methyl or tert-butyl, C₁-C₇-alkoxy, such as methoxy, halo-C₁-C₇-alkyl, such as trifluoromethyl, hydroxyl, hydroxyl-C₁-C₇-alkyl, such as hydroxymethyl, cyano, amino, N-mono- or N,N-di-C₁-C₇-alkylamino, such as dimethylamino, N-C₁-C₇-alkanoylamino, such as acetylamino, C₁-C₇-alkanoyloxy, such as acetoxy, C₁-C₇-alkanoyl, such as acetyl, C₁-C₇-alkoxycarbonyl, such as ethoxycarbonyl, carbamoyl, N-mono- or N,N-di-(C₁-C₇-alkyl)-carbamoyl, such as N,N-di(ethyl)-carbamoyl, sulfamoyl and a bivalent ligand that is bound to two adjacent carbon atoms in the aryl ring (thus forming a ring with the atoms to which it is bound) where the bivalent ligand is selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; or by unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo, such as chloro, and C₁-C₇-alkyl, such as methyl, especially pyridin-2-yl or pyridin-3-yl, thiazolyl, such as thiazol-2-yl, indolyl, such as indol-5-yl, C₁-C₇-alkyl-indolyl, such as N-methyl-5-indolyl, benzofuranyl, such as 5-benzofuranyl, benzothiophenyl, such as 5-benzo[b]thiophenyl, or benzothiazolyl, such as 2-benzothiazolyl;
R₃ and R₄ together with the binding nitrogen form a ring with (including the binding nitrogen) 4 to 8 ring atoms, more preferably an N-piperidinyl (very preferred), an N-pyrrolidinyl or an N-azepanyl ring; and
n is 1;
and/or a (preferably pharmaceutically acceptable) salt thereof.

A further more preferred embodiment of the invention relates to a compound of the formula I, wherein
R₁ is C₁-C₇-alkyl, especially methyl;
R₂ is C₁-C₇-alkyl, preferably C₂-C₄-alkyl, that is substituted by unsubstituted or substituted phenyl or naphthyl, wherein the substituents, preferably one or more, especially up to three, are independently selected from those mentioned above under substituted, preferably selected from halo, such as fluoro, chloro or bromo, C₁-C₇-alkyl, such as methyl or tert-butyl, halo-C₁-C₇-alkC₁-C₇-alkyl, such as trifluoromethyl, C₁-C₇-alkoxy, such as methoxy, halo-C₁-C₇-alkoxy, such as trifluoromethoxy, hydroxyl, hydroxyl-C₁-C₇-alkyl, such as hydroxymethyl, nitro, cyano, amino, N-mono- or N,N-di-C₁-C₇-alkylamino, such as dimethylamino, N-C₁-C₇-alkanoylamino, such as acetylamino, C₁-C₇-alkanoyloxy, such as acetoxy, C₁-C₇-alkanoyl, such as acetyl, carboxy, C₁-C₇-alkoxycarbonyl, such as ethoxycarbonyl, carbamoyl, N-mono- or N,N-di-(C₁-C₇-alkyl)-carbamoyl, such as N,N-di(ethyl)-carbamoyl, sulfamoyl, phenyl; from a bivalent ligand that is bound to two adjacent carbon atoms in the aryl ring (thus forming a ring with the atoms to which it is bound) where the bivalent ligand is selected from the group consisting of -O-CH₂-O-,-O-CH₂-CH₂-O-, -CH₂-O-CH₂-, -CH₂-CH,-CH₂-, -CH₂-CH₂-CH₂-CH₂-, -O-CF₂-O-, =N-O-N= and =N-S-N=; and from unsubstituted or substituted heterocyclyl selected from pyridinyl, such as pyridine-2-yl or pyridine-3-yl, thiazolyl, such as thiazol-2-yl, indolyl, such as indol-5-yl, C₁-C₇-alkyl-indolyl, such as N-methyl-5-indolyl, benzofuranyl, such as 5-benzofuranyl, benzothiophenyl, such as 5-benzo[b]thiophenyl, and benzothiazolyl, such as 2-benzothiazolyl;
R₃ and R₄ together with the binding nitrogen form a ring with (including the binding nitrogen) 4 to 8 ring atoms, more preferably an N-piperidinyl (very preferred), an N-pyrrolidinyl or an N-azepanyl ring; and
n is 1;
and/or a (preferably pharmaceutically acceptable) salt thereof.

Especially preferred (both as such as well as regarding to their use) is any one or more of the compounds given in the Examples, and/or the pharmaceutically acceptable salts thereof, more especially a compound selected from the compounds given in Examples A1, A3, A5, A29, A42, A48, A51, A52, A53, A67, B1, B2, B3, C2, D1, D3, D5, D7, D8, D9, D10, D11, D12 to D28, D30 to D35, D37 to D41, D43 to D47, D50 to D59, E1, E3, E5, E7, E9, E12, E13, E14, E15, E18, E20, E21, E24, E28, E29, E33, E35, E36, E37, E38, E41, E44, G3, G7, G8, H2, H5, H6 and H3, or in a broader aspect of the invention F1, F3, F5, F8, F10, F11, F13 to F19, F22, F23, F24; most especially in the form of the isomer most active as agonist in a test system described above; and/or a pharmaceutically acceptable salt thereof.

### Manufacturing Processes

The compounds of the formula I, and/or the salts thereof, can be prepared according to methods that are, per se, known in the art, but are part of the invention due to the novelty of the compounds of the formula I as well as some of the starting materials. Preferably, the invention also relates to a process for the manufacture of a compound of the formula I, wherein
(a) for the synthesis of a compound of the formula I wherein n is 1 and R₁, R₂, R₃ and R₄ have the meanings given above and below for a compound of the formula I, a methylene compound of the formula II, 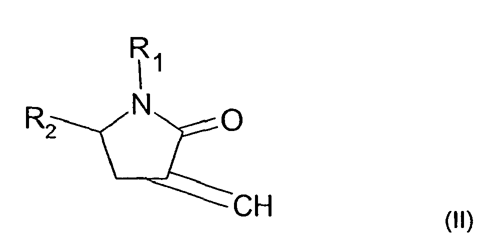 or a salt thereof where a salt-forming group is present, wherein R₁ and R₂ are as defined above or below for compounds of the formula I, is reacted with an imino compound of the formula III,

   HN(R₃R₄) (III)

   or a salt therof, wherein R₃ and R₄ have the meanings indicated above and below for a compound of the formula I; to a corresponding compound of the formula I, and/or a pharmaceutically acceptable salt thereof; to the corresponding compound of the formula I;
and, if desired, transforming a compound of formula I into a different compound of formula I, transforming a salt of an obtainable compound of formula I into the free compound or a different salt, transforming an obtainable free compound of formula I into a salt, and/or separating obtainable mixtures of isomers of compounds of formula I into the individual isomers.

All process steps described here can be carried out under known reaction conditions, preferably under those specifically mentioned, in the absence of or usually in the presence of solvents or diluents, preferably such as are inert to the reagents used and able to dissolve these, in the absence or presence of catalysts, condensing agents or neutralising agents, for example ion exchangers, typically cation exchangers, for example in the H⁺ form, depending on the type of reaction and/or reactants at reduced, normal, or elevated temperature, for example in the range from -100°C to about 190°C, preferably from about -80°C to about 150°C, for example at -80 to -60°C, at room temperature, at - 20 to 40°C or at the boiling point of the solvent used, under atmospheric pressure or in a closed vessel, where appropriate or expedient under pressure, and/or in an inert atmosphere, for example under argon or nitrogen.

The solvents from which those solvents that are suitable for any particular reaction may be selected include, for example, water, esters, such as lower alkyl-lower alkanoates, for example ethyl acetate, ethers, such as aliphatic ethers, for example diethyl ether, or cyclic ethers, for example tetrahydrofuran, liquid aromatic hydrocarbons, such as benzene or toluene, alcohols, such as methanol, ethanol or 1- or 2-propanol, or phenols, such as phenol, nitriles, such as aceto nitrile, halogenated hydrocarbons, such as methylene chloride, acid amides, such as dimethylformamide, bases, such as heterocyclic nitrogen bases, for example pyridine, carboxylic acid anhydrides, such as lower alkanoic acid anhydrides, for example acetic anhydride, cyclic, linear or branched hydrocarbons, such as cyclohexane, hexane or iso pentane, or mixtures of those solvents, for example aqueous solutions, unless otherwise indicated in the description of the processes. Such solvent mixtures may also be used in working up, for example by chromatography or partitioning.

Preferably, the reactions described under (a) are led under the following reaction conditions:

### Variant (a):

The reaction preferably takes place under customary reaction conditions useful in the addition of nucleophiles to double bonds, for example in the presence or (preferably if the imino compound of the formula III is liquid at the temperature used for the reaction) absence of a solvent, preferably at elevated temperatures, for example from 40 °C to the reflux temperature of the reaction mixture, preferably from 50 to 100 °C; preferably the compound of the formula III is used in molar excess over the compound of the formula II, for example in a 1.1 to 10 fold molar excess.

A leaving group X in a compound of the formula V or VI is preferably arylsulfonyloxy, such as toluenesulfonyloxy, lower alkanesulfonyloxy, such as methanesulfonyloxy, or especially halo, such as chloro, bromo or iodo, most especially bromo, or is formed *in situ,* for example from a hydroxy group according to known methods.

In a compound of the formula V, K is preferably tetramethylen, pentamethylen or heptamethylen (thus forming a pyrrolidinyl, piperidinyl or azepanyl moiety with the nitrogen to which it is bound).

### Optional Reactions/Conversions:

Compounds of the formula I may, if desired, be converted into different compounds of the formula I.

For example, a triple bond in a compound of the formula I wherein R₂ is substituted lower alkynyl may be reduced to the corresponding saturated bond yielding a compound wherein R₂ is substituted lower alkyl. The raction may take place under standard reaction conditions, e.g. by hydrogenation in the presence of a hydrogenation catalyst, e.g. Pt or Pd, in free form or on a carrier material, such as carbon, in an appropriate solvent, such as an alcohol, e.g. methanol.

An (especially 2-) halo-, e.g. 2-bromo-thiophenyl moiety or an (e.g. 4-)bromophenyl moiety R₂ in a compound of the formula I can be converted into the corresponding (unsubstituted or substituted (especially 2-halo or trihalomethyl) phenyl) moiety by reaction with the boronic acid of the corresponding unsubstituted or substituted benzene compound, thus yielding the corresponding 2-(unsubstituted or substituted phenyl)-thiophenyl or (unsubstituted or substituted phenyl)-phenyl compound. The reaction preferably takes place in the presence of a base, such as sodium carbonate, in an appropriate solvent, such as toluene and an alcohol, e.g. ethanol, a catalyst, such as Pd(OAc)₂ and triphenylphosphin, preferably at elevated temperatures, e.g. 50 °C to the reflux temperature of the reaction mixture, preferably at about 100 °C.

An unsubstituted or substituted imidazolyl (especially 2-imidazolyl) moiety R₂ that carries a removable N-substituent at the 1-nitrogen (e.g. a protective group), especially unsubstituted or substituted benzyl, such as benzyl or mono- or die-(methoxy)benzyl, such as 2,4-dimethoxybenzyl, e.g. can be converted to the corresponding moiety with free imidazolyl nitrogen, thus yielding the corresponding compound with a free 1-nitrogen in unsubstituted or substituted imidazolyl R₂. The reaction takes place under standard conditions for the removal of (unsubstituted or substituted)benzyl protecting groups, for example as described in standard textbooks referenced concerning protecting groups below, e.g. in the presence of acid, such as trifluoroacetic acid, in an appropriate solvent, e.g. anisole, or by catalytic transfer hydrogenation with an appropriate hydrogen donor, such as cyclohexene, cyclohexadiene, cis-decalin, formic acid or especially ammonium formiate in the presence of a catalyst, such as a noble metal in free or preferably carrier-bound form, e.g. Pd black or especially Pd-C, in an appropriate solvent, e.g. an alcohol, such as methanol, at preferred temperatures from 20 to the reflux temperature of the reaction mixture, e.g. up to about 100 °C.

Salts of a compound of formula I with a salt-forming group may be prepared in a manner known per se from the free compound. For example, acid addition salts of compounds of formula I may be obtained by treatment of the free compound with an acid or with a suitable anion exchange reagent. Salts of a compound of the formula I can usually be converted to free compounds, e.g. by treating with suitable basic agents, for example with alkali metal carbonates, hydrogencarbonates, or hydroxides, typically potassium carbonate or sodium hydroxide. Salts of a compound of the formula I may also be converted into different salts by treatment with appropriate salts. e.g. using a molar excess thereof over the salt of a compound of the formula I.

Stereoisomeric mixtures of a compound of the formula I, e.g. mixtures of diastereomers or cis/trans-isomers, as well as of starting materials can be separated into their corresponding isomers in a manner known per se by means of suitable separation methods. Diastereomeric mixtures or mixtures of cis/trans compounds for example may be separated into their individual diastereomers or cis/trans isomers by means of fractionated crystallization, chromatography (e.g. on silica gel, for example by thick layer chromatography), solvent distribution, and similar procedures. This separation may take place either at the level of one of the starting compounds or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

In the following description of some paradigmatic methods of preparation for starting materials as well as in the processes mentioned above and below, functional groups that are not to participate in the reaction and which would disturb the desired reaction or lead to side reactions are present in protected form, where required. The protection of functional groups and the respective protecting groups are, for example, described in the literature, for example in standard textbooks such as J. F. W. McOmie, "Protective Groups in Organic Chemistry", Plenum Press, London and New York 1973; in T. W. Greene and P. G. M. Wuts, "Protective Groups in Organic Synthesis", Third edition, Wiley, New York 1999; in "The Peptides"; Volume 3 (editors: E. Gross and J. Meienhofer), Academic Press, London und New York 1981, in "Methoden der organischen Chemie", Houben Weyl, 4. Ausgabe, Band 15/I, Georg Thieme Verlag, Stuttgart 1974, in H.-D. Jakubke and H. Jescheit, "Aminosäuren, Peptide, Proteine", Verlag Chemie, Weinheim, Deerfield Beach, and Basel 1982, and/or in Jochen Lehmann, "Chemie der Kohlenhydrate: Monosaccharide und Derivate", Georg Thieme Verlag, Stuttgart 1974. The removal of protecting groups is possible under custommary conditions, preferably as described in the mentioned references, and at appropriate reaction stages and steps. The groups that have to be protected are known to the person having skill in the art, and therefore the introduction, presence and/or removal of protecting groups are mentioned only if very important for the process steps described below. Although not especially mentioned, it is clear that the starting materials can also be used in the form of salts where salt-forming groups are present and the formation of salts does not lead to undesired reactions.

In view of the close relationship between the starting materials (starting materials and intermediates) in free form and in the form of their salts, any reference hereinbefore and hereinafter to a free compound or a salt thereof is to be understood as meaning also the corresponding salt or free compound or salt/free compound mixture, respectively, where appropriate and expedient.

The starting materials are known in the art or can be prepared according to or in analogy to methods that are known in the art.

Compounds of the formula II can, for example, be prepared by reacting a compound of the formula VII,

R₂-CH=NH-R₁ (VII)

wherein R₁ and R₂ are as defined for a compound of the formula I, with an acrylate compound of the formula VIII, 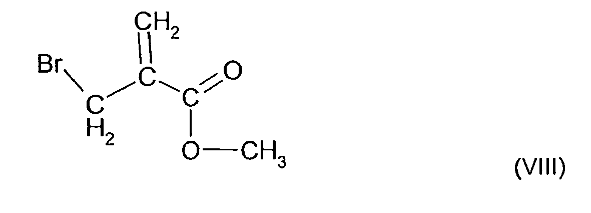 that has been treated in the presence of activated Zn (preferably activated before by treatment with hydrochloric acid and subsequent wasching with water, an alcohol, e.g. ethanol, and a dry ether, e.g. dry diethylether) in an appropriate solvent, at temperatures that are preferably in the range from 0 °C to 50 °C, preferably in the range from 20 to 30 °C, in an appropriate solvent, e.g. an ether, such as tetrahydrofurane.

A compound of the formula VII can, for example, be prepared by reacting an aldehyde compound of the formula IX,

R₂-CHO (IX)

wherein R₂ is as defined for a compound of the formula I, with an amino compound of the formula X,

R₁-NH₂ (X)

wherein R₁ is as defined for a compound of the formula I (preferably methyl), under customary reaction conditions for the synthesis of imides from aldehydes, e.g. in an appropriate solvent, such as an alcohol, e.g. methanol or ethanol, water, or mixtures of two or more of these solvents, at preferred temperatures between 0 °C and the reflux temperature of the reaction mixture, e.g. from about room temperature to about 80 °C or at reflux temperature.

The aldehydes of the formula IX are known or can be prepared according to or in analogy to methods that are known in the art. For example, they can be prepared as or in analogy to the methods described in the examples.

Thus, compounds of the formula IX wherein R₂ is unsubstituted or substituted alkynyl can be obtained from a compound of the formula XI,

R₂ₐ C≡CH (XI)

wherein R₂ₐ is a substitutent of substituted lower alkynyl as described for a compound of the formula I, by reacting it with first with a lithiating agent, such as butyllithium or lithium diisopropylamide, in an appropriate solvent, such as hexane and/or tetrahydrofurane, at low temperatures, e.g in the range of -50 to -80 °C, and then with a carbonylating agent, especially morpholine-4-carboxyaldehyde or an ortho formic tri-lower alkyl ester, such as ortho formic acid triethylester, in the presence of Znl₂ (see e.g. Org. Synth. Coll. Vol. IV, p. 801) in a solvent and at a temperature as just mentioned.

Compounds of the formula IX wherein R₂ is imidazolyl substituted by unsubstituted or substituted aryl can be prepared from a compound of the formula XII,

Ar-CHO (XII)

wherein Ar is unsubstitued or substituted aryl by reaction with ammonia or an appropriate (especially C₁-C₇-alkyl, preferably methyl or an unsubstituted or substituted phenyl-C₁-C₇-alkyl, such as unsubstituted or substituted benzyl)amine at elevated temperatures, e.g. between 30 °C and reflux temperature, in an appropriate solvent, such as ethanol, and then reacting the resulting imine with tosylmethyl isocyanide in the presence of a base, such as an alkali metal carbonate, e.g. sodium carbonate, in the presence or absence of an appropriate solvent, such as methanol or ethanol, at elevated temperatures, e.g. from 30 °C to 100 °C or in the presence of a solvent to the reflux temperature of the reaction mixture, to the corresponding imidazole of the formula XIII, 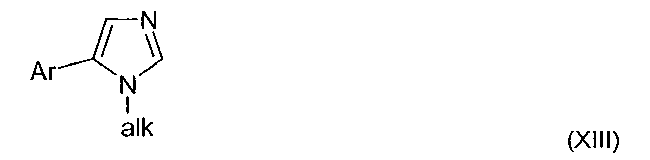 wherein Ar is as just defined and alk is hydrogen or preferably an aliphatic substituent, preferably C₁-C₇-alkyl, such as methyl, or unsubstituted or substituted phenyl-C₁-C₇alkyl, such as benzyl, which is then reacted first with a lithiating reagent, e.g. butyllithium, in an appropriate solvent, e.g. an ether, such as tetrahydrofurane, at low temperatures, such as - 50 to -80 °C, and then with N,N-dimethylmethanamide, preferably in a solvent as just mentioned and at preferred temperatures between -80 °C and room temperature, to give the corresponding compound of the formula IX. Alternatively, the compound of the formula XIII can be prepared from a compound of the formula XII as described above by reacting it in the presence of sodium cyanide with tosylmethyl isocyanide in the presence or absence of an appropriate solvent, such as methanol or ethanol, at a preferred temperature between 0 and 50 °C, e.g. at room temperature, and reacting the resulting 4,5-dihydrooxazole derivative with ammonia or an appropriate (especially C₁-C₇-alkyl, preferably methyl or an unsubstituted or substituted phenyl-C₁-C₇-alkyl, such as unsubstituted or substituted benzyl)amine at elevated temperatures, e.g. between 30 °C and reflux temperature, for example at 100 to 140 °C, in an appropriate solvent, such as toluene or xylene, to give the corresponding compound of the formula XIII.

A compound of the formula IX wherein R₂ is 5-(unsubstituted or substituted aryl)-thiazol-2-yl can be prepared by reacting 2-halothiazole, such as 2-bromothiazole, under customary coupling conditions, e.g. with an (unsubstituted or (especially 2-)-substituted aryl)boronic acid in the presence of a complex catalyst (e.g. Pd(OAc)₂) and triphenylphospine in an appropriate solvent, such as toluene and/or an alcohol, e.g. ethanol, preferably at elevated temperatures, e.g. 50 °C to the reflux temperature of the reaction mixture, preferably at about 100 °C, or with a corresponding (unsubstituted or substituted aryl)iodide treating first with Zn and then with Pd(OAc) in the presence of triphenylphosphine; and then reacting the resulting 2-(unsubstituted or substituted aryl)-substituted thiazole first with a lithiating agent, such as lithium diisopropylamide, in an appropriate solvent, such as an ether, e.g. tetrahydrofurane, at low temperatures, e.g. from -80 to -50 °C, and then with N-formyl-morpholine under the same conditions to give the corresponding compound of the formula IX. Alternatively, an (unsubstituted or substituted)aryl-thiocarbamate can be reacted with 2-chloro-propandial in the presence of phosphortrichloride to the corresponding 5-(unsubstituted or substituted aryl)-thiazole-5-carbaldehyde of the formula IX (see e.g. Chem. Ber. 97, 1986 (1964).

A compound of the formula IX wherein R₂ is (unsubstituted or substituted aryl)-cycloprop-2-yl can be prepared in analogy to Example C1 according to Tetrahedron Lett. 42, 6447 (1986) starting from the corresponding (unsubstituted or substituted aryl)-2-propenaldehyde instead of trans-cinnamaldehyde used in Example C1.

Compounds of the formula IV can be prepared according to or in analogy to methods that are known in the art; for example, a compound of the formula II, as defined above, is first reacted with an alkali metal cyanide, especially KCN, in an appropriate solvent, e.g. a mixture of water and an N,N-di-lower alkyl-alkanoylamide, such as dimethylformamide, to give a corresponding compound of the formula XIV, 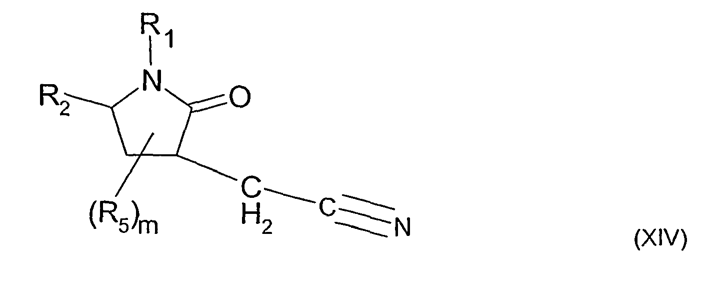 wherein R₁ and R₂ are as defined for a compound of the formula I, and reducing this compound, preferably using a complex hydride, e.g. sodium borohydride in the presence of CoCl₂ , in an appropriate solvent, such as an alcohol, e.g. ethanol, at preferred temperatures between 0 and 50 °C, e.g. about at room temperature (see e.g. Chem. Commun. (1999), page 2333).

The starting materials of the formulae III, V and VI as well as VIII, X and other starting materials are known in the art, commercially available and/or available-according to or in analogy to methods that are known in the art.

The invention relates also to those forms of the process in which a compound obtainable as intermediate at any stage of the process is used as starting material and the remaining process steps are carried out, or in which a starting material is formed under the reaction conditions or is used in the form of a derivative, for example in protected form or in the form of a salt, or a compound obtainable by the process according to the invention is produced under the process conditions and processed further in situ. In the process of the present invention there are preferably used those starting materials which result in the compounds of formulal described at the beginning as being especially valuable. Special preference is given to reaction conditions and processes of manufacture that are analogous to those mentioned in the Examples. The invention also relates to novel starting materials described above and below that are useful in the synthesis of compounds of the formula I.

### Examples

The following Examples serve to illustrate the invention without limiting the scope thereof:

### Abbreviations:

- [α]²⁰_{D}: sense of rotation
- Et: ethyl
- ESI: electrospray ionisation
- ether: diethylether
- LDA: lithium diisopropylamide
- m.p.: melting point (°C)
- Me: methyl
- Me₂: dimethyl
- Me₃: trimethyl
- MS: Mass Spectroscopy
- Phe: phenyl
- THF: tetrahydrofurane
- TosMIC: tosylmethyl isocyanide

### Example A1: (+)-cis-5-(3,4-Dimethyl-phenyl)-1-methyl-3-piperidin-1-ylmethyl-pyrrolidin-2-one hydrochloride

A solution of 5-(3,4-dimethyl-phenyl)-1-methyl-3-methylene-pyrrolidine-2-one (1.6 g, 7.4 mmol) and piperidine (3.7 ml) is heated up to 85 °C and stirred for 5 h. The reaction mixture is then cooled to room temperature and evaporated *in vacuo.* Chromatography of the residue on silica gel with acetic acid ethyl ester/ethanol/conc. aq. NH₃ (9:1:0.1) yields the racemic title compound as an oil (0.8 g). The racemic mixture is resolved on Chiralcel OD-H (Daicel Chiral Technologies, Inc., Exton USA; a polysaccharide-type chiral stationary phase) with n-hexane and isopropanol 19:1. The hydrochloride salt of the (+)-enantiomer is crystallized from isopropanol/ether, m.p. 143-147 °C, [α]²⁰D positive (c=0.5, HCl 1M). MS for C₁₉H₂₈N₂O (ESI) MH⁺ m/z 301.

The starting material is prepared as follows:

### Preparation of 5-(3,4-dimethyl-phenyl)-1-methyl-3-methylene-pyrrolidine-2-one:

a) A mixture of 3,4-dimethyl-benzaldehyde (1.0 g, 7.45 mmol) (Aldrich) and 33% solution of methylamine in ethanol (1.3 ml) is stirred at room temperature for 60 min and evaporated *in vacuo* to yield (3,4-dimethyl-benzylidene)-methyl-amine (1.08 g).
b) 2-Bromomethyl-acrylic acid methyl ester (4.0 g, 22.3 mmol) (Aldrich) is added slowly under cooling (internal temperature below 30 °C) to a mixture of *activated* zinc powder (washed in sequence with 2 M hydrochloric acid, water, ethanol and dry ether) (1.9 g) and absolute THF (15 ml). After stirring at room temperature for 30 min, a solution of (3,4-dimethyl-benzylidene)-methyl-amine (1.08 g, 7.40 mmol) in THF (4 ml) is added slowly. After 90 min saturated NH₄Cl solution (5 ml) is added under ice cooling, and the mixture is extracted with acetic acid ethyl ester. The organic phase is dried over sodium sulfate, filtered and evaporated. Chromatography on silica gel with acetic acid ethyl ester yields the title compound as a yellow powder, m.p. 180-184 °C, MS for C₁₄H₁₇NO (ESI) MH⁺ m/z 216.

The following compounds of formula I are prepared analogously to example A1:

### Example A2: (-)-cis-5-(3,4-Dimethyl-phenyl)-1-methyl-3-piperidin-1-ylmethyl-pyrrolidin-2-one hydrochloride

m.p. 138-142 °C, [α]²⁰_{D} negative (c=0.5, HCl 1M). MS for C₁₉H₂₈N₂O (ESI) MH⁺ m/z 301.

### Example A3: (+)-cis-5-(3,5-Dimethyl-phenyl)-1-methyl-3-piperidin-1-ylmethyl-pyrrolidin-2-one hydrochloride

m.p. 184-190 °C, [α]²⁰_{D} positive (c=0.5, HCl 1M). MS for C₁₉H₂₈N₂O (ESI) MH⁺ m/z 301.

### Example A4: (-)-cis-5-(3,5-Dimethyl-phenyl)-1-methyl-3-piperidin-1-ylmethyl-pyrrolidin-2-one hydrochloride

m.p. 172-180 °C, [α]²⁰_{D} negative (c=0.5, HCl 1M). MS for C₁₉H₂₈N₂O (ESI) MH⁺ m/z 301.

### Example A5: (+)-cis-1-Methyl-3-piperidin-1-ylmethyl-5-(5,6,7,8-tetrahydro-naphthalen-2-yl) -pyrrolidin-2-one hydrochloride

m.p. 208-210 °C, [α]²⁰_{D} positive (c=0.5, HCl 1M). MS for C₂₁H₃₀N₂O (ESI) MH⁺ m/z 327.

### Example A6: (-)-cis-1-Methyl-3-piperidin-1-ylmethyl-5-(5,6,7,8-tetrahydro-naphthalen-2-yl) -pyrrolidin-2-one hydrochloride

m.p. 234-238 °C, [α]²⁰_{D} negative (c=0.5, HCl 1M). MS for C₂₁H₃₀N₂O (ESI) MH⁺ m/z 327.

### Example A7: (-)-cis-5-(4-Bromo-phenyl)-1-methyl-3-piperidin-1-ylmethyl-pyrrolidin-2-one

m.p. 55-58 °C, [α]²⁰_{D} negative (c=0.5, HCl 1M). MS for C₁₇H₂₃BrN₂O (ESI) MH⁺ m/z 351/353.

### Examples A8 to A68

The following compounds of formula (A) in cis-form regarding the substituents R₂ and (R₃R₄N)-CH₂- at the pyrrolidinone ring are prepared according to methods as described herein, especially in the preceding Examples:

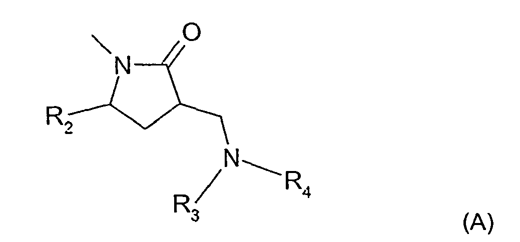

| Example | R₂ | R₃ | R₄ |
|---|---|---|---|
| A8 | 3-MeO-Phe | together | -(CH₂)₅- |
| A9 | 3-MeO-Phe | together | -(CH₂)₄- |
| A10 | 2-Cl-Phe | together | -(CH₂)₅- |
| A11 | 2-Cl-Phe | together | -(CH₂)₆- |
| A12 | 3,4-Me₂-Phe | together | -(CH₂)₂-N(Me)-(CH₂)₂- |
| A13 | 3,4-(-OCH₂O-)Phe | together | -(CH₂)₂-N(Me)-(CH₂)₂- |
| A14 | 2-Br-Phe | together | -(CH₂)₅- |
| A15 | 2-F-Phe | together | -(CH₂)₅- |
| A16 | 2-F₃C-Phe | together | -(CH₂)₅- |
| A17 | 2-MeO-Phe | together | -(CH₂)₅- |
| A18 | 2-Cl,6-F-Phe | together | -(CH₂)₅- |
| A19 | 2-Cl,6-Cl-Phe | together | -(CH₂)₅- |
| A20 | 2-Cl,4-Cl-Phe | together | -(CH₂)₅- |
| A21 | Phe | together | -(CH₂)₅- |
| A22 | 2-Cl,3-Cl-Phe | together | -(CH₂)₅- |
| A23 | 4-Cl,2-F-Phe | together | -(CH₂)₅- |
| A24 | 2-Cl,4-F-Phe | together | -(CH₂)₅- |
| A25 | 3-Me-Phe | together | -(CH₂)₅- |
| A26 | 3-F₃C-Phe | together | -(CH₂)₅- |
| A27 | 3-Cl-Phe | together | -(CH₂)₅- |
| A28 | 3-Br-Phe | together | -(CH₂)₅- |
| A29 | 3-(n-hexyl-O)-Phe | together | -(CH₂)₅- |
| A30 | 3-(Phe-O)-Phe | together | -(CH₂)₅- |
| A31 | 3-O₂N-Phe | together | -(CH₂)₅- |
| A32 | 3-H₂N-Phe | together | -(CH₂)₅- |
| A33 | 3-(Me₂N)-Phe | together | -(CH₂)₅- |
| A34 | 3-MeSO₂-Phe | together | -(CH₂)₅- |
| A35 | 4-F-Phe | together | -(CH₂)₅- |
| A36 | 4-Cl-Phe | together | -(CH₂)₅- |
| A37 | 4-Br-Phe | together | -(CH₂)₅- |
| A38 | 4-Me-Phe | together | -(CH₂)₅- |
| A39 | 4-Et-Phe | together | -(CH₂)₅- |
| A40 | 4-isopropyl-Phe | together | -(CH₂)₅- |
| A41 | 4-tert-butyl-Phe | together | -(CH₂)₅- |
| A42 | 4-cyclohexyl-Phe | together | -(CH₂)₅- |
| A43 | 4-cyclopentyl-Phe | together | -(CH₂)₅- |
| A44 | 4-MeO-Phe | together | -(CH₂)₅- |
| A45 | 4-F₃C-Phe | together | -(CH₂)₅- |
| A46 | 4-MeSO₂-Phe | together | -(CH₂)₅- |
| A47 | 4-(Me₂N)-Phe | together | -(CH₂)₅- |
| A48 | 4-(pyrrolidin-1-yl)-Phe | together | -(CH₂)₅- |
| A49 | 2,5-(Me₂)-Phe | together | -(CH₂)₅- |
| A50 | 3,4-(Me₂)Phe | together | -(CH₂)₅- |
| A51 | 3,4-Et₂-Phe | together | -(CH₂)₅- |
| A52 | 3,4-(-CH₂-CH₂-CH₂-)Phe | together | -(CH₂)₅- |
| A53 | 3,4-(-CH₂-CH₂-CH₂-CH₂-)Phe | together | -(CH₂)₅- |
| A54 | 3,4-(-O-CH₂-CH₂-O-)Phe | together | -(CH₂)₅- |
| A55 | 3,5-Me₂-Phe | together | -(CH₂)₅- |
| A56 | 3,5-(F₃C-)₂Phe | together | -(CH₂)₅- |
| A57 | 3-F₃C,4-F-Phe | together | -(CH₂)₅- |
| A58 | 2,4,5-(Me₃)-Phe | together | -(CH₂)₅- |
| A59 | 3-MeO, 5-MeO-Phe | together | -(CH₂)₅- |
| A60 | 3,5-Cl₂-Phe | together | -(CH₂)₅- |
| A61 | 2-Cl,6-F,3-Me-Phe | together | -(CH₂)₅- |
| A62 | 6-Cl,2F,3-Me-Phe | together | -(CH₂)₅- |
| A63 | 3,4-(-O-CH₂-O-)Phe | together | -(CH₂)₅- |
| A64 | 3,4-(-O-CF₂-O-)Phe | together | -(CH₂)₅- |
| A65 | 2,3-(=N-S-N=)Phe | together | -(CH₂)₅- |
| A66 | 3-Phe-Phe | together | -(CH₂)₅- |
| A67 | 4-(2-thiophenyl)-Phe | together | -(CH₂)₅- |
| A68 | 4-(benzofuran-2-yl)-Phe | together | -(CH₂)₅- |

Any of these compounds is a mixture of isomers or a pure enantiomer (especially the isomer most active as agonist in a test system described above). Where compounds are already described specifically, here the isomers or isomer mixtures not mentioned before are meant.

### Example B1: (±)-cis-1-Methyl-3-piperidin-1-ylmethyl-5-((E)-styryl)-pyrrolidin-2-one

A solution of 1-methyl-3-methylene-5-styryl-pyrrolidine-2-one (106 mg, 0.5 mmol) and piperidine (0.3 ml) is heated up to 85 °C and stirred for 4 h. The reaction mixture is cooled to room temperature and evaporated *in vacuo.* The residue is purified by thick layer chromatography on silica gel (acetic acid ethyl ester/ethanol/conc. aq. NH₃ 9:1:0.1) to yield the title compound as an oil (20 mg). MS for C₁₉H₂₆N₂O (ESI) MH⁺ m/z 299.

The starting material is prepared as follows:

### Preparation of 1-methyl-3-methylene-5-styryl-pyrrolidine-2-one:

a) A mixture of 3-phenyl-propenal (1.0 g, 7.56 mmol) (Aldrich) and a 40% aqueous solution of methylamine (1.3 ml) is stirred at 85 °C for 30 min. The reaction mixture is cooled to room temperature and extracted with ether. The organic phase is dried over sodium sulfate and evaporated to yield methyl-(3-phenyl-allylidene)-amine (1.05 g).
b) 2-Bromomethyl-acrylic acid methyl ester (1.85 g, 10.3 mmol) is added slowly under cooling (internal temperature below 30 °C) to a mixture of *activated* zinc powder (0.9 g) and absolute THF (20 ml). After stirring at room temperature for 30 min, a solution of methyl-(3-phenyl-allylidene)-amine (0.50 g, 3.44 mmol) in THF (20 ml) is added slowly. After 60 min saturated NH₄Cl solution (2 ml) is added under ice cooling and the mixture extracted with acetic acid ethyl ester. The organic phase is dried over sodium sulfate, filtered and evaporated. Chromatography on silica gel with cyclohexane/acetic acid ethyl ester 2:1 yields the title compound as a yellow oil, MS for C₁₄H₁₅NO (ESI) MH⁺ m/z 214.

The following compounds of formula I are prepared analogously to example B1:

### Example B2: (±)-cis-1-Methyl-5-((E)-1-methyl-2-phenyl-vinyl)-3-piperidin-1-ylmethyl-pyrrolidin-2-one

MS for C₂₀H₂₈N₂O (ESI) MH⁺ m/z 313.

### Example B3: (±)-cis-5-[(E)-2-(2-Chloro-phenyl)-1-methyl-vinyl]-1-methyl-3-piperidin-1-ylmethyl-pyrrolidin-2-one

MS for C₂₀H₂₇ClN₂O (ESI) MH⁺ m/z 347/349.

### Example C1: 1-Methyl-5-(2-phenyl-cyclopropyl)-3-piperidin-1-ylmethyl-pyrrolidin-2-one

A solution of 1-methyl-3-methylene-5-(2-phenyl-cyclopropyl)-pyrrolidin-2-one (65 mg, 0.29 mmol) in piperidine (0.3 ml) is heated up to 65 °CC and stirred for 5 h. The reaction mixture is cooled and evaporated. The crude product is purified by thick layer chromatography (ethyl acetate/ethanol/conc. ammonia, 90:10:1) to yield the title product as colorless oil. [α]²²_{D} negative (c=0.25 in methanol). MS for C₂₀H₂₈N₂O (ESI) MH⁺ m/z 313.

The starting material is prepared as follows:

### Preparation of 1-methyl-3-methylene-5-(2-phenyl-cyclopropyl)-pyrrolidin-2-one:

a) A mixture of 2-phenyl-cyclopropanecarbaldehyd (470 mg, 3.21 mmol) [prepared e.g. according to Mori, Atsunori; Arai, Isao; Yamamoto, Hisashi; Nakai, Hisao; Arai, Yoshinobu, Tetrahedron 42, 6447(1986). Asymmetric Simmons-Smith reactions using homochiral protecting groups] and 40% aqueous solution of methylamine (5.6 ml) is stirred at 80 °C for 1 h. The reaction mixture is cooled to room temperature and extracted with ethyl acetate. The organic phase is dried over sodium sulfate and evaporated to give methyl-[1-(2-phenylcyclopropyl)-methylidene]-amine as oil.
b) 2-Bromomethyl-acrylic acid methyl ester (1.7 g, 9.6 mmol) is added slowly under cooling (internal temperature below 30 °C) to a mixture of *activated* zinc powder (0.8 g) and absolute THF (5 ml). The reaction mixture is stirred at room temperature for 60 min. Keeping the reaction mixture at room temperature, a solution of methyl-[1-(2-phenyl-cyclopropyl)-methylidene]-amine (5.1 g, 3.2 mmol) in THF (5 ml) is added drop wise. The reaction mixture is stirred at room temperature for 90 min, subsequently quenched with a saturated ammonium chloride solution and extracted with ether. The organic phase is dried over sodium sulfate, filtered and evaporated. Chromatography on silica gel with cyclohexane/acetone 80:20 yields the title compound as a yellow oil, MS for C₁₅H₁₇NO (ESI) MH⁺ m/z 228.

The following compound of formula I is prepared analogously to example C1:

### Example C2: 5-[2-(2-Chloro-phenyl)-cyclopropyl]-1-methyl-3-piperidin-1-ylmethyl-pyrrolidin-2-one

m.p. 87-90 °C, [α]²⁰_{D} negative (c=0.35, methanol). MS for C₂₀H₂₇ClN₂O (ESI) MH⁺ m/z 347/349.

### Example D1: (+)-cis-1-Methyl-3-piperidin-1-ylmethyl-5-m-tolylethynyl-pyrrolidin-2-one hydrochloride

A solution of 1-methyl-3-methylene-5-m-tolylethynyl-pyrrolidine-2-one (1.6 g, 7.1 mmol) and 3.6 ml piperidine is heated up to 85 °C and stirred for 90 min. The reaction mixture is cooled to room temperature and evaporated *in vacuo*. Chromatography of the residue on silica gel with acetic acid ethyl ester/ethanol/conc. aq. NH₃ (9:1:0.1) yields the racemic title compound as an oil (510 mg). The racemic mixture is resolved on Chiralpak AD (Daicel Chiral Technologies, Inc., Exton, USA; chiral stationary phase) with n-hexane and ethanol 19:1. The hydrochloride salt of the (+)-enantiomer is crystallized from isopropanol/ether, m.p. 163-167 °C, [α]²⁰_{D} positive (c=0.5, HCl 1M). MS for C₂₀H₂₆N₂O (ESI) MH⁺ m/z 311.

The starting material is prepared as follows:

### Preparation of 1-methyl-3-methylene-5-m-tolylethynyl-pyrrolidine-2-one:

a) 49.8 ml of a solution of n-BuLi in hexan (1.6 M) is added at -60 °C under argon to a solution of 1-ethynyl-3-methyl-benzene (10.0 g, 36.2 mmol) in absolute THF (90 ml). After 1 h at -60 °C, morpholine-4-carboxaldehyde (4.6 g, 39.8 mmol) is added. The reaction mixture is quenched with ice/diluted HCl solution (30 ml) and extracted with ether. The organic phase is dried over sodium sulfate and evaporated. Chromatography on silica gel with cyclohexane/ethyl acetate 10:1 yields m-tolyl-propynal as a yellow liquid.
b) A mixture of m-tolyl-propynal (3.8 g, 26.4 mmol) and 33% solution of methylamine in ethanol (100 ml) is stirred at room temperature for 45 min and, after evaporation *in vacuo,* yields methyl-[3-m-tolyl-prop-2-yn-(E)-ylidene]-amine (3.8 g).
c) 2-Bromomethyl-acrylic acid methyl ester (13.0 g, 72.7 mmol) is added slowly under cooling to a mixture of *activated* zinc powder and absolute THF (18 ml) so that the reaction temperature stays below 30 °C. After stirring of the mixture at room temperature for 20 min, a solution of methyl-[3-m-tolyl-prop-2-yn-(E)-ylidene]-amine (3.8 g, 24.2 mmol) in THF (4 ml) is added slowly. After stirring of the reaction mixture at room temperature for 90 min, saturated NH₄Cl solution (2 ml) followed by acetic acid ethyl ester (2 ml) are added under ice cooling. The organic phase is dried over sodium sulfate, filtered and evaporated. Chromatography on silica gel with cyclohexane/ethyl acetate 2:1 yields the title compound as a yellow liquid. MS for C₁₅H₁₅NO (ESI) MH⁺ m/z 226.

The following compounds of formula I are prepared analogously to example D1:

### Example D2: (-)-cis-1-Methyl-3-piperidin-1-ylmethyl-5-m-tolylethynyl-pyrrolidin-2-one hydrochloride

m.p. 169-172 °C, [α]²⁰_{D} negative (c=0.5, HCl 1M). MS for C₂₀H₂₆N₂O (ESI) MH⁺ m/z 311.

### Example D3: (+)-cis-1-Methyl-5-phenylethynyl-3-piperidin-1-ylmethyl-pyrrolidin-2-one hydrochloride

m.p. 197-201 °C, [α]²⁰_{D} positive (c=0.5, HCl 1M). MS for C₁₉H₂₄N2O (ESI) MH⁺ m/z 297.

### Example D4: (-)-cis-1-Methyl-5-phenylethynyl-3-piperidin-1-ylmethyl-pyrrolidin-2-one hydrochloride

m.p. 199-203 °C, [α]²⁰_{D} negative (c=0.5, HCl 1M). MS for C₁₉H₂₄N₂O (ESI) MH⁺ m/z 297.

### Example D5: (+)-cis-5-(2-Chloro-phenylethynyl)-1-methyl-3-piperidin-1-ylmethyl-pyrrolidin-2-one hydrochloride

m.p. 166-169 °C, [α]²⁰_{D} positive (c=0.5, HCl 1M). MS for C₁₉H₂₃ClN₂O (ESI) MH⁺ m/z 3317333

### Example D6: (-)-cis-5-(2-Chloro-phenylethynyl)-1-methyl-3-piperidin-1-ylmethyl-pyrrolidin-2-one hydrochloride

m.p. 160-165 °C, [α]²⁰_{D} negative (c=0.5, HCl 1M). MS for C₁₉H₂₃ClN₂O (ESI) MH⁺ m/z 331/333

### Example D7: (+)-cis-N-[2-(1-Methyl-5-oxo-4-piperidin-1-ylmethyl-pyrrolidin-2-ylethynyl)-phenyl]-acetamide hydrochloride

m.p. 135-140 °C, [α]²⁰_{D} positive (c=0.5, HCl 1M). MS for C₂₁H₂₇N₃O₂ (ESI) MH⁺ m/z 354.

### Example D8: (+)-cis-1-Methyl-5-(1-methyl-1H-indol-5-ylethynyl)-3-piperidin-1-ylmethyl-pyrrolidin-2-one

[α]²⁰_{D} positive (c=0.25, HCl 1 M). MS for C₂₂H₂₇N₃O (ESI) MH⁺ m/z 350.

### Example D9: (+)-cis-5-Benzofuranyl-5-ylethynyl-1-methyl-3-piperidin-1-ylmethyl-pyrrolidin-2-one hydrochloride

m.p. 217-222 °C, [α]²⁰_{D} positive (c= 0.5, HCl 1M). MS for C₂₁H₂₄N₂O₂(ESI) MH⁺ m/z 337.

### Examples D10 to D60:

The following compounds of formula (D) in cis-form regarding the substituents R₂*-C≡C- and (R₃R₄N)-CH₂- at the pyrrolidinone ring are prepared according to methods as described herein, especially in the preceding Examples:

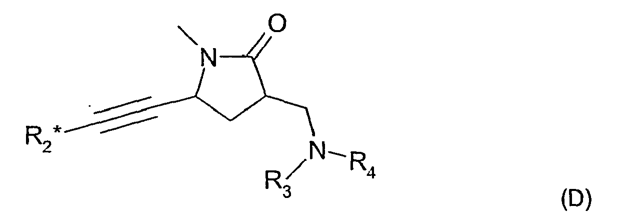

| Example | R₂* | R₃ | R₄ |
|---|---|---|---|
| D10 | Phe | together | -(CH₂)₅- |
| D11 | 2-Cl-Phe | together | -(CH₂)₅- |
| D12 | 2-Br-Phe | together | -(CH₂)₅- |
| D13 | 2-F-Phe | together | -(CH₂)₅- |
| D14 | 2-Me-Phe | together | -(CH₂)₅- |
| D15 | 2-MeO-Phe | together | -(C2)5- |
| D16 | 2-F₃C-Phe | together | -(CH₂)₅- |
| D17 | 2-CN-Phe | together | -(CH₂)₅- |
| D18 | 2-hydroxy-Phe | together | -(CH₂)₅- |
| D19 | 3-Cl-Phe | together | -(CH₂)₅- |
| D20 | 3-MeO-Phe | together | -(CH₂)₅- |
| D21 | 3-F₃C-Phe | together | -(CH₂)₅- |
| D22 | 3-(Me₂N)-Phe | together | -(CH₂)₅- |
| D23 | 3-hydroxy-Phe | together | -(CH₂)₅- |
| D24 | 3-acetoxy-Phe | together | -(CH₂)₅- |
| D25 | 3-amino-Phe | together | -(CH₂)₅- |
| D26 | 3-acetylamino-Phe | together | -(CH₂)₅- |
| D27 | 3-hydroxymethyl-Phe | together | -(CH₂)₅- |
| D28 | 3-acetyl-Phe | together | -(CH₂)₅- |
| D29 | 3-carboxy-Phe | together | -(CH₂)₅- |
| D30 | 3-ethoxycarbonyl-Phe | together | -(CH₂)₅- |
| D31 | 3-N,N-diethylcarbamoyl-Phe | together | -(CH₂)₅- |
| D32 | 3-sulfamoyl-Phe | together | -(CH₂)₅- |
| D33 | 4-Cl-Phe | together | -(CH₂)₅- |
| D34 | 4-F-Phe | together | -(CH₂)₅- |
| D35 | 4-Me-Phe | together | -(CH₂)₅- |
| D36 | 4-Me-O-Phe | together | -(CH₂)₅- |
| D37 | 4-tert-butyl-Phe | together | -(CH₂)₅- |
| D38 | 3,4-(-O-CH₂-O-)Phe | together | -(CH₂)₅- |
| D39 | 3,4-(-O-CH₂-CH₂-O-)Phe | together | -(CH₂)₅- |
| D40 | 2,3-((-CH₂)₄-)Phe | together | -(CH₂)₅- |
| D41 | 3,4-((-CH₂)₄-)Phe | together | -(CH₂)₅- |
| D42 | 3,4,5-trimethoxy-Phe | together | -(CH₂)₅- |
| D43 | 4-Phe-Phe | together | -(CH₂)₅- |
| D44 | 2-Cl,3-Cl-Phe | together | -(CH₂)₅- |
| D45 | 2-F,5-F-Phe | together | -(CH₂)₅- |
| D46 | 2-Cl-,5-methyl-Phe | together | -(CH₂)₅- |
| D47 | 3-Me,5-Me-Phe | together | -(CH₂)₅- |
| D48 | 3-F₃C-,5-F₃C-Phe | together | -(CH₂)₅- |
| D49 | 3-acetyl,5-acetyl-Phe | together | -(CH₂)₅- |
| D50 | 4-hydroxy-3-Me-Phe | together | -(CH₂)₅- |
| D51 | 2,3-(=N-S-N=)Phe | together | -(CH₂)₅- |
| D52 | 2,3-(-CH=CH-CH=CH-)Phe | together | -(CH₂)₅- |
| D53 | 5-indolyl | together | -(CH₂)₅- |
| D54 | 5-benzofuranyl | together | -(CH₂)₅- |
| D55 | 5-benzo[b]thiophenyl | together | -(CH₂)₅- |
| D56 | 2-pyridinyl | together | -(CH₂)₅- |
| D57 | 3-pyridinyl | together | -(CH₂)₅- |
| D58 | 2-thiazolyl | together | -(CH₂)₅- |
| D59 | 2-benzothiazolyl | together | -(CH₂)₅- |
| D60 | H | together | -(CH₂)₅- |
| D61 | 2-Me-Phe | -CH₂-CH₃ | -CH₂-CH₃ |
| D62 | 4-Cl-Phe | -CH₂CH₃ | -CH₂-CH₃ |

Any of these compounds is a mixture of isomers or a pure enantiomer (especially the isomer most active as agonist in a test system described above). Where compounds are already described specifically, here the isomers or isomer mixtures not mentioned before are meant.

### Example E1: (+)-cis-5-(5-Bromo-thiophen-2-yl)-1-methyl-3-piperidin-1-ylmethyl-pyrrolidin-2-one

A solution of 5-(5-bromo-thiopheny-2-yl)-1-methyl-3-methylene-pyrrolidine-2-one (4,1 g, 15,1 mmol) and 4.0 ml piperidine is heated up to 65 °C and stirred for 2h. The reaction mixture is cooled to room temperature and evaporated *in vacuo.* Crystallization of the residue from diethyl ether yields the racemic title compound, m.p. 69-71 °C. The racemic mixture is resolved on Chiralcel OD-H (Daicel Chiral Technologies, Inc., Exton, USA; polysaccharide based chiral stationary phase) with n-hexane and isopropanol 19:1. [α]²⁷_{D} positive (c 0.5, methanol). MS for C₁₅H₂₁BrN₂OS (ESI) MH⁺ m/z 357/359.
a) A mixture of 5-bromo-thiophene-2-carbaldehyde (Aldrich) (4.0 g, 21.0 mmol) and a 40% aqueous solution of methylamine (3.7 ml) is stirred at 80 °C for 60 min. The reaction mixture is cooled to room temperature and extracted with ether. The organic phase is dried over sodium sulfate and evaporated to yield (5-bromo-thiophen-2-ylmethylene)-methyl-amine.
b) 2-Bromomethyl-acrylic acid methyl ester (10.3 g, 57.3 mmol) is added slowly under cooling (internal temperature below 30 °C) to a mixture of *activated* zinc powder (5.0 g) and absolute THF (35 ml). After stirring of the mixture at room temperature for 60 min, a solution of (5-bromo-thiophen-2-ylmethylene)-methyl-amine (3.9 g, 19.1 mmol) in THF (8 ml) is added slowly. After 60 min saturated NH₄Cl solution (10 ml) is added under ice cooling and the mixture extracted with acetic acid ethyl ester. The organic phase is dried over sodium sulfate, filtered and evaporated. Chromatography on silica gel with cyclohexane/ethyl acetate 2:1 yields 5-(5-bromothiophen-2-yl)-1-methyl-3-methylene-pyrrolidine-2-one, m.p. 177-180 °C.

### Example E2: (-)-cis-5-(5-bromo-thiophen-2-yl)-1-methyl-3-piperidin-1-ylmethyl-pyrrolidin-2-one

The title compound is prepared in analogy to that of the preceding example: [α]²⁶_{D} negative (c 0.5, methanol). MS for C₁₅H₂₁BrN₂OS (ESI) MH⁺ m/z 357/359.

### Example E3: (+)-cis-5-[5-(2-Chloro-phenyl)-thiophen-2-yl]-1-methyl-3-piperidin-1-yl-methyl-pyrrolidin-2-one

A solution of (+)-cis-5-(5-bromo-thiophen-2-yl)-1-methyl-3-piperidin-1-ylmethyl-pyrrolidin-2-one (1.0 g, 2.80 mmol), 2-chlorobenzeneboronic acid (657 mg, 4.2 mmol) (Aldrich), 2 M aqueous Na₂CO₃ solution (4.0 ml) and ethanol (2 ml) in toluene (15 ml) is degassed and flushed with argon before Pd(OAc)₂ (19 mg) and triphenylphosphine (73 mg) are added. The mixture is stirred at 100 °C for 2 h. After addition of charcoal the mixture is filtered and the filtrate extracted with ethyl acetate. The organic phase is dried over sodium sulfate and evaporated. Chromatography on silica gel eluting with acetic acid ethyl ester/methanol (4:1) yields the title compound (0.9 g), m.p. 73-75 °C, [α]²⁰_{D} positive (c 0.5, HCl 1M). MS for C₂₀H₂₆N₂O (ESI) MH⁺ m/z 311.

The following compounds of formula I are prepared analogously to example E3:

### Example E4: (-)-cis-5-[5-(2-Chloro-phenyl)-thiophen-2-yl]-1-methyl-3-piperidin-1-yl-methyl-pyrrolidin-2-one hydrochloride

m.p. 168-172 °C, [α]²⁰_{D} negative (c 0.5, HCI 1M). MS for C₂₀H₂₆N₂O (ESI) MH⁺ m/z 311.

### Example E5: (+)-cis-5-[3,5-Dichloro-phenyl)-thiophen-2-yl]-1-methyl-3-piperidin-1-yl-methyl-pyrrolidin-2-one hydrochloride

m.p. 179-181 °C, [α]²³_{D} positive (c 0.25, HCl 1M). MS for C₂₁H₂₄Cl₂N₂OS (ESI) MH⁺ m/z 423/425.

### Example E6: (-)-cis-5-[3,5-Dichloro-phenyl)-thiophen-2-yl]-1-methyl-3-piperidin-1-yl-methyl-pyrrolidin-2-one hydrochloride

m.p. 179-181 °C, [α]²⁶_{D} negative (c 0.25, methanol). MS for C₂₁H₂₄Cl₂N₂OS (ESI) MH⁺ m/z 423/425.

### Example E7: (+)-cis-5-[2,4-Dimethoxy-phenyl)-thiophen-2-yl]-1-methyl-3-piperidin-1-yl-methyl-pyrrolidin-2-one hydrochloride

m.p. 238-239 °C, [α]²²_{D} positive (c 0.25, water). MS for C₂₃H₃₀N₂O₃S (ESI) MH⁺ m/z 415.

### Example E8: (-)-cis-5-[2,4-Dimethoxy-phenyl)-thiophen-2-yl]-1-methyl-3-piperidin-1-yl-methyl-pyrrolidin-2-one hydrochloride

m.p. 240-241 °C, [α]²²_{D} negative (c 0.25, water). MS for C₂₃H₃₀N₂O₃S (ESI) MH⁺ m/z 415.

### Example E9: (+)-cis-5-[5-(3-Chloro-phenyl)-thiophen-2-yl]-1-methyl-3-piperidin-1-yl-methyl-pyrrolidin-2-one hydrochloride

m.p. 162-163 °C, [α]²³_{D} positive (c 0.25, HCl 1M). MS for C₂₁H₂₅ClN₂OS (ESI) MH⁺ m/z 389/391.

### Example E10: (-)-cis-5-[5-(3-Chloro-phenyl)-thiophen-2-yl]-1-methyl-3-piperidin-1-yl-methyl-pyrrolidin-2-one hydrochloride

m.p. 155-158 °C, [a]²³_{D} negative (c 0.28, HCl 1M). MS for C₂₁H₂₅ClN₂OS (ESI) MH⁺ m/z 389/391.

### Examples E11 to E20:

The following compounds of formula (E-I) in cis-form regarding the substituents 5-R₂**-thiophen-2-yl and 1-piperidinylmethyl at the pyrrolidinone ring, are prepared according to methods as described herein, especially in the preceding Examples:

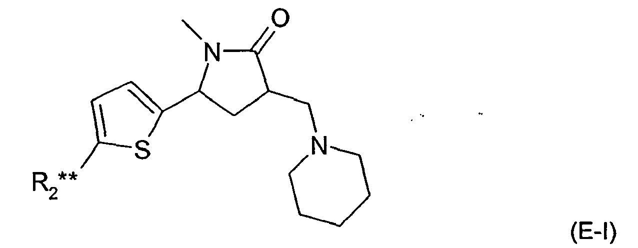

| Example | R₂** |
|---|---|
| E11 | Me |
| E12 | Phe |
| E13 | 2-Cl-Phe |
| E14 | 3-Br-Phe |
| E15 | 2-Me-Phe |
| E16 | 2-F₃C-Phe |
| E17 | 2-F₃C-O-Phe |
| E18 | 3-Cl-Phe |
| E19 | 3-F₃C-Phe |
| E20 | 3-nitro-Phe |
| E21 | 3-CN-Phe |
| E22 | 4-Cl-Phe |
| E23 | 4-F-Phe |
| E24 | 4-Me-Phe |
| E25 | 2-Cl,3-CI-Phe |
| E26 | 2-Cl,4-Cl-Phe |
| E27 | 4-Cl,2-Me-Phe |
| E28 | 2-MeO,4-MeO-Phe |
| E29 | 2-F₃C,4-F₃C-Phe |
| E30 | 3-Cl,5-Cl-Phe |
| E31 | 3-F,4-Me-Phe |
| E32 | 3-Cl,4-Cl-Phe |
| E33 | 3,5-Me₂-Phe |
| E34 | 3-F₃C,5F₃C-Phe |
| E35 | 3,4-(-O-CH₂-O-)Phe |
| E36 | thiophen-2-yl |
| E37 | thiophen-3-yl |
| E38 | quinolin-5-yl |
| E39 | quinolin-8-yl |
| E40 | 2-naphthyl |
| E41 | 3,4-(=N-O-N=)Phe |

Any of these compounds is a mixture of isomers or a pure enantiomer (especially the isomer most active as agonist in a test system described above). Where compounds are already described specifically, here the isomers or isomer mixtures not mentioned before are meant..

### Examples E42 to E44:

The following compounds of formula (E-II) in cis-form regarding the substituents 4-R₂**-thiophen-2-yl and 1-piperidinylmethyl at the pyrrolidinone ring are prepared according to methods as described herein, especially in the preceding Examples:

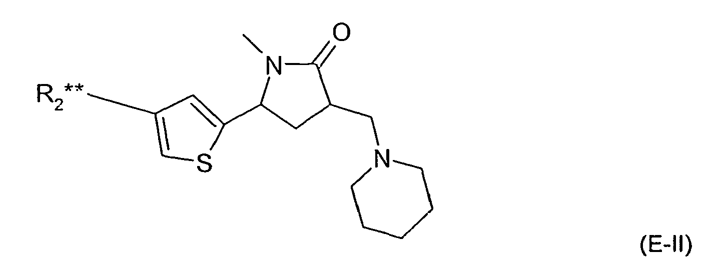

| Example | R₂** |
|---|---|
| E42 | Br |
| E43 | Phe |
| E44 | 2-Cl-Phe |

Any of these compounds is a mixture of isomers or a pure enantiomer (especially the isomer most active as agonist in a test system described above).

### Example F1: (+)-cis-5-[2-(2-Chloro-phenyl)-thiazol-5-yl]-1-methyl-3-piperidin-1-yl-methyl-pyrrolidin-2-one hydrochloride

A solution of 5-[2-(2-chloro-phenyl)-thiazol-5-yl]-1-methyl-3-methylene-pyrrolidine-2-one (1.6 g, 5.2 mmol) and piperidine (2.1 ml) is heated up to 75 °C C and stirred for 4 h. The reaction mixture is cooled to room temperature and evaporated *in vacuo.* Chromatography of the residue on silica gel with acetic acid ethyl ester/ethanol/conc. aq. NH₃ (9:1:0.1) yields the racemic title compound as an oil (1.48 g). The racemic mixture is resolved on Chiralpak AS (Daicel Chiral Technologies, Inc., Exton, USA; chiral stationary phase) with n-hexane and isopropanol 3:1. The hydrochloride salt of the (+)-enantiomer is crystallized from isopropanol/ether, m.p. 178-183 °C, [α]²⁰_{D} positive (c=0.5, HCl 1M). MS for C₂₀H₂₄ClN₃OS (ESI) MH⁺ m/z 390/392.

The starting material is prepared as follows:

### Preparation of 5-[2-(2-chloro-phenyl)-thiazol-5-yl]-1-methyl-3-methylene-pyrrolidine-2-one:

a) A solution of 2-bromo-thiazole (3.5 g, 21.3 mmol), 2-chlorobenzeneboronic acid (4.2 g, 26.8 mmol), 2 M aqueous Na₂CO₃ solution (20 ml) and ethanol (7 ml) in toluene (50 ml) is degassed and flushed with argon before Pd(OAc)₂ (145 mg) and triphenylphosphine (565 mg) are added. The mixture is stirred at 100 °C for 4 h. After addition of charcoal the mixture is filtered and the filtrate extracted with ethyl acetate. The organic phase is dried over sodium sulfate and evaporated. Chromatography on silica gel with cyclohexane/acetic acid ethyl ester (10:1) yields 2-(2-chloro-phenyl)-thiazole (3.4 g).
b) A 2 M solution of LDA in THF (13.9 ml, 27.8 mmol) is added at -70 °C under argon to a solution of 2-(2-chloro-phenyl)-thiazole (4.2 g, 21.5 mmol) in absolute THF (160 ml) and stirred at -65 °C for 30 min. Subsequently, morpholine-4-carboxaldehyde (3.2 g, 27.8 mmol) is added dropwise and stirred at -65 °C. After 4 h the reation mixture is quenched with ice in 1 M HCl and the mixture is extracted with ether. The organic phase is dried over sodium sulfate and evaporated. The residue is crystallized from cyclohexane to yield 2-(2-chloro-phenyl)-thiazole-5-carb-aldehyde (3.25 g), m.p. 95-97 °C.
c) A mixture of 2-(2-chloro-phenyl)-thiazole-5-carbaldehyde (1.0 g, 4.5 mmol) and 40% aqueous solution of methylamine (0.8 ml) is stirred at 80 °C for 45 min. The reaction mixture is cooled to room temperature and extracted with ether. The organic phase is dried over sodium sulfate and evaporated to yield [2-(2-chloro-phenyl)-thiazol-5-ylmethylene]-methylamine (1.0 g), m.p. 81-83 °C.
d) 2-Bromomethyl-acrylic acid methyl ester (4.1 g, 22.9 mmol) is added slowly under cooling (internal temperature below 30 °C) to a mixture of *activated* zinc powder and absolute THF (20 ml). After stirring of the mixture at room temperature for 1 h a solution of [2-(2-chlorophenyl)-thiazol-5-ylmethylene]-methyl-amine (1.8 g, 7.6 mmol) in THF (10 ml) is added slowly. After 30 min, the reaction mixture is quenched with saturated NH₄Cl solution (15 ml) and extracted with acetic acid ethyl ester. The organic phase is dried over sodium sulfate, filtered and evaporated. Chromatography on silica gel with ethyl acetate yields the racemic title compound (2.55 g), m.p. 96-105 °C.

The following compounds of formula I are prepared analogously to example F1:

### Example F2: (-)-cis-5-[2-(2-Chloro-phenyl)-thiazol-5-yl]-1-methyl-3-piperidin-1-yl-methyl-pyrrolidin-2-one hydrochloride

m.p. 171-176 °C, [α]²⁰_{D} negative (c=0.5, HCl 1M). MS for C₂₀H₂₄ClN₃OS (ESI) MH⁺ m/z 390/392.

### Example F3: (+)-cis-5-[2-(3-Chloro-thiophen-2-yl)-thiazol-5-yl]-1-methyl-3-piperidin-1-ylmethyl-pyrrolidin-2-one hydrochloride

m.p. 215-220 °C, [α]²⁰_{D} positive (c=0.5, HCl 1M). MS for C₁₈H₂₂ClN₃OS₂ (ESI) MH⁺ m/z 396/398.

### Example F4: (-)-cis-5-[2-(3-Chloro-thiophen-2-yl)-thiazol-5-yl]-1-methyl-3-piperidin-1-ylmethyl-pyrrolidin-2-one hydrochloride

m.p. 207-210 °C, [α]²⁰_{D} negative (c=0.5, HCl 1M). MS for C₁₈H₂₂ClN₃OS₂ (ESI) MH⁺ m/z 396/398.

### Example F5: (+)-cis-5-(2-Benzo[1,2,5]oxadiazol)-5-yl-thiazol-5-yl)-1-methyl-3-piperidin-1-ylmethyl-pyrrolidin-2-one hydrochloride

m.p. 129-135 °C, [α]²⁰_{D} positive (c=0.5, HCl 1M). MS for C₁₈H₂₃N₅O₂S (ESI) MH⁺ m/z 398.

### Example F6: (-)-cis-5-(2-Benzo[1,2,5]oxadiazol-5-yl)-thiazol-5-yl)-1-methyl-3-piperidin-1-ylmethyl-pyrrolidin-2-one hydrochloride

m.p. 123-129 °C, [α]²⁰_{D} negative (c=0.5, HCl 1M). MS for C₁₈H₂₃N₅O₂S (ESI) MH⁺ m/z 398.

### Examples F7 to F28:

The following compounds of formula (F) in cis-form regarding the substituents 5-R₂**-thiazol-2-yl and 1-piperidinylmethyl at the pyrrolidinone ring are prepared according to methods as described herein, especially in the preceding Examples:

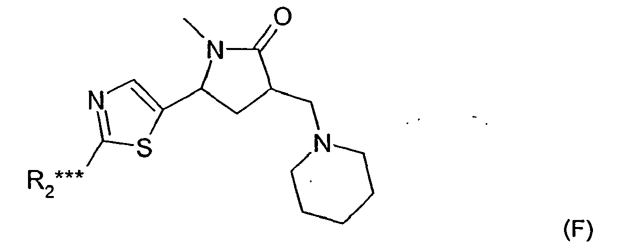

| Example | R₂*** |
|---|---|
| F7 | Phe |
| F8 | 2-Cl-Phe |
| F9 | 2-F-Phe |
| F10 | 2-F₃C-Phe |
| F11 | 4-F₃C-Phe |
| F12 | 2-Cl,6-Cl-Phe |
| F13 | 2-Cl,4-Cl-Phe |
| F14 | 2-Cl,3-Cl-Phe |
| F15 | 2-Cl,5-Cl-Phe |
| F16 | 4-MeO-Phe |
| F17 | thiophen-2-yl |
| F18 | 3-Cl-thiophen-2-yl |
| F19 | thiophen-3-yl |
| F20 | pyridin-3-yl |
| F21 | benzofuran-2-yl |
| F22 | 3,4-(-O-CH₂-O-)Phe |
| F23 | 3,4-(=N-O-N=)Phe |
| F24 | 3,4-(=N-S-N=)Phe |
| F25 | 2-Me-thiazol-4-yl |
| F26 | 2-Cl-benzyl |
| F27 | 2,2-di-(Cl)-benzyl- |
| F28 | 3,4-(=N-S-N=)Phe |

Any of these compounds is a mixture of isomers or a pure enantiomer (especially the isomer most active as agonist in a test system described above). Where compounds are already described specifically, here the isomers or isomer mixtures not mentioned before are meant.

### Example G1: (±)-cis-5-(5-(2H-1,3-Benzodioxol-5-yl)-1-methyl-1H-imidazol-2-yl)-1-methyl-3-piperidin-1-ylmethyl-pyrrolidin-2-one

A mixture of piperidine (560 µl, 5.6 mmol) and 5-(5-(2*H*-1,3-benzodioxol-5-yl)-1-methyl-1H-imidazol-2-yl)-1-methyl-3-methylene-pyrrolidin-2-one (440 mg, 1.4 mmol) is heated at 65 °C for a period of 24 h. The mixture is evaporated and the residue (353 mg) purified by thick layer chromatography (AcOEt / EtOH / NH₃, 9:1:0.1) to afford 38.2 mg of the desired compound. MS for (ESI) MH⁺ m/z 397.

The starting material is prepared as follows:

### Preparation of 5-(5-(2H-1,3-benzodioxol-5-yl)-1-methyl-1H-imidazol-2-yl)-1-methyl-3-methylene-pyrrolidin-2-one:

a) A mixture of 5-benzo[1,3]dioxol-5-yl-1-methyl-1H-imidazole-2-carbaldehyde (1.5 g, 6.3 mmol) in ethanol (10 ml) and a 33% ethanolic solution of methylamine (4 ml, 31.7 mmol) is heated to reflux for 8 h. The mixture is evaporated and the residue is recrystallized from ether to afford [1-(5-(2*H*-1,3-benzodioxol-5-yl)-1-methyl-1H-imidazol-2-yl)-meth-(E)-ylidene]-methyl-amine (1.34 g).
b) 2-Bromomethyl-acrylic acid methyl ester (2.96 g, 16.5 mmol) in absolute THF (3 ml) is added to a suspension of *activated* zinc powder in absolute THF (3 ml). The addition is maintained at a rate so that the reaction temperature does not surpass 30 °C. The mixture is stirred for one additional h at room temperature. [1-(5-(2*H*-1,3-benzodioxol-5-yl)-1-methyl-1H-imidazol-2-yl)-meth-(E)-ylidene]-methyl-amine (1.3 g, 5.5 mmol) dissolved in THF (3 ml) is added slowly to the zinc reagent. After 60 min saturated NH₄Cl solution (3.5 ml) is added under ice cooling and the mixture is extracted with acetic acid ethyl ester. The organic phase is dried and evaporated. Chromatography on silica gel with CH₂Cl₂/MeOH 95:5 affords 440 mg of the title compound.
c) General synthesis of the imidazole system (method A) (1-methylimidazole derivatives):

### 5-(2H-1,3-Benzodioxol-5-yl)-1-methyl-1H-imidazole

(i) A 40% aqueous solution methylamine (4.14 g ,53.28 mmol) in ethanol (67 ml) is slowly added to a solution of piperonal (5 g, 33.3 mmol) in ethanol (33 ml). The reaction mixture is heated at 83 °C C for 16 h. The solution is evaporated and the residue extracted with methylene chloride. The organic phase is dried over magnesium sulfate. The organic layer is evaporated to afford crude [1-(2*H*-1,3-benzodioxol-5-yl)-meth-(E)-ylidene]-methyl-amine (4.98 g) which is used directly for the next step.
(ii) Potassium carbonate (8.4 g, 60.8 mmol) is added to an emulsion of TosMIC (11.9 g, 60.8 mmol) and [1-(2*H*-1,3-benzodioxol-5-yl)-meth-(E)-ylidene]-methyl-amine (4.9 g, 30.4 mmol). The suspension is stirred at room temperature for 30 min and is then heated to reflux for 16 h. The solution is evaporated and the residue is taken up in methylene chloride, washed with brine and dried over magnesium sulfate. The crude product is partially purified by chromatography on silica gel with CH₂Cl₂/MeOH 92:2 to afford after recrystallization from ether 2.9 g of the title compound.
(iii) 5-(2*H*-1,3-Benzodioxol-5-yl)-1-methyl-1H-imidazole-2-carbaldehyde
   A 1.6 N solution of n-butyllithium in THF (13.5 ml, 21.5 mmol) is slowly added to a solution of 5-(2*H*-1,3-benzodioxol-5-yl)-1-methyl-1H-imidazole (2.9 g, 14.3 mmol) in THF (30 ml) at -78 °C. The red solution is then stirred for 1.5 h after which DMF (1.15 g, 15.8 mmol) is added. The solution is allowed to warm to room temperature and is stirred for 1 h. Ether is added to the reaction mixture. The organic phase is washed first with 1 M HCl and then with a saturated solution of NaHCO₃. The crude product is crystallized from ether to afford 1.46 g of the title compound.

The following compound of the formula I are prepared in an analogous manner:

### Example G2: (±)-cis-5-[5-(2-Chloro-phenyl)-1-methyl-1H-imidazol-2-yl]-1-methyl-3-piperidin-1-ylmethyl-pyrrolidin-2-one

MS (ESI) MH⁺ m/z 387.1

### Example G3: (±)-cis-5-(5-(2H-1,3-Benzodioxol-5-yl)-1-benzyl-1H-imidazol-2-yl)-1-methyl-3-piperidin-1-ylmethyl-pyrrolidin-2-one

MS (ESI) MH⁺- piperidine m/z 388.3

### Example G4: (±)-cis-5-(4-(2H-1,3-Benzodioxol-5-yl)-1-benzyl-1H-imidazol-2-yl)-1-methyl-3-piperidin-1-ylmethyl-pyrrolidin-2-one

MS (ESI) MH⁺ m/z 473.6

### Example G5: (±)-cis-5-[1-Benzyl-4-(2-chloro-phenyl)-1H-imidazol-2-yl]-1-methyl-3-piperidin-1-ylmethyl-pyrrolidin-2-one

MS (ESI) MH⁺ m/z 463.6

### Example G6: (±)-cis-5-[4-(2-Chloro-phenyl)-1-(2,4-dimethoxy-benzyl)-1H-imidazol-2-yl]-1-methyl-3-piperidin-1-ylmethyl-pyrrolidin-2-one

MS (ESI) MH⁺ m/z 523.5

### Example G7: (±)-cis-5-[1-(2,4-Dimethoxy-benzyl)-4-(3-methoxy-phenyl)-1H-imidazol-2-yl]-1-methyl-3-piperidin-1-ylmethyl-pyrrolidin-2-one

MS (ESI) MH⁺-piperidine m/z 434.1

Disubstituted imidazoles are prepared as follows:

### Example G8: (±)-cis-5-(5-(2H-1,3-Benzodioxol-5-yl)-1H-imidazol-2-yl)-1-methyl-3-piperidin-1-ylmethyl-pyrrolidin-2-one

Ammonium formiate (29.4 mg, 0.465 mmol) is added in one portion to a suspension of palladium on charcoal (109 mg) and (3RS,5SR)-5-(5-(2*H*-1,3-benzodioxol-5-yl)-1-benzyl-1H-imidazol-2-yl)-1-methyl-3-piperidin-1-ylmethyl-pyrrolidin-2-one (44 mg, 0.093 mmol) in methanol (1ml). The resulting mixture is heated under reflux for 1 h, cooled to room temperature and filtered over Hyflo (diatomaceous earth; Celite Corporation, Lompoc, USA). The residue of the filtrate is purified by chromatography on silica gel with CH₂Cl₂/EtOH/ NH₄OH 92:8:1) to afford 9.2 mg of the desired compound. MS (ESI) MH⁺ m/z = 383.1

The starting material is prepared in analogy to 5-(5-(2*H*-1,3-benzodioxol-5-yl)-1-methyl-1H-imidazol-2-yl)-1-methyl-3-methylene-pyrrolidin-2-one in Example G1 but starting from 5-(2*H-*1,3-benzodioxol-5-yl)-1-benzyl-1H-imidazole which is prepared as follows:
a) General synthesis of the imidazole system (method B) (e.g. 1-benzylimidazole derivatives):
   (i) 5-(2*H*-1,3-Benzodioxol-5-yl)-1-benzyl-1H-imidazole
      NaCN (180 mg, 3.7 mmol) is added to a suspension of piperonal (5.5g, 36.6 mmol) and TosMIC (7 g, 35.9 mmol) in ethanol (100 ml) and the resulting mixture is stirred for 15 min at room temperature. The solvent is removed by evaporation and the residue is washed with a mixture of hexane/ether (1:1). The beige dried powder of 5-(2*H*-1 ,3-benzodioxol-5-yl)-4-(toluene-4-sulfonyl)-4,5-dihydro-oxazole (7.5 g) is mixed with benzylamine (9.3 g, 87 mmol) in xylene (108 ml) and heated at 137 °C for 16 h. The xylene is removed and the residue purified by chromatography (Ether/EtOH 99:1) to furnish 4-(2*H*-1,3-benzodioxol-5-yl)-1-benzyl-1H-imidazole (2.5 g).

### Example G9: (±)-cis-1-Methyl-5-(5-phenyl-1H-imidazol-2-yl)-3-piperidin-1-ylmethyl-pyrrolidin-2-one

This compound is prepared in the same manner starting from (3RS,5SR)-5-[1-benzyl-4-(2-chloro-phenyl)-1H-imidazol-2-yl]-1-methyl-3-piperidin-1-ylmethyl-pyrrolidin-2-one. MS (ESI) MH⁺ m/z = 339.2

### Example G10: (±)-cis-5-[4-(2-Chloro-phenyl)-1H-imidazol-2-yl]-1-methyl-3-piperidin-1-ylmethyl-pyrrolidin-2-one

Trifluoroacetic acid (1.31 ml, 17 mmol) is added to a solution of (3RS,5SR)-5-[4-(2-chlorophenyl)-1-(2,4-dimethoxy-benzyl)-1H-imidazol-2-yl]-1-methyl-3-piperidin-1-ylmethyl-pyrrolidin-2-one (177 mg, 0.34 mmol) in anisole (1.8 ml). The solution is stirred at 90 °C for 24 h after which an additional portion of trifluoroacetic acid (0.5 ml) is added. The reaction is stirred for another 3 h. The mixture is evaporated and the residue treated with saturated solution of sodium carbonate followed by extraction with methylene chloride. The organic layer is dried and evaporated to afford 589 mg of a crude product which is purified by chromatography on silica gel with CH₂Cl₂/EtOH/NH₄OH 92:8:1 to afford 26 mg of the desired compound. MS (ESI) MH⁺ m/z 373.2

(3RS,5SR)-5-[4-(2-chloro-phenyl)-1-(2,4-dimethoxy-benzyl)-1H-imidazol-2-yl]-1-methyl-3-piperidin-1-ylmethyl-pyrrolidin-2-one is prepared in analogy to (3RS,5SR)-5-(5-benzo[1,3]dioxol-5-yl-1-benzyl-1H-imidazol-2-yl)-1-methyl-3-piperidin-1-ylmethyl-pyrrolidin-2-one in Example G8. Instead of piperonal, 2-chlorobenzaldehyde is used.

### Example G11: (±)-cis-5-[4-(3-Methoxy-phenyl)-1H-imidazol-2-yl]-1-methyl-3-piperidin-1-ylmethyl-pyrrolidin-2-one

This compound is prepared is prepared in the same manner starting from (3RS,5SR)-5-[1-(2,4-dimethoxy-benzyl)-4-(3-methoxy-phenyl)-1H-imidazol-2-yl]-1-methyl-3-piperidin-1-ylmethyl-pyrrolidin-2-one. MS (ESI) MH⁺ m/z 369.0

### Example H1: (+)-cis-1-Methyl-3-piperidin-1-ylmethyl-5-(4'-trifluormethyl-biphenyl-4-yl)-pyrrolidin-2-one hydrochloride

A solution of (+)-cis-5-(4-bromo-phenyl)-1-methyl-3-piperidin-1-ylmethyl-pyrrolidin-2-one (Example A7) (100 mg, 0.28 mmol), 4-trifluoromethylbenzeneboronic acid (81 mg, 0.43 mmol), 2 M aqueous Na₂CO₃ solution (0.4 ml) and ethanol (0.15 ml) in toluene (3 ml) is degassed and flushed with argon before both Pd(OAc)₂ (2 mg) and triphenylphosphine (7.5 mg) are added. The mixture is stirred at 100 °C for 2 h. After addition of charcoal the mixture is filtered and the filtrate is extracted with ethyl acetate. The organic phase is dried over sodium sulfate and evaporated. Chromatography on silica gel with acetic acid ethyl ester/ethanol/conc. aq. NH₃ (9:1:0.1) yields the title compound (50 mg), m.p. 134-140 °C. MS for C₂₄H₂₇F₃N₂O (ESI) MH⁺ m/z 417.

The following compounds of formula H are prepared analogously to example H1:

### Example H2: (+)-cis-5-(2'-Chloro-biphenyl-4-yl)-1-methyl-3-piperidin-1-ylmethyl-pyrrolidin-2-one hydrochloride

m.p. 134-139 °C. MS for C₂₃H₂₇ClN₂O (ESI) MH⁺ m/z 383/385. [α]²⁰_{D} positive (c 1.0, water).

### Example H3: (-)-cis-5-(2'-Chloro-biphenyl-4-yl)-1-methyl-3-piperidin-1-ylmethyl-pyrrolidin-2-one hydrochloride

m.p. 130-135 °C. MS for C₂₃H₂₇CIN₂O (ESI) MH⁺ m/z 383/385, [α]²⁰_{D} negative (c 1.0, water).

### Example H4: cis-5-(2',6'-Dichloro-biphenyl-4-yl)-1-methyl-3-piperidin-1-ylmethyl-pyrrolidin-2-one

MS for C₂₃H₂₆Cl₂N₂O (ESI) MH⁺ m/z 417/419.

### Example H5: cis-5-(4-(Benzo[1,3]dioxol-5-yl)-phenyl)-1-methyl-3-piperidin-1-ylmethyl-pyrrolidin-2-one

m.p. 110-112 °C. MS for C₂₄H₂₈N₂O₃ (ESI) MH⁺ m/z 393.

### Example H6: cis-5-(4-Benzo[1,2,5]oxadiazol-5-yl-phenyl)-1-methyl-3-piperidin-1-ylmethyl-pyrrolidin-2-one

MS for C₂₃H₂₆N₄O₂ (ESI) MH⁺ m/z 391.

### Examples H7 to H36:

The following compounds of formula (H) in cis-form regarding the substituents 5-R₂***-Phe and 3-(1-piperidinyl or 4-morpholinyl)-CH₂- at the pyrrolidinone ring are prepared according to methods as described herein, especially in the preceding Examples:

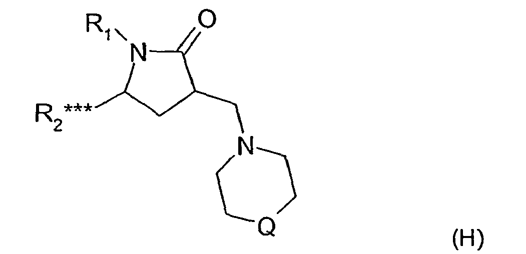

| Example | R₂*** | R₁ | Q |
|---|---|---|---|
| H7 | 4-Phe-Phe | Me | O |
| H8 | 4-Phe-Phe | Me | CH₂ |
| H9 | 4-(2-F-Phe)-Phe | Me | CH₂ |
| H10 | 4-(2-Me-Phe)-Phe | Me | CH₂ |
| H11 | 4-(2-Et-Phe)-Phe | Me | CH₂ |
| H12 | 4-(2-F₃C-Phe)-Phe | Me | CH₂ |
| H13 | 4-(2-Me-O-Phe)-Phe | Me | CH₂ |
| H14 | 4-(2-Me-S-Phe)-Phe | Me | CH₂ |
| H 15 | 4-(3-Cl-Phe)-Phe | Me | CH₂ |
| H16 | 4-(4-Cl-Phe)-Phe | Me | CH₂ |
| H17 | 4-(3-Br-Phe)-Phe | Me | CH₂ |
| H18 | 4-(4-F-Phe)-Phe | Me | CH₂ |
| H19 | 4-(4-Me-Phe)-Phe | Me | CH₂ |
| H20 | 4-(4-F₃C-Phe)-Phe | Me | CH₂ |
| H21 | 4-(4-Me-O-Phe)-Phe | Me | CH₂ |
| H22 | 4-(2-Cl,6-Cl-Phe)-Phe | Me | CH₂ |
| H23 | 4-(2-Cl,4-Cl-Phe)-Phe | Me | CH₂ |
| H24 | 4-(3,4-(-O-CH₂-O-)Phe)-Phe | Me | CH₂ |
| H25 | 4-(3,4-(-O-CF₂-O-)Phe)-Phe | Me | CH₂ |
| H26 | 4-(3,4-(=N-O-N=)Phe)-Phe | Me | CH₂ |
| H27 | 3-(Phe)-Phe | Me | CH₂ |
| H28 | 3-(2-Cl-Phe)-Phe | Me | CH₂ |
| H29 | 3-(3-Cl-Phe)-Phe | Me | CH₂ |
| H30 | 3-(4-Cl-Phe)-Phe | Me | CH₂ |
| H31 | 4-(2-Cl-Phe)-Phe | Me | CH₂ |
| H32 | 4-(2-Cl-Phe)-Phe | benzyl | CH₂ |
| H33 | quinolin-5-yl | Me | CH₂ |
| H34 | quinolin-8-yl | Me | CH₂ |
| H35 | 2-naphthyl | Me | CH₂ |
| H36 | 3,4-(=N-O-N=)Phe | Me | CH₂ |

Any of these compounds is a mixture of isomers or a pure enantiomer (especially the isomer most active as agonist in a test system described above). Where compounds are already described specifically, here the isomers or isomer mixtures not mentioned before are meant.

## Claims

1. A compound of the formula I, 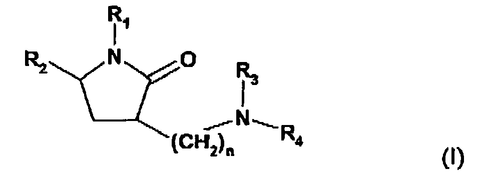 wherein
R₁ is C₁-C₇-alkyl, especially methyl;
R₂ is phenyl that is unsubstituted or substituted by one or more, especially up to three, substituents independently selected from C₁-C₇-alkyl, C₃-C₈-cycloalkyl, unsubstituted, halo and/or C₁-C₇-alkoxy-substituted phenyl- or naphthyl-C₁-C₇-alkyl, phenyl or (1- or 2-) napthyl, each phenyl or naphthyl of which is preferably present in the p-position to the bond with which the substituted phenyl is bound to the rest of the molecule and is unsubstituted or substituted with one or more, especially up to three, substituents selected from C₁-C₇-alkyl, halo-C₁-C₇-alkyl, C₁-C₇-alkoxy, halo-C₁-C₇-alkoxy, phenoxy, C₁-C₇-alkylthio, nitro, cyano, halo and a bivalent ligand that is bound to two adjacent carbon atoms in the aryl ring (thus forming a ring with the atoms to which it is bound) where the bivalent ligand is selected from the group consisting of -O-CH₂-O-, -O-CH₂-CH₂-O-, -CH₂-O-CH₂-, -CH₂-CH₂-CH₂-, -CH₂-CH₂-CH₂-CH₂-, -O-CF₂O-, =N-O-N= and =N-S-N=; hydroxy; hydroxy-C₁-C₇-alkyl, C₁-C₇-alkoxy, phenoxy, G₁-G₇-alkanoyloxy, halo, halo-C₁-C₇alkyl, such as trifluoromethyl; nitro; amino; N-mono- or N,N-di-(C₁-C₇-alkyl)amino, C₁-C₇-alkanoylamino, C₁-C₇-alkanoyl, carboxy; C₁-C₇-alkoxycarbonyl, carbamoyl, N-mono- or N,N-di-(C₁-C₇-alkyl)carbamoyl, sulfamoyl; C₁-C₇-alkylsulfonyl, a bivalent ligand that is bound to two adjacent carbon atoms in the phenyl or naphthyl ring (thus forming a ring with the atoms to which it is bound) where the bivalent ligand is selected from the group consisting of -O-CH₂-O-,-O-CH₂-CH₂-O-, -CH₂-O-CH₂-, -CH₂-CH₂-CH₂-, -CH₂-CH₂CH₂-CH₂-, -O-CF₂-O-, =N-O-N= and =N-S-N=; and unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo and C₁-C₇-alkyl, for example pyrrolidinyl, thiophenyl, thiazolyl, pyridinyl, benzofuranyl, indolyl, benzothiophenyl and benzothiazolyl; C₃-C₈-cycloalkyl that is substituted, preferably at a ring carbon different from that which binds to the central pyrrolidinone ring in formula I, by phenyl that is unsubstituted or substituted by one or more, especially up to three, substituents independently selected from those just mentioned for substituted phenyl R₂, especially phenyl or halo-substituted phenyl, such as fluoro-, chloro- or bromophenyl;
unsubstituted or substituted heterocyclyl with 5 to 7 ring atoms which is unsaturated, partially saturated or saturated, and has one to three heteroatoms selected from O, N (or NH) and S as such or annealed to benzo, such as an unsubstituted or substituted moiety selected from pyrrolidinyl, imidazolyl, thiophenyl, thiazolyl, pyridinyl, indolyl, quinolinyl, benzofuranyl, benzothiophenyl and benzothiazolyl, whereby heterocyclyl is unsubstituted or substituted by up to three moieties independently selected from halo, C₁-C₇-alkyl; unsubstituted or substituted phenyl or unsubstituted or substituted naphthyl, in each case with up to three substituents independently selected from the group consisting of C₁-C₇-alkyl, phenyl-C₁-C₇-alkyl which is unsubstituted or substituted at the phenyl ring by up to three halo substituents, C₁-C₇-alkoxy, halo, halo-C₁-C₇-alkyl, halo-C₁-C₇-alkoxy, cyano; nitro; a bivalent ligand that is bound to two adjacent carbon atoms in the phenyl ring (thus forming a ring with the atoms to which it is bound) where the bivalent ligand is selected from the group consisting of -O-CH₂-O-,-O-CH₂-CH₂-O-, -CH₂-O-CH₂-, -CH₂-CH₂-CH₂-, -CH₂-CH₂-CH₂-CH₂-, -O-CF₂-O-, =N-O-N= and =N-S-N=; unsubstituted or substituted phenyl-C₁-C₇alkyl wherein the substituents are up to three substituents independently selected from halo, such as chloro, and C₁-C₇-alkoxy, such as methoxy,; and unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo and C₁-C₇-alkyl;
substituted C₁-C₇-alkyl, preferably C₂-C₄-alkyl, that is substituted by unsubstituted or substituted phenyl or naphthyl, wherein the substituents, preferably one or more, especially up to three, are independently selected from halo, C₁-C₇-alkyl, halo-C₁-C₇-alkyl, C₁-C₇-alkoxy, halo-C₁-C₇-alkoxy, hydroxyl, hydroxyl-C₁-C₇-alkyl, nitro, cyano, amino, N-mono- or N,N-di-C₁-C₇-alkylamino, N-C₁-C₇-alkanoylamino, C₁-C₇-alkanoyloxy, C₁-C₇-alkanoyl, carboxy, C₁-C₇-alkoxycarbonyl, carbamoyl, N-mono- or N,N-di-(C₁-C₇-alkyl)-carbamoyl, such as N,N-di(ethyl)-carbamoyl, sulfamoyl, phenyl; from a bivalent ligand that is bound to two adjacent carbon atoms in the aryl ring (thus forming a ring with the atoms to which it is bound) where the bivalent ligand is selected from the group consisting of -O-CH₂-O-, -O-CH₂-CH₂-O-, -CH₂-O-CH₂-, -CH₂-CH₂-CH₂-, -CH₂CH₂-CH₂-CH₂-, -O-CF₂-O-, =N-O-N= and =N-S-N=; and from unsubstituted or substituted heterocyclyl selected from pyridinyl, thiazolyl, indolyl, C₁-C₇-alkyl-indolyl, benzofuranyl, benzothiophenyl, and benzothiazolyl, such as 2-benzothiazolyl;
unsubstituted or substituted C₁-C₇-alkenyl, preferably C₂C₄-alkenyl, especially vinyl, that is terminally substituted by unsubstituted or substituted phenyl with up to three halo substituents, and carries a hydrogen or a C₁-C₇-alkyl in the 1-position; whereby the double bond, with respect to the terminal substituents and the central pyrrolidinone ring in formula I, is in the cis,trans- or preferably in the trans- or most preferably in the cis-configuration;
or unsubstituted or substituted C₂-C₄-alkynyl, especially ethynyl that is substituted (especially terminally) either by unsubstituted or substituted phenyl or naphthyl, wherein the substituents, preferably one or more, especially up to three, are independently selected from halo, C₁-C₇-alkyl, C₁-C₇-alkoxy, halo-C₁-C₇-alkyl, hydroxyl, hydroxyl-C₁-C₇-alkyl, cyano, amino, N-mono- or N,N-di-C₁-C₇-alkylamino, N-C₁-C₇-alkanoylamino, C₁-C₇-alkanoyloxy, C₁-C₇-alkanoyl, carboxy, C₁-C₇-alkoxycarbonyl, carbamoyl, N-mono- or N,N-di-(C₁-C₇-alkyl)-carbamoyl, sulfamoyl, phenyl, and a bivalent ligand that is bound to two adjacent carbon atoms in the aryl ring (thus forming a ring with the atoms to which it is bound) where the bivalent ligand is selected from the group consisting of -O-CH₂-O-, -O-CH₂CH₂-O-, -CH₂-O-CH₂-, -CH₂-CH₂-CH₂-, -CH₂-CH₂-CH₂-CH₂-, -O-CF₂-O-, =N-O-N= and =N-S-N=; or by unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo and C₁-C₇-alkyl, especially pyridin-2-yl or pyridin-3-yl, thiazolyl, indolyl, C₁-C₇-alkyl-indolyl, benzofuranyl, benzothiophenyl, and benzothiazolyl;
R₃ and R₄ are C₁-C₇-alkyl, preferably ethyl, or together with the binding nitrogen form ring with (including the binding nitrogen) 3 to 10 ring atoms, more preferably an N-piperidinyl (very preferred), an N-pyrrolidinyl or an N-azepanyl ring; and
n is 1.

2. A compound of the formula I according to claim 1, wherein
R₁ is methyl;
R₂ is phenyl that is unsubstituted or substituted by one or more, especially up to three, substituents independently selected from C₁-C₇-alkyl; C₁-C₇alkoxy; C₃-C₈-cycloalkyl, phenyl or (1- or 2-) napthyl, each phenyl or naphthyl of which is preferably present in the p-position to the bond with which the substituted phenyl is bound to the rest of the molecule and is unsubstituted or substituted with one or more, especially up to three, substituents selected from halo, a bivalent ligand that is bound to two adjacent carbon atoms in the aryl ring (thus forming a ring with the atoms to which it is bound) where the bivalent ligand is selected from the group consisting of -O-CH₂-O-,-O-CH₂-CH₂-O-, -CH₂-O-CH₂-, -CH₂CH₂-CH₂-, -CH₂-CH₂-CH₂-CH₂-, -O-CF₂O-, =N-O-N= and =N-S-N=: pyrrolidinyl and thiophenyl, preferably in the 4-position of the phenyl to which it is bound as substituent,
C₃-C₈-cycloalkyl, especially cyclopentyl, cyclohexyl or preferably cyclopropyl that is substituted, preferably at a ring carbon different from that which binds to the central pyrrolidinone ring in formula I, especially in 2-position, by phenyl or halo-substituted phenyl;
imidazolyl, thiophenyl or thiazolyl, each of which is unsubstituted or substituted by up to three moieties independently selected from unsubstituted or substituted phenyl or unsubstituted or substituted naphthyl, phenyl or naphthyl if substituted then in each case with up to three substituents independently selected from the group consisting of C₁-C₇-alkyl, C₁-C₇-alkoxy, halo, halo-C₁-C₇-alkyl, cyano, nitro, a bivalent ligand that is bound to two adjacent carbon atoms in the phenyl ring (thus forming a ring with the atoms to which it is bound) where the bivalent ligand is selected from the group consisting of -O-CH₂-O-, -O-CH₂-CH₂-O- -CH₂-O-CH₂-, -CH₂-CH₂-CH₂-, -CH₂CH₂-CH₂-CH₂-, -O-CF₂-O-, =N-O-N= and =N-S-N=, thiophenyl, halo-thiophenyl, and quinolinyl;
unsubstituted or substituted vinyl that is terminally substituted by unsubstituted or substituted phenyl with up to three halo substituents and carries a hydrogen or a C₁-C₇-alkyl in the 1-position; whereby the double bond, with respect to the terminal substituents and the central pyrrolidinone ring in formula I, is in the cis,trans- or preferably in the trans- or most preferably in the cis-configuration; or
unsubstituted or substituted ethynyl that is substituted either by unsubstituted or substituted phenyl, wherein the substituents, preferably one or more, especially up to three, are independently selected from halo, C₁-C₇-alkyl, C₁-C₇-alkoxy, halo-C₁-C₇-alkyl, hydroxyl, hydroxyl-C₁-C₇-alkyl, cyano, amino, N-mono- or N,N-di,C₁-C₇-alkylamino, N-C₁-C₇-alkanoylamino, C₁-C₇-alkenoyloxy, C₁-C₇-alkanoyl, C₁-C₇-alkoxycarbonyl, carbamoyl, N-mono- or N,N-di-(C₁-C₇-alkyl)-carbamoyl, sulfamoyl and a bivalent ligand that is bound to two adjacent carbon atoms in the aryl ring (thus forming a ring with the atoms to which it is bound) where the bivalent ligand is selected from the group consisting of -O-CH₂-O-,-O-CH₂-CH₂-O-, -CH₂-O-CH₂-, -CH₂-CH₂-CH₂-, -CH₂-CH₂-CH₂-CH₂-, -O-CF₂-O-, =N-O-N= and N-S-N=; or by unsubstituted or substituted heterocyclyl with 5 to 7 ring atoms which is unsaturated, partially saturated or saturated, has one to three heteroatoms selected from O, N (or NH) and S as such or annealed to benzo, and is unsubstituted or substituted by up to three moieties independently selected from halo and C₁-C₇-alkyl, especially pyridin-2-yl or pyridin-3-yl, thiazolyl, indolyl, C₁-C₇-alkyl-indolyl, benzofuranyl, benzothiophenyl or benzothiazolyl;
R₃ and R₄ together with the binding nitrogen form an N-piperidinyl an N-pyrrolidinyl or an N-azepanyl ring; and
n is 1.

3. A compound of the formula I according to claim 1, wherein
R₁ is C₁-C₇-alkyl, especially methyl;
R₂ is C₁-C₇-alkyl, preferably C₂-C₄-alkyl, that is substituted by unsubstituted or substituted phenyl or naphthyl, wherein the substituents, preferably one or more, especially up to three, are independently selected from halo, C₁-C₇-alkyl, halo-C₁-C₇-alkC₁,C₇-alkyl, C₁-C₇-alkoxy, halo-C₁-C₇-alkoxy, hydroxyl, hydroxyl-C₁-C₇-alkyl, nitro, cyano, amino, N-mono- or N.N-di-C₁-C₇-alkylamino, N-C₁-C₇-alkanoylamino, C₁-C₇-alkanoyloxy, C₁-C₇-alkanoyl, carboxy, C₁-C₇-alkoxycarbonyl, carbamoyl, N-mono- or N,N-di-(C₁-C₇-alkyl)-carbamoyl, sulfamoyl, phenyl; from a bivalent ligand that is bound to two adjacent carbon atoms in the aryl ring (thus forming a ring with the atoms to which it is bound) where the bivalent ligand is selected from the group consisting of-O-CH₂-O-, -O-CH₂-CH₂-O-, -CH₂-O-CH₂,-CH₂-CH₂-CH₂-, -CH₂-CH₂-CH₂-CH₂-, -O-CF₂-O-, =N-O-N= and =N-S-NN=; and from unsubstituted or substituted heterocylyl selected from pyridinyl, thiazolyl, indolyl, C₁-C₇-alkyl-indolyl, benzofuranyl, benzothiophenyl, and benzothiazolyl, such as 2-benzothiazolyl;
R₃ and R₄ together with the binding nitrogen form a ring with (including the binding nitrogen) 4 to 8 ring atoms, more preferably an N-piperidinyl (very preferred), an N-pyrrolidinyl or an N-azepanyl ring; and
n is 1.

4. A compound of the formula I according to claim I, selected from the group of compounds of the formula (D) represented in the following table: 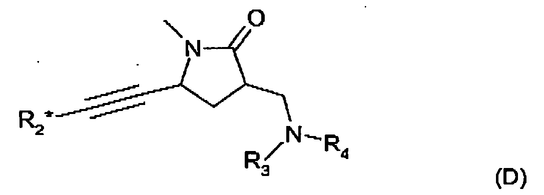
| Compound | R₂* | R₃ | R₄ |
|---|---|---|---|
| D10 | Phe | together | -(CH₂)₅- |
| D11 | 2-Cl-Phe | together | -(CH₂)₅- |
| D12 | 2-Br-Phe | together | --(CH₂)₅- |
| D13 | 2-F-Phe | together | -(CH₂)₅- |
| D14 | 2-Me-Phe | together | -(CH₂)₅- |
| D15 | 2-MeO-Phe | together | -(CH₂)₅- |
| D16 | 2-F₃C-Phe | together | -(CH₂)₅- |
| D17 | 2-CN-Phe | together | -(CH₂)₅- |
| D18 | 2-hydroxy-Phe | together | -(CH₂)₅- |
| D19 | 3-Cl-Phe | together | -(CH₂)₅- |
| D20 | 3-MeO-Phe | together | -(CH₂)₅- |
| D21 | 3-F₃C-Phe | together | -(CH₂)₅- |
| D22 | 3-(Me₂N)-Phe, | together | -(CH₂)₅- |
| D23 | 3-hydroxy-Phe | together | -(CH₂)₅- |
| D24 | 3-acetoxy-Phe | together | -(CH₂)₅- |
| D25 | 3-amino-Phe | together | -(CH₂)₅- |
| D26 | 3-acetylamino-Phe | together | -(CH₂)₅- |
| D27 | 3-hydroxymethyl-Phe | together | -(CH₂)₅- |
| D28 | 3-acetyl-Phe | together | -(CH₂)₅- |
| D30 | 3-ethoxycarbonyl-Phe | together | -(CH₂)₅- |
| D31 | 3-N,N-diethylcarbamoyl-Phe | together | -(CH₂)₅- |
| D32 | 3-sulfamoyl-Phe | together | -(CH₂)₅- |
| D33 | 4-Cl-Phe | together | -(CH₂)₅- |
| D34 | 4-F-Phe | together | -(CH₂)₅- |
| D35 | 4-Me-Phe | together | -(CH₂)₅- |
| D37 | 4-tert-butyl-Phe | together | -(CH₂)₅- |
| D38 | 3,4-(-O-CH₂-O-)Phe | together | -(CH₂)₅- |
| D39 | 3.4-(-O-CH₂-CH₂-O-)Phe | together | -(CH₂)₅- |
| D40 | 2,3-((-CH₂)₄-)Phe | together | -(CH₂)₅- |
| D41 | 3,4-((-CH₂)₄-)Phe | together | -(CH₂)₅- |
| D43 | 4-Phe-Phe | together | -(CH₂)₅- |
| D44 | 2-Cl,3-Cl-Phe | together | (CH₂)₅- |
| D45 | 2-F,5-F-Phe | together | -((CH₂)₅- |
| D46 | 2-Cl-,5-methyl-Phe | together | -(CH₂)₅- |
| D47 | 3-Me,5-Me-Phe | together | -(CH₂)₅- |
| D50 | 4-hydroxy-3-Me-Phe | together | -(CH₂)₅- |
| D51 | 2,3-(=N-S-N=)Phe | together | -(CH₂)₅- |
| D52 | 2,3-(-CH=CH-CH=CH-)Phe | together | -(CH₂)₅- |
| D53 | 5-indolyl | together | -(CH₂)₅- |
| D54 | 5-benzofuranyl | together | -(CH₂)₅- |
| D55 | 5-benzo[b]thiophenyl | together | -(CH₂)₅- |
| D56 | 2-pyridinyl | together | -(CH₂)₅- |
| D57 | 3-pyridinyl | together | -(CH₂)₅- |
| D58 | 2-thiazolyl | together | -(CH₂)₅- |
| D59 | 2-benzothiazolyl | together | -(CH₂)₅- |
in the form of a mixture of isomers or as a single isomer.

5. A compound of the formula I according to claim 1, selected from the group of compounds consisting of
cis-1-methyl-3-piperidin-1-ylmethyl-5-m-tolylethynyl-pyrrolidin-2-one;
cis-1-methyl-5-phenylethynyl-3-piperidin-1-ylmethyl-pyrrolidin-2-one;
cis-5-(2-chloro-phenylethynyl)-1-methyl-3-piperidin-1-ylmethyl-pyrrolidin-2-one;
cis-N-[2-(1-methyl-5-oxo-4-piperidin-1-ylmethyl-pyrrolidin-2-ylethynyl)-phenyl]-acetamide;
cis-1-methyl-5-(1-methyl-1H-indol-5-ylethynyl)-3-piperidin-1-ylmethyl-pyrrolidin-2-one;
cis-5-benzofuranyl-5-ylethynyl-1-methyl-3-piperidin-1-ylmethyl-pyrrolidin-2-one;
(+)-cis-1-methyl-3-piperidin-1-ylmethyl-5-m-tolylethynyl-pyrrolidin-2-one;
(+)-cis-1-methyl-5-phenylethynyl-3-piperidin-1-ylmethyl-pyrrolidin-2-one;
(+)-cis-5-(2-chloro-phenylethynyl)-1-methyl-3-piperidin-1-ylmethyl-pyrrolidin-2-one;
(+)-cis-N-[2-(1-methyl-5-oxo-4-piperidin-1-ylmethyl-pyrrolidin-2-ylethynyl)-phenyl]-acetamide;
(+)-cis-1-methyl-5-(1-methyl-1H-indol-5-ylethynyl)-3-piperidin-1-ylmethyl-pyrrolidin-2-one;
(+)-cis-5-benzofuranyl-5-ylethynyl-1-methyl-3-piperidin-1-ylmethyl-pyrrolidin-2-one

6. A compound of the formula I according to claim 1, selected from the group of compounds of the formula (E-I) and of the formula (E-2) represented in the following tables: 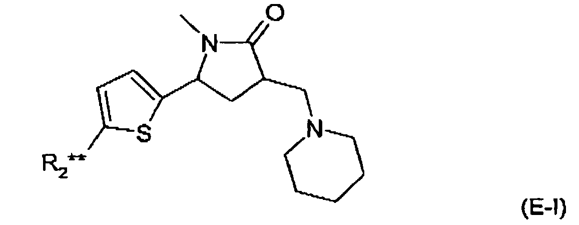
| Compound | R₂** |
|---|---|
| E12 | Phe |
| E13 | 2-Cl-Phe |
| E14 | 3-Br-Phe |
| E15 | 2-Me-Phe |
| E18 | 3-Cl-Phe |
| E20 | 3-nitro-Phe |
| E21 | 3-CN-Phe |
| E24 | 4-Me-Phe |
| E28 | 2-MeO,4-MeO-Phe |
| E29 | 2-F₃C,4-F₃C-Phe |
| E33 | 3,5-Me₂-Phe |
| E35 | 3,4-(-O-CH₂-O-)Phe |
| E36 | thiophen-2-yl |
| E37 | thiophenyl |
| E38 | quinolin-5-yl |
| E41 | 3,4-(=N-O-N=)Phe |
or 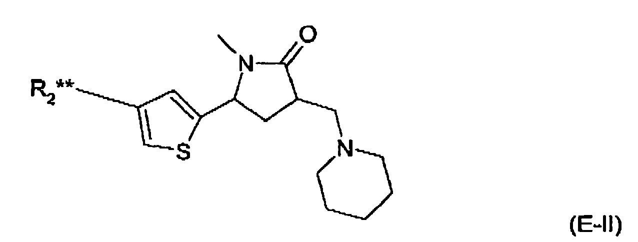
| Compound | R₂** |
|---|---|
| E42 | Br |
| E43 | Phe |
| E44 | 2-Cl-Phe |
in the form of a mixture of isomers or as a single isomer.

7. A compound of the formula I according to claim 1, selected from the group of compounds of the formula (F) represented in the following table: 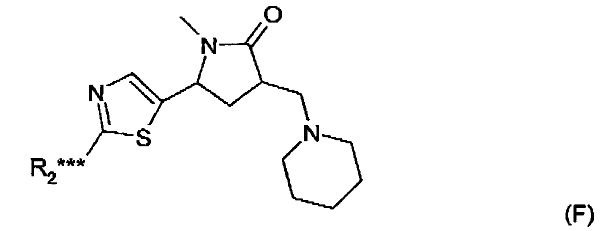
| Compound | R₂*** |
|---|---|
| F8 | 2-Cl-Phe |
| F10 | 2-F₃C-Phe |
| F11 | 4-F₃C-Phe |
| F13 | 2-Cl,4-Cl-Phe |
| F14 | 2-Cl,3-Cl-Phe |
| F15 | 2-Cl,5-Cl-Phe |
| F16 | 4-MeO-Phe |
| F17 | thiophen-2-yl |
| F18 | 3-Cl-thiophen-2-yl |
| F19 | thiophen-3-yl |
| F22 | 3,4-(-O-CH₂-O-)Phe |
| F23 | 3,4-(=N-O-N=)Phe |
| F24 | 3,4-(=N-S-N=)Phe |
in the form of a mixture of isomers or as a single isomer.

8. A pharmaceutical composition, comprising a compound of the formula I, and/or a pharmaceutically acceptable salt thereof, according to any one of claims 1 to 7, and a pharmaceutically acceptable diluent and/or carrier.

9. A compound of the formula I and/or a pharmaceutically acceptable salt thereof, according to any one of claims 1 to 7, for use in the diagnostic or therapeutic treatment of a mammal, including a human, especially for use as an alpha-7-agonist.

10. The use of a compound of the formula I, and/or a pharmaceutically acceptable salt thereof, according to any one of claims 1 to 7 for the manufacture of a medicament for the treatment or prevention of a disease or condition in the treatment of which alpha-7 receptor activation plays a role or is involved and/or in which alpha-7 receptor activity is involved.

11. A process for the manufacture of a compound of the formula I, and/or a pharmaceutically acceptable salt thereof, as defined claim 1,
wherein
for the synthesis of a compound of the formula I wherein n is 1 and R₁, R₂, R₃ and R₄ have the meanings given in claim 1 for a compound of the formula I, a methylene compound of the formula II, 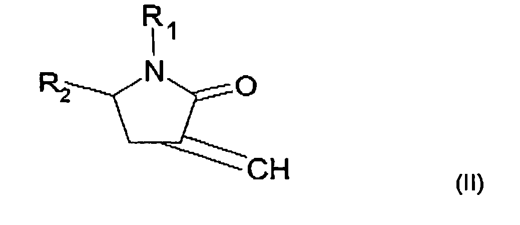 or a salt thereof where a salt-forming group is present, wherein R₁ and R₂ are as defined for compounds of the formula I in claim 1, is reacted with an imino compound of the formula III,
HN(R₃R₄) (III)
or a salt therof, wherein R₃ and R₄ have the meanings indicated in claim 1 for a compound of the formula I; to a corresponding compound of the formula I, and/or a pharmaceutically acceptable salt thereof;
or
and, if desired, transforming a compound of formula I into a different compound of formula I, transforming a salt of an obtainable compound of formula I into the free compound or a differrent salt, transforming an obtainable free compound of formula I into a salt, and/or separating obtainable mixtures of isomers of compounds of formula I into the individual isomers.

12. A salt of a compound of formula (I) according to any one of claims 1 to 7.

13. A pharmaceutically acceptable salt of a compound of formula (I) according to any one of claims 1 to 7.

14. A compound of the formula I, and/or a pharmaceutically acceptable salt thereof, according to any one of claims 1 to 7, for use as a PET ligand.

## Patentansprüche

1. Verbindungen der Formel I 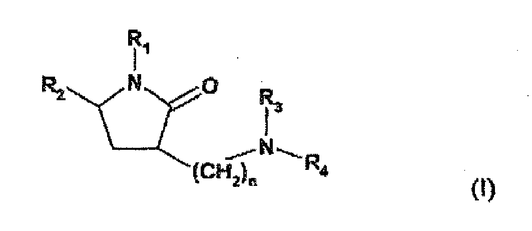 in welcher
R₁ für C₁-C₇-Alkyl, insbesondere Methyl, steht;
R₂ für Phenyl, welches unsubstituiert oder durch einen oder mehrere, insbesondere bis zu drei, Substituenten, unabhängig voneinander ausgewählt aus C₁-C₇-Alkyl, C₃-C₈-Cycloalkyl, unsubstituiertes, halogen- und/oder C₁-C₇-alkoxysubstituiertes Phenyl- oder Naphthyl-C₁-C₇-alkyl, Phenyl oder (1-oder 2-)Naphthyl, wobei dieses Phenyl bzw. Naphthyl jeweils vorzugsweise in der p-Position zu der Bindung, über die das substituierte Phenyl an den Rest des Moleküls gebunden ist, steht und unsubstituiert oder durch einen oder mehrere, insbesondere bis zu drei, Substituenten, ausgewählt aus C₁-C₇-Alkyl, Halogen-C₁-C₇-alkyl, C₁-C₇-Alkoxy, Halogen-C₁-C₇-alkoxy, Phenoxy, C₁-C₇-Alkylthio, Nitro, Cyano, Halogen und einem zweiwertigen Liganden, der an zwei benachbarte Kohlenstoffatome im Arylring gebunden ist (und so mit den Atomen, an die er gebunden ist, einen Ring bildet), wobei der zweiwertige Ligand ausgewählt ist aus der Gruppe bestehend aus -O-CH₂-O-,-O-CH₂-CH₂-O-, -CH₂-O-CH₂-, -CH₂-CH₂-CH₂-, -CH₂-CH₂-CH₂-CH₂-, -O-CF₂-O-, =N-O-N= und =N-S-N=, substituiert ist; Hydroxy; Hydroxy-C₁-C₇-alkyl, C₁-C₇-Alkoxy, Phenoxy, C₁-C₇-Alkanoyloxy, Halogen, Halogen-C₁-C₇-alkyl wie Trifluormethyl; Nitro; Amino; N-Mono- oder N,N-Di-(C₁-C₇-alkyl)amino, C₁-C₇-Alkanoylamino, C₁-C₇-Alkanoyl, Carboxy; C₁-C₇-Alkoxycarbonyl, Carbamoyl, N-Mono- oder N,N-Di-(C₁-C₇-alkyl)carbamoyl, Sulfamoyl; C₁-C₇-Alkylsulfonyl, einen zweiwertigen Liganden, der an zwei benachbarte Kohlenstoffatome im Phenyl- bzw. Naphthylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= und =N-S-N=; und unsubstituiertes oder substituiertes Heterocyclyl mit 5 bis 7 Ringatomen, welches ungesättigt, teilweise gesättigt oder gesättigt ist, eins bis drei Heteroatome ausgewählt aus O, N (oder NH) und S, als solche oder kondensiert mit Benzo, aufweist und unsubstituiert oder durch bis zu drei Einheiten, unabhängig voneinander ausgewählt aus Halogen und C₁-C₇-Alkyl, substituiert ist, zum Beispiel Pyrrolidinyl, Thiophenyl, Thiazolyl, Pyridinyl, Benzofuranyl, Indolyl, Benzothiophenyl und Benzothiazolyl, substituiert ist;
C₃-C₈-Cycloalkyl, welches substituiert ist, vorzugsweise an einem Ringkohlenstoff, bei dem es sich nicht um den handelt, der an den zentralen Pyrrolidinonring in Formel I bindet, durch Phenyl, welches unsubstituiert oder durch einen oder mehrere, insbesondere bis zu drei, Substituenten, unabhängig voneinander ausgewählt aus den gerade für substituiertes Phenyl-R₂ erwähnten, insbesondere Phenyl oder halogensubstituiertes Phenyl, wie Fluor-, Chlor- oder Bromphenyl, substituiert ist;
unsubstituiertes oder substituiertes Heterocyclyl mit 5 bis 7 Ringatomen, welches ungesättigt, teilweise gesättigt oder gesättigt ist und eins bis drei Heteroatome ausgewählt aus O, N (oder NH) und S aufweist, als solche oder kondensiert mit Benzo, wie z.B. eine unsubstituierte oder substituierte Einheit, ausgewählt aus Pyrrolidinyl, Imidazolyl, Thiophenyl, Thiazolyl, Pyridinyl, Indolyl, Chinolinyl, Benzofuranyl, Benzothiophenyl und Benzothiazolyl, wobei Heterocyclyl unsubstituiert oder durch bis zu drei Einheiten, unabhängig voneinander ausgewählt aus Halogen, substituiert ist; C₁-C₇-Alkyl, unsubstituiertes oder substituiertes Phenyl oder unsubstituiertes oder substituiertes Naphthyl, jeweils mit bis zu drei Substituenten, unabhängig voneinander ausgewählt aus der Gruppe bestehend aus C₁-C₇-Alkyl, Phenyl-C₁-C₇-alkyl, welches unsubstituiert oder am Phenylring durch bis zu drei Halogensubstituenten, substituiert ist; C₁-C₇-Alkoxy, Halogen, Halogen-C₁-C₇-alkyl, Halogen-C₁-C₇-alkoxy, Cyano; Nitro; einen zweiwertigen Liganden, der an zwei benachbarte Kohlenstoffatome im Phenylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= und =N-S-N=; unsubstituiertes oder substituiertes Phenyl-C₁-C₇-alkyl, wobei es sich bei den Substituenten um bis zu drei Substituenten, unabhängig voneinander ausgewählt aus Halogen wie Chlor and C₁-C₇-Alkoxy wie Methoxy, handelt; und unsubstituiertes oder substituiertes Heterocyclyl mit 5 bis 7 Ringatomen, welches ungesättigt, teilweise gesättigt oder gesättigt ist, ein bis drei Heteroatome ausgewählt aus O, N (oder NH) und S, als solche oder kondensiert mit Benzo, aufweist und unsubstituiert oder durch bis zu drei Einheiten, unabhängig voneinander ausgewählt aus Halogen und C₁-C₇-Alkyl, substituiert ist;
substituiertes C₁-C₇-Alkyl, vorzugsweise C₂-C₄-Alkyl, welches durch unsubstituiertes oder substituiertes Phenyl oder Naphthyl substituiert ist, wobei die Substituenten, vorzugsweise einer oder mehrere, insbesondere bis zu drei, unabhängig voneinander ausgewählt sind aus Halogen, C₁-C₇-Alkyl, Halogen-C₁-C₇-Alkyl, C₁-C₇-Alkoxy, Halogen-C₁-C₇-alkoxy, Hydroxyl, Hydroxyl-C₁-C₇-alkyl, Nitro, Cyano, Amino, N-Mono- oder N,N-Di-C₁-C₇-alkylamino, N-C₁-C₇-Alkanoylamino, C₁-C₇-Alkanoyloxy, C₁-C₇-Alkanoyl, Carboxy, C₁-C₇-Alkoxycarbonyl, Carbamoyl, N-Mono- or N,N-Di-(C₁-C₇-alkyl)carbamoyl wie N,N-Di(ethyl)carbamoyl, Sulfamoyl, Phenyl; aus einem zweiwertigen Liganden, der an zwei benachbarte Kohlenstoffatome im Arylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= und =N-S-N=; und aus unsubstituiertem oder substituiertem Heterocyclyl, ausgewählt aus Pyridinyl, Thiazolyl, Indolyl, C₁-C₇-Alkylindolyl, Benzofuranyl, Benzothiophenyl und Benzothiazolyl wie 2-Benzothiazolyl;
unsubstituiertes oder substituiertes C₁-C₇-Alkenyl, vorzugsweise C₂-C₄-Alkenyl, insbesondere Vinyl, das terminal durch unsubstituiertes oder substituiertes Phenyl mit bis zu drei Halogensubstituenten substituiert ist; und
Wasserstoff oder C₁-C₇-Alkyl in der 1-Position trägt; wobei die Doppelbindung, bezogen auf die terminalen Substituenten und den zentralen Pyrrolidinonring in Formel I, in der cis, trans- oder vorzugsweise in der trans- oder ganz besonders bevorzugt in der cis-Konfiguration vorliegt;
oder unsubstituiertes oder substituiertes C₂-C₄-Alkinyl, insbesondere Ethinyl, das (insbesondere terminal) entweder durch unsubstituiertes oder substituiertes Phenyl oder Naphthyl substituiert ist, wobei die Substituenten, vorzugsweise einer oder mehrere, insbesondere bis zu drei, unabhängig voneinander ausgewählt sind aus Halogen, C₁-C₇-Alkyl, C₁-C₇-Alkoxy, Halogen-C₁-C₇-alkyl, Hydroxyl, Hydroxyl-C₁-C₇-alkyl, Cyano, Amino, N-Mono- oder N,N-Di-C₁-C₇-alkylamino, N-C₁-C₇-Alkanoylamino, C₁-C₇-Alkanoyloxy, C₁-C₇-Alkanoyl, Carboxy, C₁-C₇-Alkoxycarbonyl, Carbamoyl, N-Mono- oder N,N-Di(C₁-C₇-alkyl)carbamoyl, Sulfamoyl, Phenyl und einem zweiwertigen Rest, der an zwei benachbarte Kohlenstoffatome im Arylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= und =N-S-N=; oder durch unsubstituiertes oder substituiertes Heterocyclyl mit 5 bis 7 Ringatomen, welches ungesättigt, teilweise gesättigt oder gesättigt ist, ein bis drei Heteroatome ausgewählt aus O, N (oder NH) und S aufweist, als solche oder kondensiert mit Benzo, und unsubstituiert oder durch bis zu drei Einheiten, unabhängig voneinander ausgewählt aus Halogen und C₁-C₇-Alkyl, substituiert ist, insbesondere Pyridin-2-yl oder Pyridin-3-yl, Thiazolyl, Indolyl, C₁-C₇-Alkylindolyl, Benzofuranyl, Benzothiophenyl wie 5-Benzo[b]thiophenyl und Benzothiazolyl steht;
R₃ und R₄ für C₁-C₇-Alkyl, vorzugsweise Ethyl, stehen, oder zusammen mit dem bindenden Stickstoff einen Ring mit (einschließlich des bindenden Stickstoffs) 3 bis 10 Ringatomen, besonders bevorzugt einen N-Piperidinyl- (sehr bevorzugt), einen N-Pyrrolidinyl- oder einen N-Azepanylring, bilden; und
n für 1 steht.

2. Verbindungen der Formel I nach Anspruch 1, wobei
R₁ für Methyl steht;
R₂ für Phenyl, welches unsubstituiert oder durch einen oder mehrere, insbesondere bis zu drei, Substituenten, unabhängig voneinander ausgewählt aus C₁-C₇-Alkyl; C₁-C₇-Alkoxy; C₃-C₈-Cycloalkyl, Phenyl oder (1- oder 2-)Naphthyl, wobei dieses Phenyl bzw. Naphthyl jeweils vorzugsweise in der p-Position zu der Bindung, über die das substituierte Phenyl an den Rest des Moleküls gebunden ist, steht, und unsubstituiert oder durch einen oder mehrere, insbesondere bis zu drei, Substituenten, ausgewählt aus Halogen, einem zweiwertigen Liganden, der an zwei benachbarte Kohlenstoffatome im Arylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= und =N-S-N=, substituiert ist; Pyrrolidinyl und Thiophenyl, vorzugsweise in der 4-Position des Phenyls, an das es als Substituent gebunden ist, substituiert ist, C₃-C₈-Cycloalkyl, insbesondere Cyclopentyl, Cyclohexyl oder vorzugsweise Cyclopropyl, welches substituiert ist, vorzugsweise an einem Ringkohlenstoff, bei dem es sich nicht um den handelt, der an den zentralen Pyrrolidinonring in Formel I bindet, insbesondere in der 2-Position, durch Phenyl oder halogensubstituiertes Phenyl;
Imidazolyl, Thiophenyl oder Thiazolyl, die jeweils unsubstituiert oder durch bis zu drei Einheiten, unabhängig voneinander ausgewählt aus unsubstituiertem oder substituiertem Phenyl oder unsubstituiertem oder substituiertem Naphthyl, Phenyl oder Naphthyl, falls substituiert dann jeweils durch bis zu drei Substituenten, unabhängig voneinander ausgewählt aus der Gruppe bestehend aus C₁-C₇-Alkyl, C₁-C₇-Alkoxy, Halogen, Halogen-C₁-C₇-alkyl, Cyano, Nitro, einem zweiwertigen Liganden, der an zwei benachbarte Kohlenstoffatome im Phenylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= und =N-S-N=, Thiophenyl, Halogenthiophenyl und Chinolinyl substituiert sind;
unsubstituiertes oder substituiertes Vinyl, das terminal durch unsubstituiertes oder substituiertes Phenyl mit bis zu drei Halogensubstituenten, substituiert ist und Wasserstoff oder C₁-C₇-Alkyl in der 1-Position trägt; wobei die Doppelbindung, bezogen auf die terminalen Substituenten und den zentralen Pyrrolidinonring in Formel I, in der cis,trans- oder vorzugsweise in der trans- oder ganz besonders bevorzugt in der cis-Konfiguration vorliegt;
oder unsubstituiertes oder substituiertes Ethinyl, welches entweder durch unsubstituiertes oder substituiertes Phenyl substituiert ist, wobei die Substituenten, vorzugsweise einer oder mehrere, insbesondere bis zu drei, unabhängig voneinander ausgewählt sind aus Halogen, C₁-C₇-Alkyl, C₁-C₇-Alkoxy, Halogen-C₁-C₇-alkyl, Hydroxyl, Hydroxyl-C₁-C₇-alkyl, Cyano, Amino, N-Mono- oder N,N-Di-C₁-C₇-alkylamino, N-C₁-C₇-Alkanoylamino, C₁-C₇-Alkanoyloxy, C₁-C₇-Alkanoyl, C₁-C₇-Alkoxycarbonyl, Carbamoyl, N-Mono- oder N,N-Di-(C₁-C₇-alkyl)carbamoyl, Sulfamoyl und einem zweiwertigen Liganden, der an zwei benachbarte Kohlenstoffatome im Arylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= und N-S-N=; oder durch unsubstituiertes oder substituiertes Heterocyclyl mit 5 bis 7 Ringatomen, welches ungesättigt, teilweise gesättigt oder gesättigt ist, ein bis drei Heteroatome ausgewählt aus O, N (oder NH) und S aufweist, als solche oder kondensiert mit Benzo, und unsubstituiert oder durch bis zu drei Einheiten, unabhängig voneinander ausgewählt aus Halogen und C₁-C₇-Alkyl, substituiert ist, insbesondere Pyridin-2-yl oder Pyridin-3-yl, Thiazolyl, Indolyl, C₁-C₇-Alkyl-indolyl, Benzofuranyl, Benzothiophenyl oder Benzothiazolyl steht;
R₃ und R₄ zusammen mit dem bindenden Stickstoff einen N-Piperidinyl-, einen N-Pyrrolidinyl- oder einen N-Azepanylring bilden; und
n für 1 steht.

3. Verbindungen der Formel I nach Anspruch 1, wobei
R₁ für C₁-C₇-Alkyl, insbesondere Methyl, steht;
R₂ für C₁-C₇-Alkyl, vorzugsweise C₂-C₄-Alkyl, steht, welches durch unsubstituiertes oder substituiertes Phenyl oder Naphthyl substituiert ist, wobei die Substituenten, vorzugsweise einer oder mehrere, insbesondere bis zu drei, unabhängig voneinander aus den oben unter substituiert erwähnten ausgewählt sind, und vorzugsweise ausgewählt sind aus Halogen, C₁-C₇-Alkyl, Halogen-C₁-C₇-alkyl, C₁-C₇-Alkoxy, Halogen-C₁-C₇-alkoxy, Hydroxyl, Hydroxyl-C₁-C₇-alkyl, Nitro, Cyano, Amino, N-Mono- oder N, N-Di -C₁-C₇-alkylamino, N-C₁-C₇-Alkanoylamino, C₁-C₇-Alkanoyloxy, C₁-C₇-Alkanoyl, Carboxy, C₁-C₇-Alkoxycarbonyl, Carbamoyl, N-Mono- oder N,N-Di-(C₁-C₇-alkyl) carbamoyl, Sulfamoyl, Phenyl; aus einem zweiwertigen Liganden, der an zwei benachbarte Kohlenstoffatome im Arylring gebunden ist (und so mit den Atomen, an die er gebunden ist, einen Ring bildet), wobei der zweiwertige Ligand ausgewählt ist aus der Gruppe bestehend aus -O-CH₂-O-, -O-CH₂-CH₂-O-, -CH₂-O-CH₂-, -CH₂-CH₂-CH₂-, -CH₂-CH₂-CH₂-CH₂-, -0-CF₂-O-, =N-O-N= und =N-S-N=; und aus unsubstituiertem oder substituiertem Heterocyclyl, ausgewählt aus Pyridinyl, Thiazolyl, Indolyl, C₁-C₇-Alkylindolyl, Benzofuranyl, Benzothiophenyl und Benzothiazolyl;
R₃ und R₄ zusammen mit dem bindenden Stickstoff einen Ring mit (einschließlich des bindenden Stickstoffs) 4 bis 8 Ringatomen, besonders bevorzugt einen N-Piperidinyl- (sehr bevorzugt), einen N-Pyrrolidinyl- oder einen N-Azepanylring bilden; und
n für 1 steht.

4. Verbindungen der Formel I nach Anspruch 1, ausgewählt aus der Gruppe von Verbindungen der Formel (D), die in der folgenden Tabelle wiedergegeben sind: 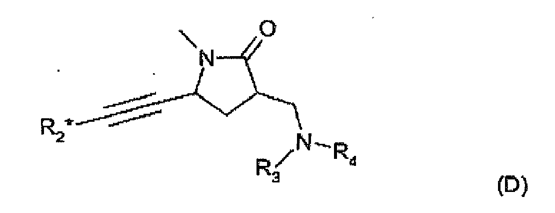
| Verbindung | R₂* | R₃ | R₄ |
|---|---|---|---|
| D10 | Phe | zusammen | -(CH₂)₅- |
| D11 | 2-Cl-Phe | zusammen | -(CH₂)₅- |
| D12 | 2-Br-Phe | zusammen | -(CH₂)₅- |
| D13 | 2-F-Phe | zusammen | -(CH₂)₅- |
| D14 | 2-Me-Phe | zusammen | -(CH₂)₅- |
| D15 | 2-MeO-Phe | zusammen | -(CH₂)₅- |
| D16 | 2-F₃C-Phe | zusammen | -(CH₂)₅- |
| D17 | 2-CN-Phe | zusammen | -(CH₂)₅- |
| D18 | 2-Hydroxy-Phe | zusammen | -(CH₂)₅- |
| D19 | 3-Cl-Phe | zusammen | -(CH₂)₅- |
| D20 | 3-MeO-Phe | zusammen | -(CH₂)₅- |
| D21 | 3-F₃C-Phe | zusammen | -(CH₂)₅- |
| D22 | 3- (Mₑ2N) -Phe | zusammen | -(CH₂)₅- |
| D23 | 3-Hydroxy-Phe | zusammen | -(CH₂)₅- |
| D24 | 3-Acetoxy-Phe | zusammen | -(CH₂)₅- |
| D25 | 3-Amino-Phe | zusammen | -(CH₂)₅- |
| D26 | 3-Acetylamino-Phe | zusammen | -(CH₂)₅- |
| D27 | 3-Hydroxymethyl-Phe | zusammen | -(CH₂)₅- |
| D28 | 3-Acetyl-Phe | zusammen | -(CH₂)₅- |
| D30 | 3-Ethoxycarbonyl-Phe | zusammen | -(CH₂)₅- |
| D31 | 3-N,N-Diethyl-carbamoyl-Phe | zusammen | -(CH₂)₅- |
| D32 | 3-Sulfamoyl-Phe | zusammen | -(CH₂)₅- |
| D33 | 4-Cl-Phe | zusammen | -(CH₂)₅- |
| D34 | 4-F-Phe | zusammen | -(CH₂)₅- |
| D35 | 4-Me-Phe | zusammen | -(CH₂)₅- |
| D37 | 4-tert.-Butyl-Phe | zusammen | -(CH₂)₅- |
| D38 | 3, 4- (-O-CH₂-O-) Phe | zusammen | -(CH₂)₅- |
| D39 | 3, 4- (-O-CH₂-CH₂-O-)Phe | zusammen | -(CH₂)₅- |
| D40 | 2,3-((-CH₂)₄-)Phe | zusammen | -(CH₂)₅- |
| D41 | 3,4-((-CH₂)₄-) Phe | zusammen | -(CH₂)₅- |
| D43 | 4-Phe-Phe | zusammen | -(CH₂)₅- |
| D44 | 2-Cl, 3-Cl-Phe | zusammen | -(CH₂)₅- |
| D45 | 2-F,5-F-Phe | zusammen | -(CH₂)₅- |
| D46 | 2-Cl-, 5-Methyl-Phe | zusammen | -(CH₂)₅- |
| D47 | 3-Me, 5-Me-Phe | zusammen | -(CH₂)₅- |
| D50 | 4-Hydroxy-3-Me-Phe | zusammen | -(CH₂)₅- |
| D51 | 2, 3- (=N-S-N=) Phe | zusammen | -(CH₂)₅- |
| D52 | 2,3-(-CH=CH-CH=CH-) Phe | zusammen | -(CH₂)₅- |
| D53 | 5-Indolyl | zusammen | -(CH₂)₅- |
| D54 | 5-Benzofuranyl | zusammen | -(CH₂)₅- |
| D55 | 5-Benzo[b]thiophenyl | zusammen | -(CH₂)₅- |
| D56 | 2-Pyridinyl | zusammen | -(CH₂)₅- |
| D57 | 3-Pyridinyl | zusammen | -(CH₂)₅- |
| D58 | 2-Thiazolyl | zusammen | -(CH₂)₅- |
| D59 | 2-Benzothiazolyl | zusammen | -(CH₂)₅- |
in Form einer Mischung von Isomeren oder als einzelnes Isomer.

5. Verbindungen der Formel I nach Anspruch 1, ausgewählt aus der Gruppe von Verbindungen bestehend aus
cis-1-Methyl-3-piperidin-1-ylmethyl-5-m-tolylethinylpyrrolidin-2-on;
cis-1-Methyl-5-phenylethinyl-3-piperidin-1-ylmethylpyrrolidin-2-on;
cis-5-(2-Chlorphenylethinyl)-1-methyl-3-piperidin-1-ylmethylpyrrolidin-2-on;
cis-N-[2-(1-Methyl-5-oxo-4-piperidin-1-ylmethylpyrrolidin-2-ylethinyl)phenyl]acetamid;
cis-1-Methyl-5-(1-methyl-1H-indol-5-ylethinyl)-3-piperidin-1-ylmethylpyrrolidin-2-on;
cis-5-Benzofuranyl-5-ylethinyl-1-methyl-3-piperidin-1-ylmethylpyrrolidin-2-on;
(+)cis-1-Methyl-3-piperidin-1-ylmethyl-5-m-tolylethinylpyrrolidin-2-on;
(+)cis-1-Methyl-5-phenylethinyl-3-piperidin-1-ylmethylpyrrolidin-2-on;
(+)cis-5-(2-Chlorphenylethinyl)-1-methyl-3-piperidin-1-ylmethylpyrrolidin-2-on;
(+)cis-N-[2-(1-Methyl-5-oxo-4-piperidin-1-ylmethylpyrrolidin-2-ylethinyl)phenyl]acetamid;
(+)cis-1-Methyl-5-(1-methyl-1H-indol-5-ylethinyl)-3-piperidin-1-ylmethylpyrrolidin-2-on;
(+)cis-5-Benzofuranyl-5-ylethinyl-1-methyl-3-piperidin-1-ylmethylpyrrolidin-2-on.

6. Verbindungen der Formel I nach Anspruch 1, ausgewählt aus der Gruppe von Verbindungen der Formel (E-1) und der Formel (E-2), die in den folgenden Tabellen wiedergegeben sind: 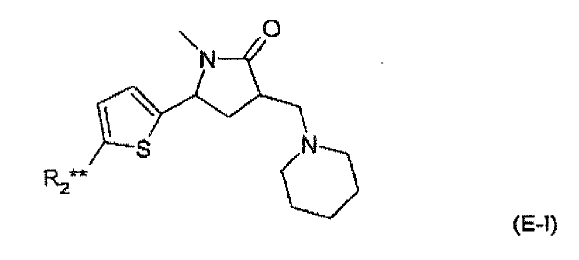
| Verbindung | R₂** |
|---|---|
| E12 | Phe |
| E13 | 2-Cl-Phe |
| E14 | 3-Br-Phe |
| E15 | 2-Me-Phe |
| E18 | 3-Cl-Phe |
| E20 | 3-Nitro-Phe |
| E21 | 3-CN-Phe |
| E24 | 4-Me-Phe |
| E28 | 2-MeO,4-MeO-Phe |
| E29 | 2-E₃C,4-F₃C-Phe |
| E33 | 3,5-Me₂-Phe |
| E35 | 3,4-(-O-CH₂-O-)Phe |
| E36 | Thiophen-2-yl |
| E37 | Thiophen-3-yl |
| E38 | Chinolin-5-yl |
| E41 | 3,4-(=N-O-N=)Phe |
oder 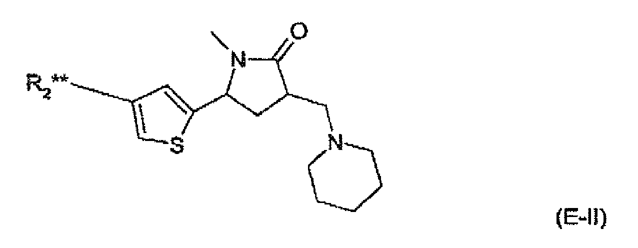
| Verbindung | R₂** |
|---|---|
| E42 | Br |
| E43 | Phe |
| E44 | 2-Cl-Phe |
in Form einer Mischung von Isomeren oder als einzelnes Isomer.

7. Verbindungen der Formel I nach Anspruch 1, ausgewählt aus der Gruppe von Verbindungen der Formel (F), die in der folgenden Tabelle wiedergegeben sind: 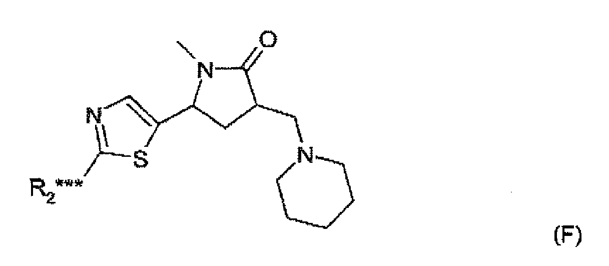
| Verbindung | R₂*** |
|---|---|
| F8 | 2-Cl-Phe |
| F10 | 2-F₃C-Phe |
| F11 | 4-F₃C-Phe |
| F13 | 2-Cl, 4-Cl-Phe |
| F14 | 2-Cl, 3-Cl-Phe |
| F15 | 2-Cl, 5-Cl-Phe |
| F16 | 4-MeO-Phe |
| F17 | Thiophen-2-yl |
| F18 | 3-Cl-Thiophen-2-yl |
| F19 | Thiophen-3-yl |
| F22 | 3,4-(-O-CH₂-O)Phe |
| F23 | 3,4-(=N-O-N=)Phe |
| F24 | 3,4-(=N-S-N=)Phe |
in Form einer Mischung von Isomeren oder als einzelnes Isomer.

8. Pharmazeutische Zusammensetzung, enthaltend eine Verbindung der Formel I und/oder ein pharmazeutisch unbedenkliches Salz davon nach einem der Ansprüche 1 bis 7 und ein pharmazeutisch unbedenkliches Verdünnungsmittel und/oder einen pharmazeutisch unbedenklichen Träger.

9. Verbindungen der Formel I und/oder deren pharmazeutisch unbedenkliche Salze nach einem der Ansprüche 1 bis 7 zur Verwendung bei der diagnostischen oder therapeutischen Behandlung eines Säugetieres einschließlich eines Menschen, insbesondere zur Verwendung als alpha-7-Agonist.

10. Verwendung einer Verbindung der Formel I und/oder eines pharmazeutisch unbedenklichen Salzes davon nach einem der Ansprüche 1 bis 7 zur Herstellung eines Medikaments zur Behandlung oder Prävention einer Krankheit oder eines Leidens, bei deren/dessen Behandlung die Aktivierung des alpha-7-Rezeptors eine Rolle spielt bzw. daran beteiligt ist und/oder an der alpha-7-Rezeptoraktivität beteiligt ist.

11. Verfahren zur Herstellung einer Verbindung der Formel I und/oder eines pharmazeutisch unbedenklichen Salzes davon nach Anspruch 1, bei dem man
zur Synthese einer Verbindung der Formel I, in welcher n für 1 steht und R₁, R₂, R₃ und R₄ die in Anspruch 1 für eine Verbindung der Formel I angeführten Bedeutungen haben, eine Methylenverbindung der Formel II 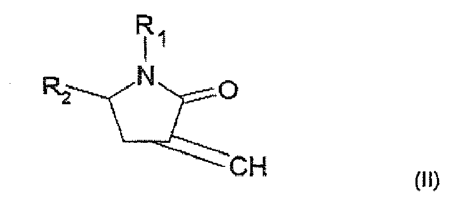 oder ein Salz davon, wenn eine salzbildende Gruppe vorhanden ist, in welcher R₁ und R₂ wie in Anspruch 1 für Verbindungen der Formel I definiert sind, mit einer Iminoverbindung der Formel III
HN(R₃R₄) (III)
oder einem Salz davon, wobei R₃ and R₄ die in Anspruch 1 für eine Verbindung der Formel I angegebenen Bedeutungen haben; zu einer entsprechenden Verbindung der Formel I und/oder einem pharmazeutisch unbedenklichen Salz davon umsetzt;
und, falls gewünscht, eine Verbindung der Formel I in einer andere Verbindung der Formel I umwandelt, ein Salz einer erhältlichen Verbindung der Formel I in die freie Verbindung oder ein anderes Salz umwandelt, eine erhältliche freie Verbindung der Formel I in ein Salz umwandelt und/oder erhältliche Mischungen von Isomeren von Verbindungen der Formel I in die einzelnen Isomere trennt.

12. Salze einer Verbindung der Formel (I) nach einem der Ansprüche 1 bis 7.

13. Pharmazeutisch unbedenkliche Salze einer Verbindung der Formel (I) nach einem der Ansprüche 1 bis 7.

14. Verbindungen der Formel I und/oder deren pharmazeutisch unbedenkliche Salze nach einem der Ansprüche 1 bis 7 zur Verwendung als PET-Liganden.

## Revendications

1. Composé de formule I, 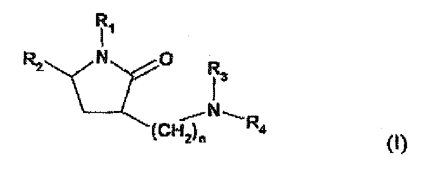 où
R₁ représente un groupement alkyle en C₁-C₇, spécialement méthyle ;
R₂ représente un groupement phényle éventuellement substitué par un ou plusieurs, spécialement jusqu'à trois, substituants indépendamment choisi parmi les groupements alkyle en C₁-C₇, cycloalkyle en C₃-C₈, phényl- ou naphtyl-(alkyle en C₁-C₇) non substitués, halogénés et/ou substitués par alkoxy en C₁-C₇, phényle ou (1- ou 2-) naphtyle, chacun desdits groupements phényle ou naphtyle étant préférentiellement en position para vis-à-vis de la liaison par laquelle le groupement phényle substitué est lié au reste de la molécule, et étant éventuellement substitué par un ou plusieurs, spécialement jusqu'à trois, substituants choisis parmi les groupements alkyle en C₁-C₇, halogénoalkyle en C₁-C₇, alkoxy en C₁-C₇, halogénoalkoxy en C₁-C₇, phénoxy, alkylthio en C₁-C₇, nitro, cyano, halogéno et les ligands bivalents liés à deux atomes de carbone adjacents dans le cycle arylique, formant ainsi un cycle avec les atomes auxquels ils sont liés, les ligands bivalents étant préférentiellement choisis dans le groupe constitué par -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= et =N-S-N= ; hydroxy ; hydroxyalkyle en C₁-C₇, alkoxy en C₁-C₇, phénoxy, alcanoyloxy en C₁-C₇, halogéno, halogénoalkyle en C₁-C₇, tel que trifluorométhyle ; nitro ; amino ; N-mono- ou N,N-di-(alkyle en C₁-C₇)amino, (alcanoyle en C₁-C₇)-amino, alcanoyle en C₁-C₇, carboxy ; (alkoxy en C₁-C₇)carbonyle, carbamoyle, N-mono- ou N,N-di-(alkyle en C₁-C₇)carbamoyle, sulfamoyle ; alkylsulfonyle en C₁-C₇, un ligand bivalent lié à deux atomes de carbone adjacents du cycle phénylique ou naphtylique, formant ainsi un cycle avec les atomes auxquels il est lié, le ligand bivalent étant choisi dans le groupe constitué par -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= et =N-S-N= ; et un groupement hétérocyclyle éventuellement substitué comportant entre 5 et 7 chaînons, insaturé, partiellement saturé ou saturé, comportant entre un et trois hétéroatomes choisis parmi O, N (ou NH) et S, tel quel ou annelé à un groupement benzo, et éventuellement substitué par jusqu'à trois groupements indépendamment choisis parmi les groupements halogéno et alkyle en C₁-C₇, par exemple pyrrolidinyle, thiophényle, thiazolyle, pyridinyle, benzofurannyle, indolyle, benzothiophényle et benzothiazolyle ; cycloalkyle en C₃-C₈ substitué, préférentiellement au niveau d'un carbone de cycle différent de celui qui est lié au cycle pyrrolidinone central dans la formule I, par un groupement phényle éventuellement substitué par un ou plusieurs, spécialement jusqu'à trois, substituants indépendamment choisis parmi ceux mentionnés ci-avant pour R₂ lorsqu'il représente un groupement phényle substitué, spécialement phényle ou phényle halogéné, tel que fluoro, chloro ou bromophényle ;
un groupement hétérocyclyle éventuellement substitué comportant entre 5 et 7 chaînons, insaturé, partiellement saturé ou saturé, comportant entre un et trois hétéroatomes choisis parmi O, N (ou NH) et S, tel quel ou annelé à un groupement benzo, par exemple un groupement éventuellement substitué choisi parmi les groupements pyrrolidinyle, imidazolyle, thiophényle, thiazolyle, pyridinyle, indolyle, quinolinyl, benzofurannyle, benzothiophényle et benzothiazolyle, le groupement hétérocyclyle étant éventuellement substitué par jusqu'à trois groupements indépendamment choisis parmi les groupements halogéno et alkyle en C₁-C₇; phényle éventuellement substitué ou naphtyle éventuellement substitué, dans chaque cas par jusqu'à trois substituants indépendamment choisis dans le groupe constitué par les groupements alkyle en C₁-C₇, phényl-(alkyle en C₁-C₇) éventuellement substitué au niveau du cycle phénylique par jusqu'à trois substituants halogéno, alkoxy en C₁-C₇, halogéno, halogénoalkyle en C₁-C₇, halogénoalkoxy en C₁-C₇, cyano ; nitro ; un ligand bivalent lié à deux atomes de carbone adjacents dans le cycle phénylique, formant ainsi un cycle avec les atomes auxquels il est lié, le ligand bivalent étant choisi dans le groupe constitué par -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= et =N-S-N= ; phénylalkyle en C₁-C₇ éventuellement substitué où les substituants sont jusqu'à trois substituants indépendamment choisis parmi les groupements halogéno, tels que chloro, et alkoxy en C₁-C₇, tels que méthoxy ; et un groupement hétérocyclyle éventuellement substitué comportant entre 5 et 7 chaînons, insaturé, partiellement saturé ou saturé, comportant entre un et trois hétéroatomes choisis parmi O, N (ou NH) et S, tel quel ou annelé à un groupement benzo, et éventuellement substitué par jusqu'à trois groupements indépendamment choisis parmi les groupements halogéno et alkyle en C₁-C₇;
alkyle en C₁-C₇ substitué, préférentiellement alkyle en C₂-C₄, substitué par un groupement phényle ou naphtyle éventuellement substitué, où les substituants, préférentiellement un ou plusieurs, spécialement jusqu'à trois, sont choisis indépendamment parmi les groupements halogéno, alkyle en C₁-C₇, halogénoalkyle en C₁-C₇, alkoxy en C₁-C₇, halogénoalkoxy en C₁-C₇, hydroxyle, hydroxylalkyle en C₁-C₇, nitro, cyano, amino, N-mono- ou N,N-di-(alkyle en C₁-C₇)amino, N-(alcanoyle en C₁-C₇)-amino, alcanoyloxy en C₁-C₇, alcanoyle en C₁-C₇, carboxy, (alkoxy en C₁-C₇)carbonyle, carbamoyle, N-mono- ou N,N-(alkyle en C₁-C₇)-carbamoyle, tels que N,N-di(éthyl)-carbamoyle, sulfamoyle, phényle ; parmi les ligands bivalents liés à deux atomes de carbone adjacents du cycle arylique, formant ainsi un cycle avec les atomes auxquels ils sont liés, les ligands bivalents étant choisis dans le groupe constitué par - O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= et =N-S-N= et parmi les groupements hétérocyclyle éventuellement substitués choisis parmi les groupements pyridinyle, thiazolyle, indolyle, (alkyle en C₁-C₇)-indolyle, benzofurannyle, benzothiophényle et benzothiazolyle, tels que 2-benzothiazolyle ;
alcényle en C₁-C₇ éventuellement substitué, préférentiellement alcényle en C₂-C₄, spécialement vinyle, substitué en position terminale par un groupement phényle éventuellement substitué par jusqu'à trois substituant halogénés, et portant un groupement alkyle en C₁-C₇ en position 1 ; où la double liaison, par rapport aux substituants terminaux et au cycle pyrrolidinone central de la formule I, est en configuration *cis, trans* ou préférentiellement *trans* ou le plus préférentiellement *cis ;*
ou un groupement alcynyle en C₂-C₄ éventuellement substitué, spécialement éthynyle substitué (spécialement en position terminale) par un groupement phényle ou naphtyle éventuellement substitué, les substituants, préférentiellement un ou plusieurs, spécialement jusqu'à trois, étant choisis indépendamment parmi les groupements halogéno, alkyle en C₁-C₇, alkoxy en C₁-C₇, halogénoalkyle en C₁-C₇, hydroxyle, hydroxylalkyle en C₁-C₇, cyano, amino, N-mono- ou N,N-di-(alkyle en C₁-C₇)amino, N-(alcanoyle en C₁-C₇)-amino, alcanoyloxy en C₁-C₇, alcanoyle en C₁-C₇, carboxy, (alkoxy en C₁-C₇)-carbonyle, carbamoyle, N-mono- ou N,N-di-(alkyle en C₁-C₇)-carbamoyle, sulfamoyle, phényle, et les ligands bivalents liés à deux atomes de carbone adjacents du cycle arylique, formant ainsi un cycle avec les atomes auxquels ils sont liés, les ligands bivalents étant préférentiellement choisis dans le groupe constitué par -O-CH₂-O-, -O-CH₂-CH₂-O-, -CH₂-O-CH₂-, -CH₂-CH₂-CH₂-, - CH₂-CH₂-CH₂-CH₂-, -O-CF₂-O-, =N-O-N= et =N-S-N= ; ou par un groupement hétérocyclyle éventuellement substitué comportant entre 5 et 7 chaînons, insaturé, partiellement saturé ou saturé, comportant entre un et trois hétéroatomes choisis parmi O, N (ou NH) et S, tel quel ou annelé à un groupement benzo, et éventuellement substitué par jusqu'à trois groupements indépendamment choisis parmi les groupements halogéno et alkyle en C₁-C₇, spécialement pyridin-2-yle ou pyridin-3-yle,
thiazolyle, indolyle, (alkyle en C₁-C₇)-indolyle, benzofurannyle, benzothiophényle, et benzothiazolyle ;
R₃ et R₄ représentent des groupements alkyle en C₁-C₇, préférentiellement éthyle, ou forment ensemble et avec l'atome azote auxquels ils sont liés un cycle comportant entre 3 et 10 chaînons, y compris l'azote de liaison, plus préférentiellement un cycle N-pipéridinyle (très préférentiellement), N-pyrrolidinyle ou N-azépanyle ; et
n est égal à 1.

2. Composé de formule I conforme à la revendication 1, où
R₁ représente un groupement méthyle ;
R₂ représente un groupement phényle éventuellement substitué par un ou plusieurs, spécialement jusqu'à trois, substituants indépendamment choisis parmi les groupements alkyle en C₁-C₇ ; alkoxy en C₁-C₇ ; cycloalkyle en C₃-C₈, phényle ou 1- ou 2-naphtyle, chacun desdits groupements phényle ou naphtyle étant préférentiellement en position para vis-à-vis de la liaison par laquelle le groupement phényle substitué est lié au reste de la molécule, et étant éventuellement substitué par un ou plusieurs, spécialement jusqu'à trois, substituants choisis parmi les groupements halogéno, les ligands bivalents liés à deux atomes de carbone adjacents du cycle arylique, formant ainsi un cycle avec les atomes auxquels ils sont liés, les ligands bivalents étant préférentiellement choisis dans le groupe constitué par -O-CH₂-O-, -O-CH₂-CH₂-O-, -CH₂-O-CH₂, -CH₂-CH₂-CH₂-, -CH₂-CH₂-CH₂-CH₂-, -O-CF₂-O-, =N-O-N= et =N-S-N= ; pyrrolidinyle et thiophényle, préférentiellement en position 4 du phényle auxquels ils sont liés au titre de substituants,
cycloalkyle en C₃-C₈, spécialement cyclopentyle, cyclohexyle ou préférentiellement cyclopropyle substitué, préférentiellement au niveau d'un carbone de cycle différent de celui qui est lié au cycle pyrrolidinone central dans la formule I, spécialement en position 2, par un groupement phényle ou phényle halogéné ;
imidazolyle, thiophényle ou thiazolyle, chacun étant éventuellement substitué par jusqu'à trois groupements indépendamment choisis parmi les groupements phényle éventuellement substitués et naphtyle éventuellement substitués par, dans chaque cas, jusqu'à trois substituants indépendamment choisis dans le groupe constitué par les groupements alkyle en C₁-C₇, alkoxy en C₁-C₇, halogéno, halogénoalkyle en C₁-C₇, cyano, nitro, les ligands bivalents liés à deux atomes de carbone adjacents du cycle phénylique, formant ainsi un cycle avec les atomes auxquels ils sont liés, les ligands bivalents étant préférentiellement choisis dans le groupe constitué par -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, -N-O-Net =N-S-N=, thiophényle, halogénothiophényle et quinolinyle ;
vinyle éventuellement substitué, substitué en position terminale par un groupement phényle éventuellement substitué par jusqu'à trois substituant halogénés, et portant un atome d'hydrogène ou un groupement alkyle en C₁-C₇ en position 1 ; où la double liaison, par rapport aux substituants terminaux et au cycle pyrrolidinone central de la formule I, est en configuration *cis, trans*
ou préférentiellement *trans* ou le plus préférentiellement *cis* ; ou
un groupement éthynyle éventuellement substitué, substitué par un groupement phényle éventuellement substitué, les substituants, préférentiellement un ou plusieurs, spécialement jusqu'à trois, étant choisis indépendamment parmi les groupements halogéno, alkyle en C₁-C₇, alkoxy en C₁-C₇, halogénoalkyle en C₁-C₇, hydroxyle, hydroxylalkyle en C₁-C₇, cyano, amino, N-mono- ou N,N-di-(alkyle en C₁-C₇)amino, N-(alcanoyle en C₁-C₇)-amino, alcanoyloxy en C₁-C₇, alcanoyle en C₁-C₇, (alkoxy en C₁-C₇)-carbonyle, carbamoyle, N-mono- ou N,N-di-(alkyle en C₁-C₇)-carbamoyle, sulfamoyle et les ligands bivalents liés à deux atomes de carbone adjacents du cycle arylique, formant ainsi un cycle avec les atomes auxquels ils sont liés, les ligands bivalents étant préférentiellement choisis dans le groupe constitué par -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= et =N-S-N= ; ou par un groupement hétérocyclyle éventuellement substitué comportant entre 5 et 7 chaînons, insaturé, partiellement saturé ou saturé, comportant entre un et trois hétéroatomes choisis parmi O, N (ou NH) et S, tel quel ou annelé à un groupement benzo, et éventuellement substitué par jusqu'à trois groupements indépendamment choisis parmi les groupements halogéno et alkyle en C₁-C₇, spécialement pyridin-2-yle ou pyridin-3-yle, thiazolyle, indolyle, (alkyle en C₁-C₇)-indolyle, benzofurannyle, benzothiophényle ou benzothiazolyle ;
R₃ et R₄ forment ensemble et avec l'atome d'azote auxquels ils sont liés un cycle N-pipéridinyle, N-pyrrolidinyle ou N-azépanyle ; et
n est égal à 1.

3. Composé de formule I conforme à la revendication 1, où
R₁ représente un groupement alkyle en C₁-C₇, spécialement méthyle ;
R₂ représente un groupement alkyle en C₁-C₇, préférentiellement alkyle en C₂-C₄, substitué par un groupement phényle ou naphtyle éventuellement substitué, où les substituants, préférentiellement un ou plusieurs, spécialement jusqu'à trois, sont choisis indépendamment parmi les groupements halogéno, alkyle en C₁-C₇, halogénoalkyle en C₁-C₇, alkoxy en C₁-C₇, halogénoalkoxy en C₁-C₇, hydroxyle, hydroxylalkyle en C₁-C₇, nitro, cyano, amino, N-mono- ou N,N-di-(alkyle en C₁-C₇)amino, N-(alcanoyle en C₁-C₇)-amino, alcanoyloxy en C₁ -C₇, alcanoyle en C₁-C₇, carboxy, (alkoxy en C₁-C₇)carbonyle, carbamoyle, N-mono- ou N,N-(alkyle en C₁-C₇)-carbamoyle, sulfamoyle, phényle ; parmi les ligands bivalents liés à deux atomes de carbone adjacents du cycle arylique, formant ainsi un cycle avec les atomes auxquels ils sont liés, les ligands bivalents étant choisis dans le groupe constitué par -O-CH₂-O-, -O-CH₂-CH₂-O-, -CH₂-O-CH₂-, -CH₂-CH₂-CH₂-, -CH₂-CH₂-CH₂-CH₂-, -O-CF₂-O-, =N-O-N= et =N-S-N= ; et parmi les groupements hétérocyclyle éventuellement substitués choisis parmi les groupements pyridinyle, thiazolyle, indolyle, (alkyle en C₁-C₇)-indolyle, benzofurannyle, benzothiophényle et benzothiazolyle, tels que 2-benzothiazolyle ;
R₃ et R₄ forment ensemble et avec l'atome d'azote auxquels ils sont liés un cycle comportant entre 4 et 8 chaînons, y compris l'azote de liaison, plus préférentiellement un cycle N-pipéridinyle (très préférentiellement), N-pyrrolidinyle ou N-azépanyle ; et
n est égal à 1.

4. Composé de formule I conforme à la revendication 1, choisi dans le groupe de composés de formule (D) représentés dans le tableau suivant : 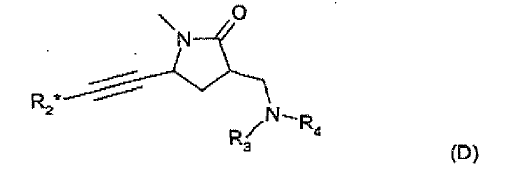
| Composé | R₂* | R₃ | R₄ |
|---|---|---|---|
| D10 | Phe | ensemble | -(CH₂)₅- |
| D11 | 2-Cl-Phe | ensemble | -(CH₂)₅- |
| D12 | 2-Br-Phe | ensemble | -(CH₂)₅- |
| D13 | 2-F-Phe | ensemble | -(CH₂)₅- |
| D14 | 2-Me-Phe | ensemble | -(CH₂)₅- |
| D15 | 2-MeO-Phe | ensemble | -(CH₂)₅- |
| D16 | 2-F₃C-Phe | ensemble | -(CH₂)₅- |
| D17 | 2-CN-Phe | ensemble | -(CH₂)₅- |
| D18 | 2-hydroxy-Phe | ensemble | -(CH₂)₅- |
| D19 | 3-Cl-Phe | ensemble | -(CH₂)₅- |
| D20 | 3-MeO-Phe | ensemble | -(CH₂)₅- |
| D21 | 3-F₃C-Phe | ensemble | -(CH₂)₅- |
| D22 | 3-(Mₑ2N)-Phe | ensemble | -(CH₂)₅- |
| D23 | 3-hydroxy-Phe | ensemble | -(CH₂)₅- |
| D24 | 3-acétoxy-Phe | ensemble | -(CH₂)₅- |
| D25 | 3-amino-Phe | ensemble | -(CH₂)₅- |
| D26 | 3-acétylamino-Phe | ensemble | -(CH₂)₅- |
| D27 | 3-hydroxyméthyl-Phe | ensemble | -(CH₂)₅- |
| D28 | 3-acétyl-Phe | ensemble | -(CH₂)₅- |
| D30 | 3-éthoxycarbonyl-Phe | ensemble | -(CH₂)₅- |
| D31 | 3-N,N-diéthylcarbamoyl-Phe | ensemble | -(CH₂)₅- |
| D32 | 3-sulfamoyl-Phe | ensemble | -(CH₂)₅- |
| D33 | 4-Cl-Phe | ensemble | -(CH₂)₅- |
| D34 | 4-F-Phe | ensemble | -(CH₂)₅- |
| D35 | 4-Me-Phe | ensemble | -(CH₂)₅- |
| D37 | 4-tert-butyl-Phe | ensemble | -(CH₂)₅- |
| D38 | 3,4-(-O-CH2-O-)Phe | ensemble | -(CH₂)₅- |
| D39 | 3,4-(-O-CH₂-CH₂-O-)Phe | ensemble | -(CH₂)₅- |
| D40 | 2,3-((-CH₂)₄-)Phe | ensemble | -(CH₂)₅- |
| D41 | 3,4-((-CH₂)₄-)Phe | ensemble | -(CH₂)₅- |
| D43 | 4-Phe-Phe | ensemble | -(CH₂)₅- |
| D44 | 2-Cl, 3-Cl-Phe | ensemble | -(CH₂)₅- |
| D45 | 2-F,5-F-Phe | ensemble | -(CH₂)₅- |
| D46 | 2-Cl-,5-méthyl-Phe | ensemble | -(CH₂)₅- |
| D47 | 3-Me, 5-Me-Phe | ensemble | -(CH₂)₅- |
| D50 | 4-hydroxy-3-Me-Phe | ensemble | -(CH₂)₅- |
| D51 | 2,3-(=N-S-N=)Phe | ensemble | -(CH₂)₅- |
| D52 | 2,3-(-CH=CH-CH=CH-)Phe | ensemble | -(CH₂)₅- |
| D53 | 5-indolyle | ensemble | -(CH₂)₅- |
| D54 | 5-benzofurannyle | ensemble | -(CH₂)₅- |
| D55 | 5-benzo[b]thiophényle | ensemble | -(CH₂)₅- |
| D56 | 2-pyridinyle | ensemble | -(CH₂)₅- |
| D57 | 3-pyridinyle | ensemble | -(CH₂)₅- |
| D58 | 2-thiazolyle | ensemble | -(CH₂)₅- |
| D59 | 2-benzothiazolyle | ensemble | -(CH₂)₅- |
sous forme d'un mélange d'isomères ou sous forme d'isomère unique.

5. Composé de formule I conforme à la revendication 1, choisi dans le groupe de composés constitué par les suivantes :
cis-1-méthyl-3-pipéridin-1-ylméthyl-5-m-tolyléthynyl-pyrrolidin-2-one ;
cis-1-méthyl-5-phényléthynyl-3-pipéridin-1-ylméthyl-pyrrolidin-2-one ;
cis-5-(2-chloro-phényléthynyl)-1-méthyl-3-pipéridin-1-ylméthyl-pyrrolidin-2-one ;
cis-N-[2-(1-méthyl-5-oxo-4-pipéridin-1-ylméthyl-pyrrolidin-2-ylméthyl)-phényl]-acétamide ;
cis-1-méthyl-5-(1-méthyl-1H-indol-5-yléthynyl)-3-pipéridin-1-ylméthyl-pyrrolidin-2-one ;
cis-5-benzofurannyl-5-yléthynyl-1-méthyl-3-pipéridin-1-ylméthyl-pyrrolidin-2-one ;
(+)-cis-1-méthyl-3-pipéridin-1-ylméthyl-5-m-tolyléthynyl-pyrrolidin-2-one ;
(+)-cis-1-méthyl-5-phényléthynyl-3-pipéridin-1-ylméthyl-pyrrolidin-2-one ;
(+)-cis-5-(2-chloro-phényléthynyl)-1-méthyl-3-pipéridin-1-ylméthyl-pyrrolidin-2-one ;
(+)-cis-N-[2-(1-méthyl-5-oxo-4-pipéridin-1-ylméthyl-pyrrolidin-2-yléthynyl)-phényl]-acétamide ;
(+)-cis-1-méthyl-5-(1-méthyl-1H-indol-5-yléthynyl)-3-pipéridin-1-ylméthyl-pyrrolidin-2-one ;
(+)-cis-5-benzofurannyl-5-yléthynyl-1-méthyl-3-pipéridin-1-ylméthyl-pyrrolidin-2-one.

6. Composé de formule I conforme à la revendication 1, choisi dans le groupe de composés de formule (E-1) et de formule (E-2) représentés dans le tableau suivant : 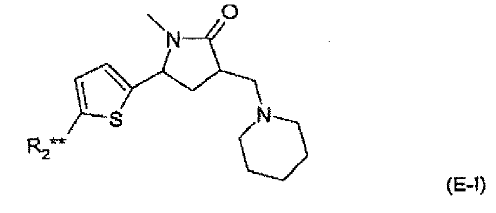
| Composé | R₂** |
|---|---|
| E12 | Phe |
| E13 | 2-Cl-Phe |
| E14 | 3-Br-Phe |
| E15 | 2-Me-Phe |
| E18 | 3-Cl-Phe |
| E20 | 3-nitro-Phe |
| E21 | 3-CN-Phe |
| E24 | 4-Me-Phe |
| E28 | 2-MeO,4-MeO-Phe |
| E29 | 2-F₃C,4-E₃C-Phe |
| E33 | 3,5-Me₂-Phe |
| E35 | 3,4-(-O-CH₂-O-)Phe |
| E36 | thiophén-2-yle |
| E37 | thiophén-3-yle |
| E38 | quinolin-5-yle |
| E41 | 3,4-(=N-O-N=)Phe |
ou 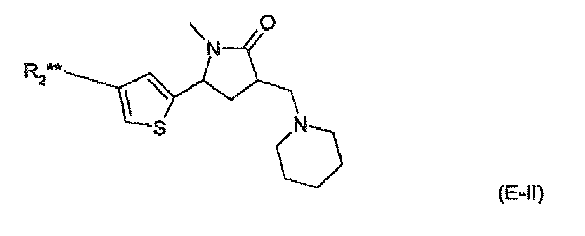
| composé | R₂** |
|---|---|
| E42 | Br |
| E43 | Phe |
| E44 | 2-Cl-Phe |
sous forme d'un mélange d'isomères ou sous forme d'isomère unique.

7. Composé de formule I conforme à la revendication 1, choisi dans le groupe de composés de formule (F) représentés dans le tableau suivant : 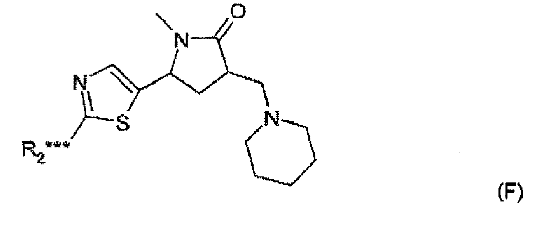
| Composé | R₂*** |
|---|---|
| F8 | 2-Cl-Phe |
| F10 | 2-F₃C-Phe |
| F11 | 4-F₃C-Phe |
| F13 | 2-Cl,4-Cl-Phe |
| F14 | 2-Cl,3-Cl-Phe |
| F15 | 2-Cl,5-Cl-Phe |
| F16 | 4-MeO-Phe |
| F17 | thiophén-2-yle |
| F18 | 3-Cl-thiophén-2-yle |
| F19 | thiophén-3-yle |
| F22 | 3,4-(-O-CH₂-O-)Phe |
| F23 | 3,4-(=N-O-N=)Phe |
| F24 | 3,4-(=N-S-N=)Phe |
sous forme d'un mélange d'isomères ou sous forme d'isomère unique.

8. Composition pharmaceutique comprenant un composé de formule I, et/ou l'un de ses sels de qualité pharmaceutique, conforme à l'une quelconque des revendications 1 à 7, associé à un diluant et/ou vecteur de qualité pharmaceutique.

9. Composé de formule I, et/ou l'un de ses sels de qualité pharmaceutique, conforme à l'une quelconque des revendications 1 à 7, pour emploi en diagnostic ou traitement thérapeutique d'un mammifère, y compris d'un humain, spécialement pour emploi en tant qu'agoniste de récepteur alpha 7.

10. Emploi d'un composé de formule I, et/ou de l'un de ses sels de qualité pharmaceutique, conforme à l'une quelconque des revendications 1 à 7, dans la fabrication d'un médicament destiné au traitement prophylactique ou thérapeutique d'une maladie ou d'un état pathologique dont le traitement fait intervenir ou implique l'activation du récepteur alpha-7 et/ou implique l'activité du récepteur alpha-7.

11. Procédé de fabrication d'un composé de formule I, et/ou d'un sel de qualité pharmaceutique dudit composé, conforme à la revendication 1,
où
pour la synthèse d'un composé de formule I où n est égal à 1 et R₁, R₂, R₃ et R₄ ont les valeurs données dans la revendication 1 pour un composé de formule I, un méthylène de formule II, 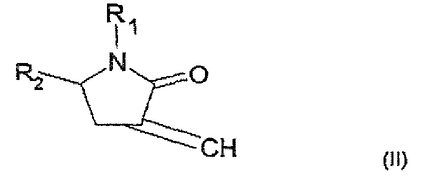 ou l'un de ses sels lorsqu'un groupement susceptible de former un sel est présent, où R₁ et R₂ sont tels que définis pour les composés de formule I dans la revendication 1, réagit avec un composé imino de formule III,
HN (R₃R₄) (III)
ou l'un de ses sels, où R₃ et R₄ ont les valeurs indiquées dans la revendication 1 pour un composé de formule I ; pour obtenir un composé correspondant de formule I, et/ou l'un de ses sels de qualité pharmaceutique ;
ou
et, si cela est recherché, conversion d'un composé de formule I en un composé différent de formule I, conversion d'un sel d'un composé pouvant être obtenu de formule I en le composé libre ou en un sel différent, conversion d'un composé libre pouvant être obtenu de formule I en un sel, et/ou séparation des mélanges pouvant être obtenus d'isomères des composés de formule I en les isomères individuels.

12. Sel d'un composé de formule (I) conforme à l'une quelconque des revendications 1 à 7.

13. Sel de qualité pharmaceutique d'un composé de formule (I) conforme à l'une quelconque des revendications 1 à 7.

14. Composé de formule I, et/ou l'un de ses sels de qualité pharmaceutique, conforme à l'une quelconque des revendications 1 à 7, pour emploi en tant que ligand pour TEP.
